# EUROPEAN PATENT SPECIFICATION

(11) **EP 1 259 597 B1**
(45) Date of publication and mention of the grant of the patent: **11.04.2007**
(21) Application number: 01910136.9
(22) Date of filing: 17.02.2001
(51) Int. Cl.: C12N 15/00, C07K 14/47

(54) **ZINC FINGER DOMAINS AND METHODS OF IDENTIFYING SAME**
ZINK-FINGER DOMäNEN UND VERFAHREN ZUR IDENTIFIZIERUNG DERSELBEN
DOMAINES A DOIGTS DE ZINC ET LEURS PROCEDES D'IDENTIFICATION

(30) Priority: 18.02.2000 KR 2000007730
(43) Date of publication of application: 27.11.2002
(73) Proprietor: Toolgen, Inc., Yuseong-gu, Taejon 305-390 (KR)
(72) Inventor: Kim, Jin-Soo, Yuseong-gu Taejon 305-727 (KR); Kwon, Young-Do, BuPyung-gu Incheon 403-011 (KR); Kim, Hyun-Won, Seongdong-gu Seoul 133-100 (KR); Ryu, Eun Hyun, Dong-gu Taejon 300-130 (KR); Hwang, Moon Sun, Yuseong-gu Taejon 305-390 (KR)
(74) Representative: Schrell, Andreas
(86) International application number: PCT/KR2001/000244
(87) International publication number: WO 2001/060970

(56) References cited:
- WO-A-98/53057
- WO-A-98/54311
- US-A- 5 789 538
- GREISMAN H A ET AL: "A general strategy for selecting high-affinity zinc finger proteins for diverse DNA target sites" SCIENCE, AMERICAN ASSOCIATION FOR THE ADVANCEMENT OF SCIENCE,, US, vol. 275, 1997, pages 657-661, XP002167950 ISSN: 0036-8075
- PELLEGRINO G R ET AL: "IDENTIFICATION AND CHARACTERIZATION OF ZINC-FINGER DOMAINS BY THE POLYMERASE CHAIN REACTION" PROCEEDINGS OF THE NATIONAL ACADEMY OF SCIENCES OF THE UNITED STATES, vol. 88, no. 2, 1991, pages 671-675, XP002233305 1991 ISSN: 0027-8424
- LISOWSKY T ET AL: "Identification of human GC-box-binding zinc finger protein, a new Kruppel-like zinc finger protein, by the yeast one-hybrid screening with a GC-rich target sequence" FEBS LETTERS, ELSEVIER SCIENCE PUBLISHERS, AMSTERDAM, NL, vol. 453, no. 3, 25 June 1999 (1999-06-25), pages 369-374, XP004259906 ISSN: 0014-5793
- DE J ET AL: "Identification of four CCCH zinc finger proteins in Xenopus, including a novel vertebrate protein with four zinc fingers and severely restricted expression" GENE, ELSEVIER BIOMEDICAL PRESS. AMSTERDAM, NL, vol. 228, no. 1-2, 4 March 1999 (1999-03-04), pages 133-145, XP004159143 ISSN: 0378-1119
- PAVLEITICH N P ET AL: "ZINC FINGER-DNA RECOGNITION: CRYSTAL STRUCTURE OF A ZIF268-DNA COMPLEX AT 2.1 A" SCIENCE, AMERICAN ASSOCIATION FOR THE ADVANCEMENT OF SCIENCE,, US, vol. 252, no. 5007, 10 May 1991 (1991-05-10), pages 809-817, XP001094171 ISSN: 0036-8075
- POMERANTZ J.L. ET AL.: 'Structure-based design of transcription factors' SCIENCE vol. 267, no. 5194, January 1995, pages 93 - 96, XP002018160
- KIM J.S. ET AL.: 'Transcriptional repression by zinc finger peptides. Exploring the potential for application in gene therapy' J. BIOL. CHEM. vol. 272, no. 47, November 1997, pages 29795 - 29800, XP002165246
- KIM J.S. ET AL.: 'Getting a handhold on DNA: Design of poly-zinc finger proteins with femtomolar dissociation constants' PROC. NATL. ACAD. SCI. USA vol. 95, no. 6, March 1998, pages 2812 - 2817, XP002167948

## Description

### TECHNICAL FIELD

This invention relates to DNA-binding proteins such as transcription factors

### BACKGROUND

Most genes are regulated at the transcriptional level by polypeptide transcription factors that bind to specific DNA sites within in the gene, typically in promoter or enhancer regions. These proteins activate or repress transcriptional initiation by RNA polymerase at the promoter, thereby regulating expression of the target gene. Many transcription factors, both activators and repressors, are modular in structure. Such modules can fold as structurally distinct domains and have specific functions, such as DNA binding, dimerization, or interaction with the transcriptional machinery. Effector domains such as activation domains or repression domains retain their function when transferred to DNA-binding domains of heterologous transcription factors (Brent and Ptashne, (1985) *Cell* 43:729-36; Dawson *et al.,* (1995) *Mol. Cell Biol.* 15:6923-31). The three-dimensional structures of many DNA-binding domains, including zinc finger domains, homeodomains, and helix-turn-helix domains, have been determined from NMR and X-ray crystallographic data. Greisman and Pabo describe a method for selecting DNA-binding proteins, especially zinc finger proteins, that recognize desired sequences ((1997) Science, 275:657-661). Zinc finger domains can be characterized and identified by the polymerase chain reaction (Pellegrino and Berg, (1991) Proc. Natl. Acad. Sci. 88:671-675).

### SUMMARY

The invention provides a rapid and scalable cell-based method for identifying and constructing chimeric transcription factors. Such transcription factors can be used, for example, for altering the expression of endogenous genes in biomedical and bioengineering applications. The transcription factors are assayed *in vivo*, i.e., in intact, living cells. Also within the invention are novel nucleic acid binding domains that can be discovered, for example, by applying the method in a screen of genomic sequences.

The invention features a method of identifying a peptide domain that recognizes a target site on a DNA. This method is sometimes referred to herein as the "domain selection method" or the *"in vivo* screening method." The method includes providing (1) cells containing a reporter construct and (2) a plurality of hybrid nucleic acids. The reporter construct has a reporter gene operably linked to a promoter that has both a recruitment site and a target site. The reporter gene is expressed above a given level when a transcription factor recognizes (i.e., binds to a degree above background) both the recruitment site and the target site of the promoter, but not when the transcription factor recognizes only the recruitment site of the promoter. Each hybrid nucleic acid of the plurality encodes a non-naturally occurring protein with the following elements: (i) a transcription activation domain, (ii) a DNA binding domain that recognizes the recruitment site, and (iii) a test zinc finger domain. The amino acid sequence of the test zinc finger domain varies among the members of the plurality of hybrid nucleic acids. The method further includes: contacting the plurality of nucleic acids with the cells under conditions that permit at least one of the plurality of nucleic acids to enter at least one of the cells; maintaining the cells under conditions permitting expression of the hybrid nucleic acids in the cells; identifying a cell that expresses the reporter gene above the given level as an indication that the cell contains a hybrid nucleic acid encoding a test zinc finger domain that recognizes the target site.

The DNA binding domain, i.e., the domain that recognizes the recruitment site and does not vary among members of the plurality, can include, for example, one, two, three, or more zinc finger domains. The cells utilized in the method can be prokaryotic or eukaryotic. Exemplary eukaryotic cells are yeast cells, e.g. *Saccharomyces cerevisiae, Schizosaccharomyces pombe,* or, *Pichia pasteuris;* insect cells such as Sf9 cells; and mammalian cells such as fibroblasts or lymphocytes.

The "given level" is the amount of expression observed when the transcription factor recognizes the recruitment site, but not the target site. The "given level" in some cases may be zero (at least within the limits of detection of the assay used).

The method can include an additional step of amplifying a source nucleic acid encoding the test zinc finger domain from a nucleic acid, e.g., genomic DNA, an mRNA mixture, or a cDNA mixture, to produce an amplified fragment. The source nucleic acid can be amplified using an oligonucleotide primer. The oligonucleotide primer can be one of a set of degenerate oligonucleotides (e.g., a pool of specific oligonucleotides having different nucleic acid sequences, or a specific oligonucleotide having a non-natural base such as inosine) that anneals to a nucleic acid encoding a conserved domain boundary. Alternatively, the primer can be a specific oligonucleotide. The amplified fragments are utilized to produce a hybrid nucleic acid for inclusion in the plurality of hybrid nucleic acids used in the aforementioned method.

The method can further include the steps of (i) identifying a candidate zinc finger domain amino acid sequence in a sequence database; (ii) providing a candidate nucleic acid encoding the candidate zinc finger domain amino acid sequence; and (iii) utilizing the candidate nucleic acid to construct a hybrid nucleic acid for inclusion in the plurality of hybrid nucleic acids used in the aforementioned method. The database can include records for multiple amino acid sequences, e.g., known and/or predicted proteins, as well as multiple nucleic acid sequences such as cDNAs, ESTs, genomic DNA, or genomic DNA computationally processed to remove predicted introns.

If desired, the method can be repeated to identify a second test zinc finger domain that recognizes a second target site, e.g., a site other than that recognized by the first test zinc finger domain. Subsequently, a nucleic acid can be constructed that encodes both the first and the second identified test zinc finger domains. The encoded hybrid protein would specifically recognize a target site that includes the target site of the first test zinc finger domain and the target site of the second test zinc finger domain.

The invention also features a method of determining whether a test zinc finger domain recognizes a target site on a promoter. This method is sometimes referred to herein as the "site selection method." The method includes the steps of providing a reporter construct and a hybrid nucleic acid. The reporter gene is operably linked to a promoter that includes a recruitment site and a target site, and is expressed above a given level when a transcription factor recognizes both the recruitment site and the target site of the promoter, but not when the transcription factor recognizes only the recruitment site of the promoter. The hybrid nucleic acid encodes a non-naturally occurring protein with the following elements: (i) a transcription activation domain, (ii) a DNA binding domain that recognizes the recruitment site, and (iii) a test zinc finger domain. The method further includes: contacting the reporter construct with a cell under conditions that permit the reporter construct to enter the cell; prior to, after, or concurrent with the aforementioned step, contacting the hybrid nucleic acid with the cell under conditions that permit the hybrid nucleic acid to enter the cell; maintaining the cell under conditions permitting expression of the hybrid nucleic acid in the cell; and detecting reporter gene expression in the cell. A level of reporter gene expression greater than the given level is an indication that the test zinc finger domain recognizes the target site.

The reporter construct and the hybrid nucleic acid can be contained in separate plasmids. The two plasmids can be introduced into the cell simultaneously or consecutively. One or both plasmids can contain selectable markers. The reporter construct and the hybrid nucleic acid can also be contained on the same plasmid, in which case only one contacting step is required to introduce both nucleic acids into a cell. In yet another implementation, one or both of the nucleic acids are stably integrated into a genome of a cell. For this method, as for any in *vivo* method described herein, the transcriptional activation domain can be replaced with a transcriptional repression domain, and a cell is identified in which the level of reporter gene expression is decreased to a level below the given level.

Another method of the invention facilitates the rapid determination of a binding preference of a test zinc finger domain by fusing two cells. The method includes: providing a first cell containing the reporter gene; providing a second cell containing the hybrid nucleic acid; fusing the first and second cells to form a fused cell; maintaining the fused cells under conditions permitting expression of the hybrid nucleic acids in the fused cell; and detecting reporter gene expression in the fused cell, wherein a level of reporter gene expression greater than the given level is an indication that the test zinc finger domain recognizes the target site. For example, the first and second cells can be tissue culture cells or fungal cells. An exemplary implementation of the method utilizes *S*. *cerevisiae* cells. The first cell has a first mating type, e.g., MATa; the second cell has a second mating type different from the first, e.g., MATα. The two cells are contacted with one another, and yeast mating produces a single cell (e.g., MATa/α) with a nucleus containing the genomes of both the first and second cells. The method can including providing multiple first cells, all of the same first mating type where each first cell has a reporter construct with a different target site. Multiple second cells, all of the same second mating type and each having a different test zinc finger domain, are also provided. A matrix is generated of multiple pair-wise matings, e.g., all possible pair-wise matings. The method is applied to determine the binding preference of multiple test zinc finger domains for multiple binding sites, e.g., a complete set of possible target sites.

The invention also provides a method of assaying a binding preference-of a test zinc finger domain. The method includes providing (1) cells, essentially all of which contain a hybrid nucleic acid, and (2) a plurality of reporter constructs. Each reporter construct of the plurality has a reporter gene operably linked to a promoter with a recruitment site and a target site. The reporter gene is expressed above a given level when a transcription factor recognizes both the recruitment site and the target site of the promoter, but not when the transcription factor binds only the recruitment site of the promoter. The second target site varies among the members of the plurality of reporter constructs. The hybrid nucleic acid encodes a,hybrid protein with the following elements: (i) a transcription activation domain, (ii) a DNA binding domain that recognizes the recruitment site, and (iii) a test zinc finger domain. The method further includes: contacting the plurality of reporter constructs with the cells under conditions that permit at least one of the plurality of reporter constructs to enter at least one of the cells; maintaining the cells under conditions permitting expression of the nucleic acids in the cells; identifying a cell that contains a reporter construct in the cell and that expresses the reporter construct above the given level as an indication that the reporter construct in the cell has a target site recognized by the zinc finger domain.

A plurality of cells, each with a different target site, can be identified by the above method if the test zinc finger domain has a binding preference for more than one target site. The method can further include identifying the cell that exhibits the highest level of reporter gene expression. Alternatively, a threshold level of reporter gene expression is determined, e.g., an increase in reporter gene expression of 2, 4, 8, 20, 50, 100, 1000 fold or greater, and all cells exhibiting reporter gene expression above the threshold are selected.

The target binding site, for example, can be between two and six nucleotides long. The plurality of reporter constructs can include every possible combination of A, T, G, and C nucleotides at two, three, or four or more positions of the target binding site.

In another aspect, the invention features a method of identifying a plurality of zinc finger domains. The method includes: carrying out the domain selection method to identify a first test zinc finger domain and carrying out the domain selection method again to identify a second test zinc finger domain that recognizes a target site different from a target site of the first test zinc finger domain. Also featured is a method of generating a nucleic acid encoding a chimeric zinc finger protein, the method includes carrying out the domain selection method twice to identify a first and second test zinc finger domain and constructing a nucleic acid encoding a polypeptide including the first and second test zinc finger domains. The nucleic acid can encode a hybrid protein that includes the two domains that specifically recognize a site that includes two subsites. The subsites are the target site of the first test zinc finger domain and target site of the second test zinc finger domain.

In still another aspect, the invention features a method of identifying a DNA sequence recognized by zinc finger domains. The method includes: carrying out the site selection method to identify a first binding preference for a first test zinc finger domain, and carrying out the site selection method again to identify a second binding preference for a second test zinc finger domain. A nucleic acid can be constructed which encodes both the first and the second identified test zinc finger domains. The nucleic acid can encode a hybrid protein including the two domains that specifically recognizes a site that includes the target site of the first test zinc finger domain and target site of the second test zinc finger domain.

The invention also features a method of identifying a peptide domain that recognizes a target site on a DNA. The method includes providing (1) cells containing a reporter construct and (2) a plurality of hybrid nucleic acids. The reporter construct has a reporter gene operably linked to a promoter that has both a recruitment site and a target site. The reporter gene is expressed below a given level when a transcription factor recognizes (i.e., binds to a degree above background) both the recruitment site and the target site of the promoter, but not when the transcription factor recognizes only the recruitment site of the promoter. Each hybrid nucleic acid of the plurality encodes a non-naturally occurring protein with the following elements: (i) a transcription repression domain, (ii) a DNA binding domain that recognizes the recruitment site, and (iii) a test zinc finger domain. The amino acid sequence of the test zinc finger domain varies among the members of the plurality of hybrid nucleic acids. The method further includes: contacting the plurality of nucleic acids with the cells under conditions that permit at least one of the plurality of nucleic acids to enter at least one of the cells; maintaining the cells under conditions permitting expression of the hybrid nucleic acids in the cells; identifying a cell that expresses the reporter gene below the given level as an indication that the cell contains a hybrid nucleic acid encoding a test zinc finger domain that recognizes the target site. Additional embodiments of this method are as for the similar method utilizing a transcription activation domain. Likewise, any other selection method described herein can be performed using a transcriptional repression domain in place of a transcriptional activation domain.

In another aspect, the methods of the invention feature certain purified polypeptides and isolated nucleic acids. Purified polypeptide of the invention include polypeptide having the amino acid sequence:
Xₐ-X-Cys-X₂₋₅-Cys-X₃-Xₐ-X-Cys-X-Ser-Asn-X_{b}-X-Arg-His-X₃₋₅-His (SEQ ID NO:68),
Xₐ-X-Cys-X₂₋₅-Cys-X₃-Xₐ-X-His-X-Ser-Asn-X_{b}-X-Lys-His-X₃₋₅-His (SEQ ID NO:69),
Xₐ-X-Cys-X₂₋₅-Cys-X₃-Xₐ-X-Ser-X-Ser-Asn-X_{b}-X-Arg-His-X₃₋₅-His (SEQ ID NO:70),
Xₐ-X-Cys-X₂₋₅-Cys-X₃-Xₐ-X-Gln-X-Ser-Thr-X_{b}-X-Val-His-X₃₋₅-His (SEQ ID NO:71),
Xₐ-X-Cys-X₂₋₅-Cys-X₃-Xₐ-X-Val-X-Ser-X_{c}-X_{b}-X-Arg-His-X₃₋₅-His (SEQ ID NO:72),
Xₐ-X-Cys-X₂₋₅-Cys-X₃-Xₐ-X-Gln-X-Ser-His-X_{b}-X-Arg-His-X₃₋₅-His (SEQ ID NO:73),
Xₐ-X-Cys-X₂₋₅-Cys-X₃-Xₐ-X-Gln-X-Ser-Asn-X_{b}-X-Val-His-X₃₋₅-His (SEQ ID NO:74),
Xₐ-X-Cys-X₂₋₅-Cys-X₃-Xₐ-X-Gln-X-Ser-X_{c}-X_{b}-X-Arg-His-X₃₋₅-His (SEQ ID NO:75),
Xₐ-X-Cys-X₂₋₅-Cys-X₃-Xₐ-X-Gln -X-Ala-His-X_{b}-X-Arg-His-X₃₋₅-His (SEQ ID NO:150),
Xₐ-X-Cys-X₂₋₅-Cys-X₃-Xₐ-X-Gln-X-Phe-Asn-X_{b}-X-Arg-His-X₃₋₅-His (SEQ ID NO:151),
Xₐ-X-Cys-X₂₋₅-Cys-X₃ -Xₐ-X-Gln-X-Ser-His-X_{b}-X-Thr-His-X₃₋₅-His (SEQ ID NO: 152),
Xₐ-X-Cys-X₂₋₅-Cys-X₃-Xₐ-X-Gln-X-Ser-His-X_{b}-X-Val-His-X₃₋₅-His (SEQ ID NO:153),
Xₐ-X-Cys-X₂₋₅-Cys-X₃-Xₐ-X-Gln-X-Ser-Asn-X_{b}-X-Ile-His-X₃₋₅-His (SEQ ID NO:154),
Xₐ-X-Cys-X₂₋₅-Cys-X₃-Xₐ-X-Gln-X-Ser-Asn-X_{b}-X-Arg-His-X₃₋₅-His (SEQ ID NO:155),
Xₐ-X-Cys-X₂₋₅-Cys-X₃-Xₐ-X-Gln-X-Thr-HiS-X_{b}-X-Gln-HiS-X₃₋₅-His (SEQ ID NO:156),
Cys-X₂₋₅-Cys-X₃-Xₐ-X-Gln-X-Thr-His-X_{b}-X-Arg-His-X₃₋₅-His (SEQ ID NO: 15 7),
Xₐ-X-Cys-X₂₋₅-Cys-X₃-Xₐ-X-Arg-X-Asp-Lys-X_{b}-X-Ile-His-X₃₋₅-His (SEQ ID NO: 15 8),
Xₐ-X-Cys-X₂₋₅-Cys-X₃-Xₐ-X-Arg-X-Ser-Asn-X_{b}-X-Arg-His-X₃₋₅-His (SEQ ID NO: 159),
Xₐ-X-Cys-X₂₋₅-Cys-X₃-Xₐ-X-Gln-X-Gly-Asn-X_{b} X-Arg-His-X₃₋₅-His (SEQ ID NO:161),
Xₐ-X-Cys-X₂₋₅-Cys-X₃-Xₐ-X-Arg-X-Asp-Glu-X_{b}-X-Arg-His-X₃₋₅-His (SEQ ID NO: 162),
Xₐ-X-Cys-X₂₋₅-Cys-X₃-Xₐ-X-Arg-X-Asp-His-X_{b}-X-Arg-His-X₃₋₅-His (SEQ ID NO:163),
Xₐ-X-Cys-X₂₋₅-Cys-X₃-Xₐ-X-Arg-X-Asp-His-X_{b}-X-Thr-His-X₃₋₅-His (SEQ ID NO:164),
Xₐ-X-Cys-X₂₋₅-Cys-X₃-Xₐ-X-Arg-X-Asp-Lys-X_{b}-X-Arg-His-X₃₋₅-His (SEQ ID NO:165),
Xₐ-X-Cys-X₂₋₅-Cys-X₃-Xₐ-X-Arg-X-Ser-His-X_{b}-X-Arg-His-X₃₋₅-His (SEQ ID NO:166), or
Xₐ-X-Cys-X₂₋₅-Cys-X₃-Xₐ-X-Arg-X-Thr-Asn-X_{b}-X-Arg-His-X₃₋₅-His (SEQ ID NO:167),
wherein Xₐ is phenylalanine or tyrosine, X_{b} is a hydrophobic residue, and X_{c} is serine or threonine. Nucleic acids of the invention include nucleic acids encoding the aforementioned polypeptides.

In addition, purified polypeptides of the methods of the invention can have amino acids sequence 50%, 60%, 70%, 80%, 90%, 93%, 95%, 96%, 98%, 99%, or 100% identical to SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, or 151. The polypeptides can be identical to SEQ ID NOs: 23, 25, 27, 29, 31, 33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143, 145, 147, 149, or 151 at the amino acid positions corresponding to the nucleic acid contacting residues of the polypeptide.
Alternatively, the polypeptides differ from SEQ ID NOs: 23,25,27,29,31,33, 35, 37, 39, 41, 43, 45, 47, 49, 51, 53, 55, 57, 59, 61, 63, 65, 67, 103, 105, 107, 111, 113, 115, 117, 119, 121, 123, 125, 127, 129, 131, 133, 135, 137, 141, 143,145, 147, 149, or 151 at at least one of the residues corresponding to the nucleic acid contacting residues of the polypeptide. The purified polypeptides can also include one or more of the following: a heterologous DNA binding domain, a nuclear localization signal, a small molecular binding domain (e.g., a steroid binding domain), an epitope tag or purification handle, a catalytic domain (e.g., a nucleic acid modifying domain, a nucleic acid cleavage domain, or a DNA repair catalytic domain) and/or a transcriptional function domain (e.g., an activation domain, a repression domain, and so forth). The methods of the invention also include isolated nucleic acid sequences encoding the aforementioned polypeptides, and isolated nucleic acid sequences that hybridize under high stringency conditions to a single stranded probe, the sequence of the probe consisting of SEQ ID NO-22, 24, 26, 28, 30, 32, 34, 36, 38, 40; 42, 44, 46, 48, 50, 52, 54, 56, 58, 60, 62, 64, 66, 102, 104, 106, 110, 112, 114, 116, 118, 120, 122, 124, 126, 128, 130, 132, 134, 136, 140, 142, 144, 146, 148, or 150 or the complements thereof. The invention further includes a method of expressing in a cell a polypeptide of the invention fused to a heterologous nucleic acid binding domain. The method includes introducing into a cell a nucleic acid encoding the aforementioned fusion protein. A nucleic acid of the invention can be operably regulated by a heterologous nucleic acid sequence, e.g., an inducible promoter (e.g., a steroid hormone regulated promoter, a small-molecule regulated promoter, or an engineered inducible system such as the tetracycline Tet-On and Tet-Off systems).

The term "base contacting positions" refers to the four amino acid positions of zinc finger domains that structurally correspond to amino acids arginine 73, aspartic acid 75, glutamic acid 76, and arginine 79 of SEQ ID NO:21. These positions are also referred to as positions -1, 2, 3, and 6. To identify positions in a query sequence that correspond to the base contacting positions, the query sequence is aligned to the zinc finger domain of interest such that the cysteine and histidine residues of the query sequence are aligned with those of finger 3 of Zif268. The ClustalW WWW Service at the European Bioinformatics Institute (http://www2.ebi.ac.uk/clustalw; Thompson *et al.* (1994) *Nucleic Acids Res.* 22:4673-4680) provides one convenient method of aligning sequences.

The term "heterologous" refers to a polypeptide that is introduced into a context by artifice, and that does not occur naturally in the same context. In distinction from an endogenous entity, a heterologous polypeptide can have a polypeptide sequence flanking it on at least one side that does not flank it in any naturally occurring polypeptide. The term "hybrid" refers to a polypeptide which comprises amino acid sequences derived from either (i) at least two different naturally occurring sequences; (ii) at least an artificial sequence (i.e., a sequence that does not occur naturally) and a naturally occurring sequence; or (iii) at least two different artificial sequences. Examples of artificial sequences include mutants of a naturally occurring sequence and *de novo* designed sequences.

As used herein, the term "hybridizes under stringent conditions" refers to conditions for hybridization in 6X sodium chloride/sodium citrate (SSC) at 45°C, followed by two washes in 0.2 X SSC, 0.1% SDS at 65°C.

The term "binding preference" refers to the discriminative property of a polypeptide for selecting one nucleic acid binding site relative to another. For example, when the polypeptide is limiting in quantity relative to the nucleic acid binding sites, a greater amount of the polypeptide will bind the preferred site relative to the other site in an *in vivo* or *in vitro* assay described herein.

As used herein, the term "recognizes" refers to the ability of a polypeptide to discriminate between one nucleic acid binding site and a second competing site such that, e.g., in the context of an assay described herein, the polypeptide remains bound to the first site in the presence of an excess of the second site. The polypeptide may not have sufficient affinity for the first site to bind alone, but may be assayed when fused as in a hybrid polypeptide of the invention to another nucleic acid binding domain that binds a nearby recruitment site.

As used herein, "degenerate oligonucleotides" refers to both (a) a population of different oligonucleotides, and (b) a single species of oligonucleotide that can anneal to more than one sequence, e.g., an oligonucleotide with an unnatural nucleotide such as inosine.

The present invention provides numerous benefits. The ability to select a DNA binding domain that recognizes a particular sequence permits the design of novel polypeptides that bind to specific site on a DNA. Thus, the invention facilitates the customized generation of novel polypeptides that can regulate the expression of a selected target, e.g., a gene required by a pathogen can be repressed, a gene required for cancerous growth can be repressed, a gene poorly expressed or encoding a mutated protein can be activated and overexpressed, and so forth.

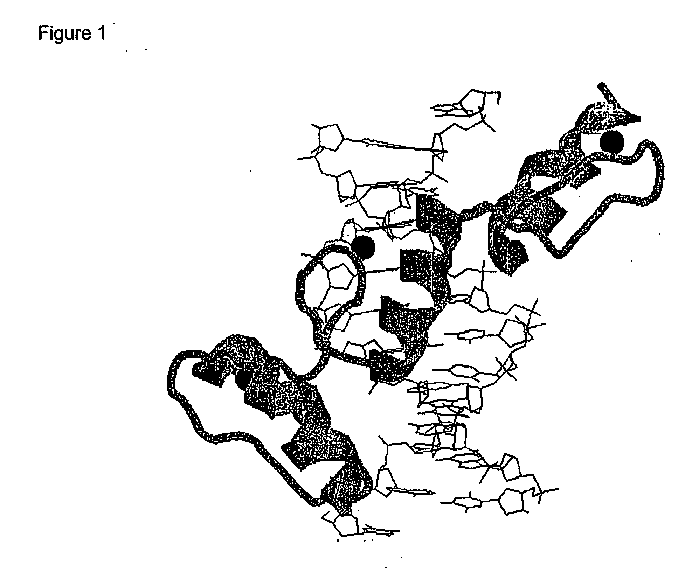

The use of zinc finger domains is particularly advantageous. First, the zinc finger motif recognizes very diverse DNA sequences. Second, the structure of naturally occurring zinc finger proteins is modular. For example, the zinc finger protein Zif268, also called "Egr-1," is composed of a tandem array of three zinc finger domains. Fig. 1 is the x-ray crystallographic structure of zinc finger protein Zif268, consisting of three fingers complexed with DNA (Pavletich and Pabo, (1991) *Science* 252:809-817). Each finger independently contacts 3-4 basepairs of the DNA recognition site. Hence, the subsite contacted by each finger can be regarded as an independent molecular recognition event. High affinity binding is achieved by the cooperative effect of having multiple zinc finger modules in the same polypeptide chain.

The use of an *in vivo* selection step enables one to identify directly those polypeptides that bind to a specific site on a DNA in the intracellular milieu. The factors associated with recognition in a cell, particularly a eukaryotic cell, can be vastly different from the factors present during an *in vitro* selection scenario. For example, in a eukaryotic nucleus, a polypeptide must compete with the myriad other nuclear proteins for a specific nucleic acid binding site. A nucleosome or another chromatin protein can occupy, occlude, or compete for the binding site. Even if unbound, the conformation of a nucleic acid in the cell is subject to bending, supercoiling, torsion, and unwinding. Conversely, the polypeptide itself is exposed to proteases and chaperones, among other factors. Moreover, the polypeptide is confronted with an entire genome of possible binding sites, and hence must be endowed with a high specificity for the desired site in order to survive the selection process. In contrast to *in vivo* selection, an *in vitro* selection can select for the highest affinity binder rather than the highest specificity binder.

The use of a reporter gene to indicate the binding ability of an expressed polypeptide chimera not only is efficient and simple, but also obviates the need to develop a complex interaction code that accounts for the energetics of the protein-nucleic acid interface and the immense number of peripheral factors, such as surrounding residues and nucleotides that also affect the binding interface. (Segal *et al.* (1999) *Proc. Natl. Acad. Sci. USA* 96:2758-2763).

The present invention avails itself of all the zinc finger domains present in the human genome, or any other genome. This diverse sampling of sequence space occupied by the zinc finger domain structural fold may have the additional advantages inherent in eons of natural selection. Moreover, by utilizing domains from the host species, a DNA binding protein engineered for a gene therapy application by the methods described herein has a reduced likelihood of being regarded as foreign by the host immune response.

The details of one or more embodiments of the invention are set forth in the accompanying drawings and the description below. Other features, objects, and advantages of the invention will be apparent from the description and drawings, and from the claims.

### DESCRIPTION OF DRAWINGS

Fig. 1 is a depiction of the three dimensional structure of the Zif268 zinc finger protein that consists of three finger domains and binds the DNA sequence, 5'-GCG TGG GCG T-3'. The black circles represent the location of the zinc ion.
Fig. 2 is an illustration of the hydrogen-bonding interactions between amino acid residues of Zif268 and DNA bases. Amino acid residues at positions -1, 2, 3, and 6 along the α-helix interact with the bases at specific positions. The bold lines represent ideal hydrogen bonding, while the dotted lines represent potential hydrogen bonding.
Fig. 3 is a recognition code table that summarizes the interactions between DNA bases and amino acid residues at positions -1, 2, 3, and 6 along the α-helix of a zinc finger domain.

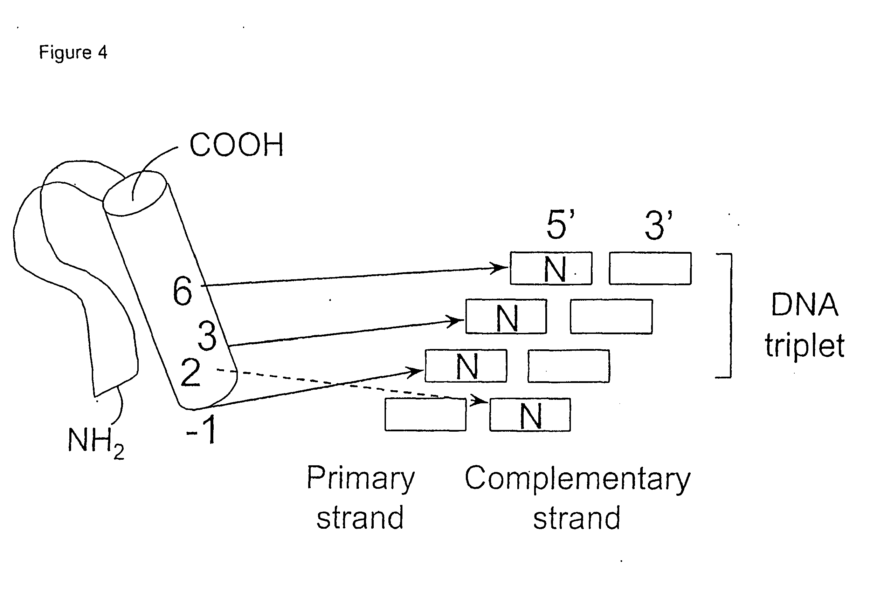

Fig. 4 is a depiction of the positions of amino acid residues and their corresponding 3 base triplets. The bold lines represent the main interactions observed, while the dotted line represents an auxiliary interaction.
Fig. 5 is a diagram illustrating the principles of the *in vivo* selection system disclosed herein. Of the various zinc finger mutants, zinc finger domain A recognizes the target sequence (designated XXX X) and activates the transcription of *HIS3* reporter gene. As a result, yeast colonies grow on a medium lacking histidine. In contrast, zinc finger domain B does not recognize the target sequence and thus the reporter gene remains repressed. As a result, no colonies grow on a medium lacking histidine. AD represents the transcriptional activation domain.
Fig. 6 is a list of 10-bp sequences found in long terminal repeats (LTR) of HIV-1 and in the promoter region of CCR5, a human gene encoding a coreceptor for HIV-1 (SEQ ID NOs:1-5, respectively). The underlined portions represent 4-bp target sequences used in the present selection.
Fig. 7 is a depiction of the base sequences of the binding sites linked to the reporter gene (SEQ ID NOs:6-17, respectively). Each binding site consists of a tandem array of 4 composite binding sequences. Each composite binding sequence was constructed by connecting truncated binding sequence 5'-GG GCG-3' recognized by finger 1 and finger 2 of Zif268 to 4-bp target sequences.
Fig 8 is a diagram of pPCFMS-Zif, a plasmid that can be used for the construction of a library of hybrid plasmids (SEQ ID NOs:18 and 19).
Fig 9 is a representation of the base sequence for the gene coding for Zif268 zinc finger protein inserted into pPCFMS-Zif and the corresponding translated amino acid sequences (SEQ ID NOs:20 and 21, respectively). Sites recognized by restriction enzymes are underlined.
Fig. 10 is a photograph of a culture plate having yeast cells obtained from retransformation and cross transformation using zinc finger proteins selected by the *in vivo* selection system.

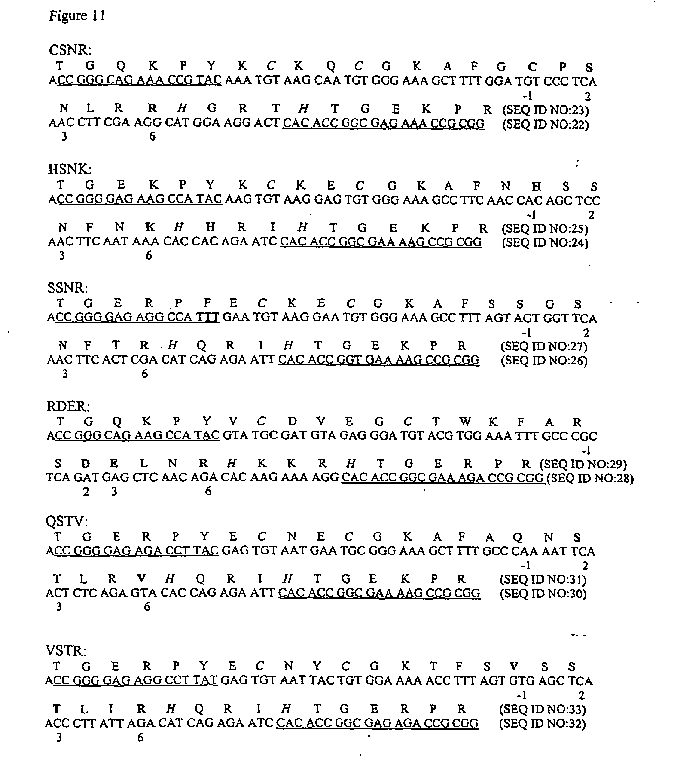

Fig. 11 is a list of some DNA sequences of zinc finger domains selected by the *in vivo* system from a zinc finger library derived from the human genome and amino acid sequences encoded by the DNA sequences (SEQ ID NOs:22-33). The DNA sequences corresponding to the degenerate PCR primers used to amplify DNA segments encoding zinc finger domains from the human genome are underlined. The four potential base-contacting positions are indicated, and the amino acid residues are shown in bold. The two Cys residues and two His residues that are expected to coordinate with the zinc ion are shown in italics.

### DETAILED DESCRIPTION

The invention features a novel screening method for determining the nucleic acid binding preferences of test zinc finger domains. The method is easily adapted to a variety of DNA binding domains, a variety of sources for these domains, and a number of library designs, reporter genes, and selection and screening systems. The screening method can be implemented as a high-throughput platform. Information obtained from the screening method is readily applied to a method of designing artificial nucleic acid binding proteins. The design method appropriates the binding preferences of test zinc finger domains to guide the modular assembly of a chimeric nucleic acid binding protein. A designed protein can be further optimized or varied with the screening method.

### DNA binding domains

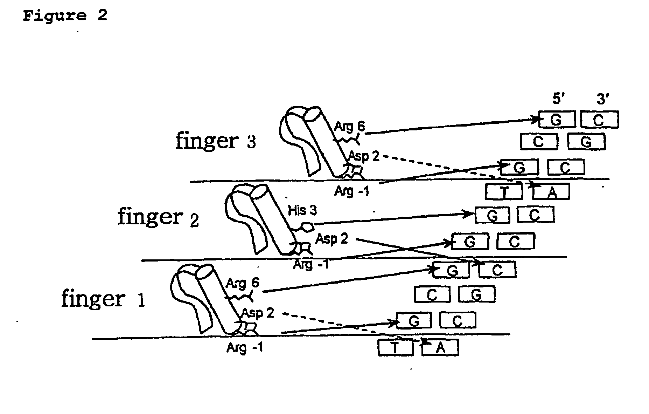

The invention utilizes collections of nucleic-acid binding domains with differing binding specificities. A variety of protein structures are known to bind nucleic acids with high affinity and high specificity. These structures are used repeatedly in a myriad of different proteins to specifically control nucleic acid function (for reviews of structural motifs which recognize double stranded DNA, see, e.g., Pabo and Sauer (1992) *Annu. Rev. Biochem.* 61:1053-95; Patikoglou and Burley (1997) *Annu. Rev. Biophys. Biomol. Struct.* 26:289-325; Nelson (1995) *Curr Opin Genet Dev.* 5:180-9). A few non-limiting examples of nucleic acid binding domains include:
**Zinc fingers.** Zinc fingers are small polypeptide domains of approximately 30 amino acid residues in which there are four amino acids, either cysteine or histidine, appropriately spaced such that they can coordinate a zinc ion (Fig. 1; for reviews, see, e.g., Klug and Rhodes, (1987) *Trends Biochem.* Sci.12:464-469(1987); Evans and Hollenberg, (1988) *Cell* 52:1-3; Payre and Vincent, (1988) *FEBS Lett.* 234:245-250; Miller *et al.,* (1985) *EMBO J.* 4:1609-1614; Berg, (1988) *Proc. Natl. Acad. Sci. U.S.A.* 85:99-102; Rosenfeld and Margalit, (1993) *J. Biomol. Struct. Dyn.* 11:557-570). Hence, zinc finger domains can be categorized according to the identity of the residues that coordinate the zinc ion, e.g., as the Cys₂-His₂ class, the Cys₂-Cys₂ class, the Cys₂-CysHis class, and so forth. The zinc coordinating residues of Cys₂-His₂ zinc fingers are typically spaced as follows: Xₐ-X-C-X₂₋₅-C-X₃-Xₐ-X₅-ψ-X₂-H-X₃₋₅-H, where ψ (psi) is a hydrophobic residue (Wolfe *et al.,* (1999) *Annu. Rev*. *Biophys. Biomol. Struct.* 3:183-212) (SEQ ID NO:76), wherein "X" represents any amino acid, wherein Xₐ is phenylalanine or tyrosine, the subscript indicates the number of amino acids, and two subscripts indicate a typical range of intervening amino acids. Typically, the intervening amino acids fold to form an anti-parallel β-sheet that packs against an α-helix, although the anti-parallel β-sheets can be short, non-ideal, or non-existent. The fold positions the zinc-coordinating side chains so they are in a tetrahedral conformation appropriate for coordinating the zinc ion. The base contacting residues are at the N-terminus of the finger and in the preceding loop region (Fig. 2). A zinc finger DNA-binding protein normally consists of a tandem array of three or more zinc finger domains.

The zinc finger domain (or "ZFD") is one of the most common eukaryotic DNA-binding motifs, found in species from yeast to higher plants and to humans. By one estimate, there are at least several thousand zinc finger domains in the human genome alone. Zinc finger domains can be isolated from zinc finger proteins. Non-limiting examples of zinc finger proteins include CF2-II, Kruppel, WT1, basonuclin, BCL-6/LAZ-3, erythroid Kruppel-like transcription factor, transcription factors Sp1, Sp2, Sp3, and Sp4, transcriptional repressor YY1, EGR1/Krox24, EGR2/Krox20, EGR3/Pilot, EGR4/AT133, Evi-1, GLI1, GLI2, GLI3, HIV-EP1/ZNP40, HIV-EP2, KR1, ZfX, ZfY, and ZNF7.

Computational methods described below can be used to identify all zinc finger domains encoded in a sequenced genome or in a nucleic acid database. Any such zinc finger domain can be utilized. In addition, artificial zinc finger domains have been designed, e.g., using computational methods (e.g., Dahiyat and Mayo, (1997) *Science* 278:82-7). The zinc finger of Dahiyat and Mayo adopts the zinc finger fold, but does not contain a zinc ion in its core. Thus, it is a zinc finger by structural similarity of its polypeptide backbone to the fold of naturally occurring zinc fingers, rather than by functional ability to coordinate a zinc ion.

**Homeodomains.** Homeodomains are simple eukaryotic domains that consist of a N-terminal arm that contacts the DNA minor groove, followed by three α-helices that contact the major groove (for a review, see, e.g., Laughon, (1991) *Biochemistry* 30:11357-67). The third α-helix is positioned in the major groove and contains critical DNA-contacting side chains. Homeodomains have a characteristic highly-conserved motif present at the turn leading into the third α-helix. The motif includes an invariant tryptophan that packs into the hydrophobic core of the domain. This motif is represented in the Prosite database (see http://www.expasy.ch/) as PDOC00027 ([L/I/V/M/F/Y/G]-[A/S/L/V/R]-X(2)-[L/I/V/M/S/T/A/C/N]-X-[L/I/V/M]-X(4)-[L/I/V]- [R/K/N/Q/E/S/T/A/I/Y]-[L/I/V/F/S/T/N/K/H]-W-[F/Y/V/C]_ X-[N/D/Q/T/A/H]-X(5)- [R/K/N/A/I/M/W], SEQ ID NO:77). Homeodomains are commonly found in transcription factors that determine cell identity and provide positional information during organismal development. Such classical homeodomains can be found in the genome in clusters such that the order of the homeodomains in the cluster approximately corresponds to their expression pattern along a body axis. Homeodomains can be identified by alignment with a homeodomain, e.g., Hox-1, or by alignment with a homeodomain profile or a homeodomain hidden Markov Model (HMM; see below), e.g., PF00046 of the Pfam database or "HOX" of the SMART database (http://smart.embl-heidelberg.de/), or by the Prosite motif PDOC00027 as mentioned above.

**Helix-turn-helix proteins.** This DNA binding motif is common among many prokaryotic transcription factors. There are many subfamilies, e.g., the LacI family, the AraC family, to name but a few. The two helices in the name refer to a first α-helix that packs against and positions a second α-helix in the major groove of DNA. These domains can be identified by alignment with a HMM, e.g., HTH_ARAC, HTH_ARSR, HTH_ASNC, HTH_CRP, HTH_DEOR, HTH_DTXR, HTH_GNTR, HTH_ICLR, HTH_LACI, HTH_LUXR, HTH_MARR, HTH_MERR, and HTH_XRE profiles available in the SMART database (http://smart.embl-heidelberg.de/).

**Helix-loop-helix proteins.** This DNA binding domain is commonly found among homo- and hetero-dimeric transcription factors, e.g., MyoD, fos, jun, E11, and myogenin. The domain consists of a dimer, each monomer contributing two α-helices and intervening loop. The domain can be identified by alignment with a HMM, e.g., the "HLH" profile available in the SMART database (http://smart.embl-heidelberg.de/). Although helix-loop-helix proteins are typically dimeric, monomeric versions can be constructed by engineering a polypeptide linker between the two subunits such that a single open reading frame encodes both the two subunits and the linker.

### Identification of DNA-binding domains

A variety of methods can be used to identify structural domains.

**Computational Methods.** The amino acid sequence of a DNA binding domain isolated by a method described herein can be compared to a database of known sequences, e.g., an annotated database of protein sequences or an annotated database which includes entries for nucleic acid binding domains. In another implementation, databases of uncharacterized sequences, e.g., unannotated genomic, EST or full-length cDNA sequence; of characterized sequences, e.g., SwissProt or PDB; and of domains, e.g., Pfam, ProDom (http://www.tooulouse.inra.fr/), and SMART (Simple Modular Architecture Research Tool, http://smart.embl-heidelberg.de/) can provide a source of nucleic acid binding domain sequences. Nucleic acid sequence databases can be translated in all six reading frames for the purpose of comparison to a query amino acid sequence. Nucleic acid sequences that are flagged as encoding candidate nucleic acid binding domains can be amplified from an appropriate nucleic acid source, e.g., genomic DNA or cellular RNA. Such nucleic acid sequences can be cloned into an expression vector. The procedures for computer-based domain identification can be interfaced with an oligonucleotide synthesizer and robotic systems to produce nucleic acids encoding the domains in a high-throughput platform. Cloned nucleic acids encoding the candidate domains can also be stored in a host expression vector and shuttled easily into an expression vector, e.g., into a translational fusion vector with Zif268 fingers I and 2, either by restriction enzyme mediated subcloning or by site-specific, recombinase mediated subcloning (see U.S. Patent No. 5,888,732). The high-throughput platform can be used to generate multiple microtitre plates containing nucleic acids encoding different candidate nucleic acid binding domains.

Detailed methods for the identification of domains from a starting sequence or a profile are well known in the art. See, for example, Prosite (Hofmann *et al.,* (1999) *Nucleic Acids Res.* 27:215-219), FASTA, BLAST (Altschul *et al.,* (1990) *J. Mol. Biol.* 215:403-10.), etc. A simple string search can be done to find amino acid sequences with identity to a query sequence or a query profile, e.g., using Perl (http://bio.perl.org/) to scan text files. Sequences so identified can be about 30%, 40%, 50%, 60%, 70%, 80%, 90%, or greater identical to an initial input sequence.

Domains similar to a query domain can be identified from a public database, e.g., using the XBLAST programs (version 2.0) of Altschul *et al.,* (1990) *J*. *Mol. Biol.* 215:403-10. For example, BLAST protein searches can be performed with the XBLAST parameters as follows: score = 50, wordlength = 3. Gaps can be introduced into the query or searched sequence as described in Altschul et al., (1997) *Nucleic Acids Res.* 25(17):3389-3402. Default parameters for XBLAST and Gapped BLAST programs are available at http://www.ncbi.nlm.nih.gov.

The Prosite profiles PS00028 and PS50157 can be used to identify zinc finger domains. In a SWISSPROT release of 80,000 protein sequences, these profiles detected 3189 and 2316 zinc finger domains, respectively. Profiles can be constructed from a multiple sequence alignment of related proteins by a variety of different techniques. Gribskov and co-workers (Gribskov *et al.,* (1990) *Meth. Enzymol.* 183:146-159) utilized a symbol comparison table to convert a multiple sequence alignment supplied with residue frequency distributions into weights for each position. See, for example, the PROSITE database and the work of Luethy *et al.,* (1994) *Protein Sci.* 3:139-1465.

Hidden Markov Models (HMM's) representing a DNA binding domain of interest can be generated or obtained from a database of such models, e.g., the Pfam database, release 2.1. A database can be searched, e.g., using the default parameters, with the HMM in order to find additional domains (see, e.g., http://www.sanger.ac.uk/Software/Pfam/HMM_search for default parameters). Alternatively, the user can optimize the parameters. A threshold score can be selected to filter the database of sequences such that sequences that score above the threshold are displayed as candidate domains. A description of the Pfam database can be found in Sonhammer *et al.,* (1997) *Proteins* 28(3):405-420, and a detailed description of HMMs can be found, for example, in Gribskov *et al.,* (1990) *Meth. Enzymol.* 183:146-159; Gribskov *et al.,* (1987) *Proc. Natl. Acad. Sci. USA* 84:4355-4358; Krogh *et al.,* (1994) *J. Mol. Biol.* 235:1501-1531; and Stultz *et al.,* (1993) *Protein Sci.* 2:305-314.

The SMART database of HMM's (Simple Modular Architecture Research Tool, http://smart.embl-heidelberg.de/; Schultz *et al.,* (1998) *Proc. Natl. Acad. Sci. USA* 95:5857 and Schultz *et al.,* (2000) *Nucl. Acids Res* 28:231) provides a catalog of zinc finger domains (ZnF_C2H2; ZnF_C2C2; ZnF_C2HC; ZnF_C3H1; ZnF_C4; ZnF_CHCC; ZnF_GATA; and ZnF_NFX) identified by profiling with the hidden Markov models of the HMMer2 search program (Durbin *et al.,* (1998) *Biological sequence analysis: probabilistic models of proteins and nucleic acids.* Cambridge University Press.; http://hmmer.wustl.edun.

**Hybridization-based Methods.** A collection of nucleic acids encoding various forms of a DNA binding domain can be analyzed to profile sequences encoding conserved amino- and carboxy-terminal boundary sequences. Degenerate oligonucleotides can be designed to hybridize to sequences encoding such conserved boundary sequences. Moreover, the efficacy of such degenerate oligonucleotides can be estimated by comparing their composition to the frequency of possible annealing sites in known genomic sequences. Multiple rounds of design can be used to optimize the degenerate oligonucleotides. For example, comparison of known Cys₂-His₂ zinc fingers revealed a common sequence in the linker region between adjacent fingers in natural sequence (Agata *et al.,* (1998) *Gene* 213:55-64). Such degenerate oligonucleotides are used to amplify a plurality of DNA binding domains. The amplified domains are inserted as test zinc finger domains into the hybrid nucleic acid, and subsequently assayed for binding to a target site by the methods described herein.

### Library Design

The method permits the screening of a collection of nucleic acids encoding DNA binding domains (for example, in the form of a plasmid, phagemid, or phage library) for functional nucleic acid binding properties. The collection can encode a diverse group of DNA binding domains, even domains of different structural folds. In one instance, the collection encodes domains of a single structural fold such as a zinc finger domain. Although the following methods are described in the context of zinc finger domains, one skilled in the art would be able to adapt them to other types of nucleic acid binding domains.

**Mutated Domains.** In still another instance, the collection is composed of nucleic acids encoding a structural domain that is assembled from a degenerate patterned library. For example, in the instance of zinc fingers, an alignment of known zinc fingers can be utilized to identify the optimal amino acids at each position. Alternatively, structural studies and mutagenesis experiments can be used to determine the preferred properties of amino acids at each position. Any nucleic acid binding domain can be used as a structural scaffold for introducing mutations. In particular, positions in close proximity to the nucleic acid binding interface or adjacent to a position so located can be targeted for mutagenesis. A mutated test zinc finger domain can be constrained at any mutated position to a subset of possible amino acids by using a patterned degenerate library. Degenerate codon sets can be used to encode the profile at each position. For example, codon sets are available that encode only hydrophobic residues, aliphatic residues, or hydrophilic residues. The library can be selected for full-length clones that encode folded polypeptides. Cho *et al.* ((2000) *J. Mol. Biol.* 297(2):309-19) provides a method for producing such degenerate libraries using degenerate oligonucleotides, and also provides a method of selecting library nucleic acids that encode full-length polypeptides. Such nucleic acids can be easily inserted into an expression plasmid using convenient restriction enzyme cleavage sites or transposase or recombinase recognition sites for the selection methods described herein.

Selection of the appropriate codons and the relative proportions of each nucleotide at a given position can be determined by simple examination of a table representing the genetic code, or by computational algorithms. For example, Cho *et al., supra,* describe a computer program that accepts a desired degenerate protein sequence and outputs a preferred oligonucleotide design that encodes the sequence.

**Isolation of a natural repertoire of domains.** A library of domains can be constructed from genomic DNA or cDNA of eukaryotic organisms such as humans. Multiple methods are available for doing this. For example, a computer search of available amino acid sequences can be used to identify the domains, as described above. A nucleic acid encoding each domain can be isolated and inserted into a vector appropriate for the expression in cells, e.g., a vector containing a promoter, an activation domain, and a selectable marker. In another example, degenerate oligonucleotides that hybridize to a conserved motif are used to amplify, e.g., by PCR, a large number of related domains containing the motif. For example, Kruppel-like Cys,His, zinc fingers can be amplified by the method of Agata *et al.,* (1998) *Gene* 213:55-64. This method also maintains the naturally occurring zinc finger domain linker peptide sequences, e.g., sequences with the pattern: Thr-Gly-(Glu/Gln)-(Lys/Arg)-Pro-(Tyr/Phe) (SEQ ID NO:78). Moreover, screening a collection limited to domains of interest, unlike screening a library of unselected genomic or cDNA sequences, significantly decreases library complexity and reduces the likelihood of missing a desirable sequence due to the inherent difficulty of completely screening large libraries.

The human genome contains numerous zinc finger domains, many of which are uncharacterized and unidentified. It is estimated that there are thousands of genes encoding proteins with zinc finger domains (Pellegrino and Berg, (1991) *Proc. Natl. Acad. Sci. USA* 88:671-675). These human zinc finger domains represent an extensive collection of diverse domains from which novel DNA-binding proteins can be constructed. If each zinc finger domain recognizes a unique 3- to 4-bp sequence, the total number of domains required to bind every possible 3- to 4-bp sequence is only 64 to 256 (4³ to 4⁴). It is possible that the natural repertoire of the human genome contains a sufficient number of unique zinc finger domains to span all possible recognition sites. These zinc finger domains are a valuable resource for constructing artificial chimeric DNA-binding proteins. Naturally occurring zinc finger domains, unlike artificial mutants derived from the human genome, have evolved under natural selective pressures and therefore may be naturally optimized for binding specific DNA sequences and *in vivo* function.

Human zinc finger domains are much less likely to induce an immune response when introduced into humans, e.g., in gene therapy applications.

### In vivo Selection of Zinc Finger Domains Possessing Specific DNA Binding Properties

Zinc finger domains with desired DNA recognition properties can be identified using the following *in vivo* screening system. A composite binding site of interest is inserted upstream of a reporter gene such that recruitment of a transcriptional activation domain to the composite binding site results in increased reporter gene transcription above a given level. An expression plasmid that encodes a hybrid protein consisting of a test zinc finger domain fused to a fixed DNA binding domain and a transcriptional activation domain is constructed.

The composite binding site includes at least two elements, a recruitment site and a target site. The system is engineered such that the fixed DNA binding domain recognizes the recruitment site. However, the binding affinity of the fixed DNA binding domain for the recruitment site is such that *in vivo* it alone is insufficient for transcriptional activation of the reporter gene. This can be verified by a control experiment.

For example, when expressed in cells, the fixed DNA binding domain (in the absence of a test zinc finger domain, or in the presence of a test zinc finger domain that is known to be nonfunctional or whose known DNA contacting residues have been replaced with an alternative amino acid such as alanine) should not be able to activate transcription of the reporter gene above a nominal level. Some leaky or low-level activation is tolerable, as the system can be sensitized by other means (e.g., by use of a competitive inhibitor for the reporter). The fixed DNA binding domain is expected not to bind stably to the recruitment site. For example, the fixed DNA binding domain can bind to the recruitment site with a dissociation constant (K_{d}) of approximately 0.1 nM, 1 nM, 1 µM, 10 µM, 100 µM, or greater. The K_{d} of the DNA binding domain for the target site can be measured *in vitro* by an electrophoretic mobility shift assay (EMSA) in the absence of a test zinc finger domain or in the absence of a test zinc finger domain with specificity for the second target site.

Thus, attachment of a functional test zinc finger domain that recognizes the target site, e.g., the variable site of the composite binding site, is necessary for the hybrid protein to bind stably to the composite binding site in cells, and thereby to activate the reporter gene. The binding preference of the test zinc finger domain for the target site results in an increase in reporter gene expression relative to the given level. For example, the fold increase of reporter gene expression obtained by dividing the observed level by the given level can be approximately v 2, 4, 8, 20, 50, 100, 1000 fold or greater. When the test zinc finger domain recognizes the target site, the K_{d} of the transcription factor comprising the DNA binding domain and the test zinc finger domain is decreased, e.g., relative to a transcription factor lacking a test zinc finger domain with specificity for the target site. For example, the dissociation constant (K_{d}) of a transcription factor complexed to a target site for which it has specificity can be approximately 50 nM, 10 nM, 1 nM, 0.1 nM, 0.01 nM or less. The K_{d} can be determined *in vitro* by EMSA.

The discovery that DNA binding specificity can be sensitively and accurately assayed by determining the ability of test zinc finger domains to augment the *in vivo* binding affinity of a fixed DNA binding domain has enabled the rapid isolation and characterization of novel zinc finger domains from the human genome.

Fixed DNA binding domains include modular domains isolated from naturally occurring DNA-binding proteins, e.g., a naturally occurring DNA-binding protein that has multiple domains or that is an oligomer. For example, both of two known zinc fingers, e.g., fingers 1 and 2 of Zif268, can be used as the fixed DNA binding domain. A skilled artisan would be able to identify from the myriad of nucleic acid binding domains (e.g., a domain family described herein, such as a homeodomain, a helix-turn-helix domain, or a helix-loop-helix domain, or a nucleic acid binding domain well characterized in the art) a fixed DNA binding domain suitable for the system. Appropriate selection of a recruitment site that is recognized by the fixed DNA binding domain is also necessary. The recruitment site can be a subsite within the natural binding site for the naturally occurring DNA binding protein from which the fixed DNA binding domain is obtained. If necessary, mutations can be introduced either into the fixed domain or into the recruitment site, in order to sensitize the system.

Cells suitable for the *in vivo* screening system include both eukaryotic and prokaryotic cells. Exemplary eukaryotic cells include yeast cells, e.g., *Saccharomyces cerevisiae, Saccharomyces pombe,* and *Pichia pastoris* cells.

The yeast one-hybrid system, using *Saccharomyces cerevisiae,* was modified to select zinc finger domains using the aforementioned screening system. First, reporter plasmids that encode the *HIS3* reporter gene were prepared. The predetermined 4-bp target DNA sequences were connected to a truncated binding sequence to provide composite binding sequences for the DNA-binding domains, and each of the composite binding sequences was operably linked to the reporter gene on separate plasmids.

The hybrid nucleic acid sequence encodes a transcriptional activation domain linked to a DNA-binding domain comprising a truncated DNA-binding domain and a zinc finger domain.

The binding sites used herein are not necessarily contiguous, although contiguous sites are frequently used. Flexible and/or extensible linkers between nucleic acid binding domains can be used to construct proteins that recognize non-contiguous sites.

According to one aspect of the present invention, a polypeptide composed of finger 1 and finger 2 of Zif268 and devoid of finger 3 can be used as a fixed DNA-binding domain. (Among the three zinc finger domains of Zif268, finger 1 refers to the zinc finger domain located at the N-terminal end, finger 2, the zinc finger domain in the middle, and finger 3 the zinc finger domain at the C-terminal end.) Alternately, any two zinc finger domains whose binding site is characterized can be used as a fixed DNA-binding domain.

Other useful fixed DNA-binding domains may be derived from other zinc finger proteins, such as Sp1, CF2-II, YY1, Kruppel, WT1, Egr2, or POU-domain proteins, such as Oct1, Oct2, and Pit1. These are provided by way of example and the present invention is not limited thereto.

According to one particular example of the present invention, the base sequence of 5'-GGGCG-3', generated by deleting 4-bp from the 5' end of the optimal Zif268 recognition sequence (5'-GCG TGG GCG-3), can be used as a recruitment site. Any target sequence of 3 to 4 bp can be linked to this recruitment site, to yield a composite binding sequence.

Activation domains. Transcriptional activation domains that may be used in the present invention include but are not limited to the Gal4 activation domain from yeast and the VP16 domain from herpes simplex virus. In bacteria, activation domain function can be emulated by fusing a domain that can recruit a wild-type RNA polymerase alpha subunit C-terminal domain or a mutant alpha subunit C-terminal domain, e.g., a C-terminal domain fused to a protein interaction domain.

**Repression domains.** If desired, a repression domain instead of an activation domain can be fused to the DNA binding domain. Examples of eukaryotic repression domains include ORANGE, groucho, and WRPW (Dawson *el al.,* (1995) *Mol. Cell Biol.* 15:6923-31). When a repression domain is used, a toxic reporter gene and/or a non-selectable marker can be used to screen for decreased expression.

**Reporter genes.** The reporter gene can be a selectable marker, e.g., a gene that confers drug resistance or an auxotrophic marker. Examples of drug resistance genes include *S*. *cerevisiae* cyclohexamide resistance (*CYH*), *S. cerevisiae* canavanine resistance gene (*CAN1*), and the hygromycin resistance gene. *S. cerevisiae* auxotrophic markers include the *URA3, HIS3, LEU2, ADE2* and *TRP1* genes. When an auxotrophic marker is the reporter gene, cells that lack a functional copy of the auxotrophic gene and so the ability to produce a particular metabolite are utilized. Selection for constructs encoding test zinc finger domains that bind a target site is achieved by maintaining the cells in medium lacking the metabolite. For example, the *HIS3* gene can be used as a selectable marker in combination with a *his3⁻* yeast strain. After introduction of constructs encoding the hybrid transcription factors, the cells are grown in the absence of histidine. Selectable markers for use in mammalian cells, such as thymidine kinase, neomycin resistance, and HPRT, are also well known to the skilled artisan.

Alternatively, the reporter gene encodes a protein whose presence can be easily detected and/or quantified. Exemplary reporter genes include *lacZ,* chloramphenicol acetyl transferase (CAT), luciferase, green fluorescent protein (GFP), beta-glucuronidase (GUS), blue fluorescent protein (BFP), and derivatives of GFP, e.g., with altered or enhanced fluorescent properties (Clontech Laboratories, Inc. CA). Colonies of cells expressing lacZ can be easily detected by growing the colonies on plates containing the colorimetric substrate X-gal. GFP expression can be detected by monitoring fluorescence emission upon excitation. Individual GFP expressing cells can be identified and isolated using fluorescence activated cell sorting (FACS).

The system can be constructed with two reporter genes, e.g., a selectable reporter gene and a non-selectable reporter gene. The selectable marker facilitates rapid identification of the domain of interest, as under the appropriate growth conditions, only cells bearing the domain of interest grow. The non-selectable reporter provides a means of verification, e.g., to distinguish false-positives, and a means of quantifying the extent of binding. The two reporters can be integrated at separate locations in the genome, integrated in tandem in the genome, contained on the same extrachromosomal element (e.g., plasmid) or contained on separate extrachromosomal elements.

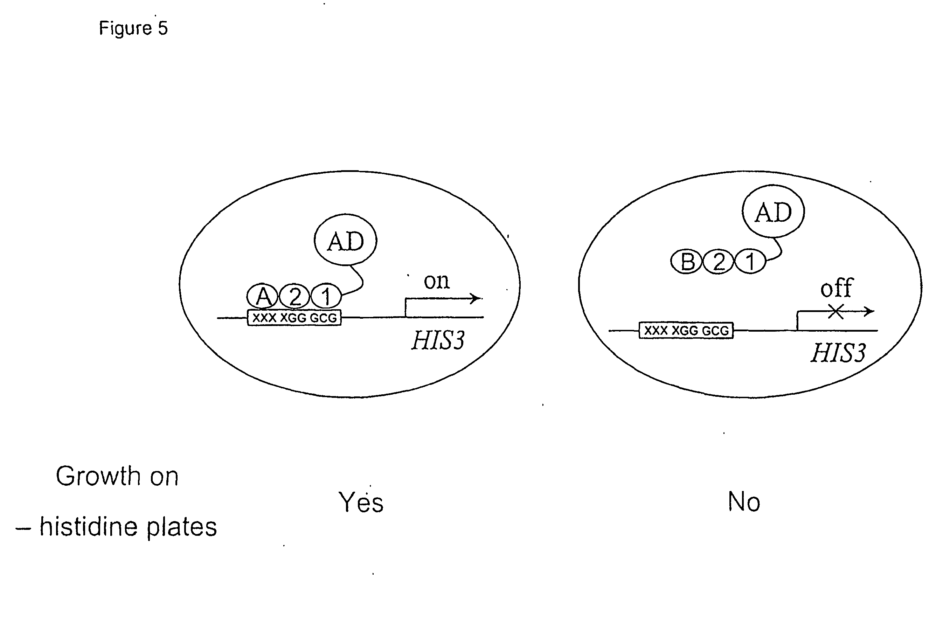

Fig. 5 illustrates the principle of the modified one-hybrid system used to select desired zinc finger domains. The DNA-binding domain of the hybrid transcription factor is composed of (a) a truncated DNA-binding domain consisting of finger 1 and finger 2 of Zif268 and (b) zinc finger domain A or B. The base sequence of the binding site located at the promoter region of the reporter gene is a composite binding sequence (5'-XXXXGGGCG-3'), which consists of a 4-bp target sequence (nucleotides 1 to 4, 5'-XXXX-3'), and a truncated binding sequence (nucleotides 5 to 9,5'-GGGCG-3').

If the test zinc finger domain (A in Fig. 5) in the hybrid transcription factor recognizes the target sequence, the hybrid transcription factor can bind the composite binding sequence stably. This stable binding leads to expression of the reporter gene through the action of the activation domain (AD in Fig. 5) of the hybrid transcription factor. As a result, when *HIS3* is used as a reporter gene, the transformed yeast grows in medium devoid of histidine. Alternatively, when *lac*Z is used as a reporter gene, the transformed yeast grows as a blue colony in a medium containing X-gal, a substrate of the *lac*Z protein. However, if the zinc finger domain (B in Fig. 5) of the hybrid transcription factor fails to recognize the target sequence, expression of the reporter gene is not induced. As a result, the transformed yeast cannot grow in the medium devoid of histidine (when *HIS3* is used as a reporter gene) or grows as a white colony in a medium containing X-gal (when *lacZ* is used as a reporter gene).

The selection method using this modified one-hybrid system is advantageous because zinc finger domains selected by virtue of this procedure are demonstrated to function in the cellular milieu. Thus, the domains are presumably able to fold, enter the nucleus, and withstand intracellular proteases and other potentially damaging intracellular agents. Furthermore, the modified one-hybrid system disclosed herein allows the isolation of desired zinc finger domains quickly and easily. The modified one-hybrid system requires only a single round of transformation of yeast cells to isolate the desired zinc finger domains.

The selection method described herein can be utilized to identify a zinc finger domain from a genome e.g., a genome of a plant or animal species (e.g., a mammal, e.g., a human). The method can also be utilized to identify a zinc finger domain from a library of mutant zinc finger domains prepared, for example, by random mutagenesis. In addition, the two methods can be used in conjunction. For example, if a zinc finger domain cannot be isolated from the human genome for a particular 3-bp or 4-bp DNA sequence, a library of zinc finger domains prepared by random or directed mutagenesis can be screened for such a domain.

Although the modified one-hybrid system in yeast is a preferred means to select zinc finger domains that recognize and bind the given target sequences, it will be apparent to a person skilled in the art that systems other than yeast one-hybrid selection can be used. For example, phage display selection may be used to screen a library of naturally occurring zinc finger domains derived from a genome of a eukaryotic organism.

The present invention encompasses the use of the one-hybrid method in a variety of cultured cells. For example, a reporter gene operably linked to target sequences may be introduced into prokaryotic or animal or plant cells in culture, and the cultured cells may then be transfected with plasmids, phages, or viruses encoding a library of zinc finger domains. Desired zinc finger domains recognizing target sequences may then be obtained from the isolated cells in which the reporter gene is activated.

The examples disclosed below demonstrate that the method can identify zinc finger domains for binding sites of interest. A library of hybrid transcription factors with a variety of zinc finger domains positioned at finger 3 was prepared. Of the novel zinc finger domains (e.g., HSNK, QSTV, and VSTR zinc fingers; see below) selected from the library, none is naturally located at the C-terminus in its corresponding parent zinc finger protein. This clearly demonstrates that zinc finger domains are modular and that novel DNA-binding domains can be constructed by mixing and matching appropriate zinc finger domains.

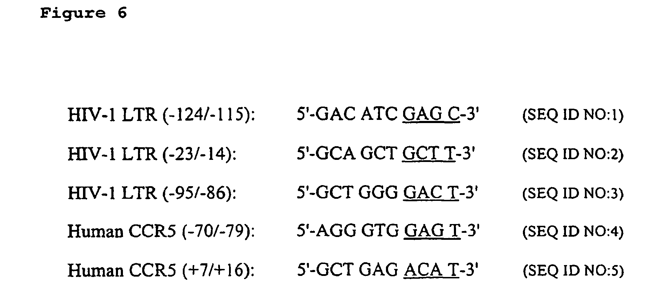

The zinc finger domains selected via the method of the present invention can be used as building blocks to make new DNA-binding proteins by appropriate rearrangement and recombination. For example, a novel DNA-binding protein recognizing the promoter region of human *CCR5,* a coreceptor of HIV-1, can be constructed as follows. The promoter region of human CCR5 contains the following 10-bp sequence: 5'-AGG GTG GAG T-3' (SEQ ID NO:4) (Fig. 6). Using the modified one-hybrid system disclosed herein, one should be able to isolate three zinc finger domains, each of which specifically recognizes one of the following 4-bp target sequences; 5'-AGGG-3', 5'-GTGG-3', and 5'-GAGT-3'. These target sequences are overlapping 4-bp segments of the *CCR5* target sequence. These three zinc finger domains can be connected with appropriate linkers and attached to a regulatory domain such as the VP16 domain and the GAL4 domain or repression domains such as the KRAB domain in order to generate novel transcription factors that specifically bind to the *CCR5* promoter. These zinc finger proteins could be used in gene therapy to help prevent proliferation of HIV-1.

### High Throughput Screening

The following method allows rapid measurement of the relative in *vivo* binding affinity for each domain in a collection for multiple possible DNA-binding sites or even all possible DNA-binding sites. A large collection of nucleic acids encoding nucleic acid binding domains is generated. Each nucleic acid binding domain is encoded as the test zinc finger domain in a hybrid nucleic acid construct, and expressed in a yeast strain of one mating type. Thus; a first set of yeast strains expressing all available or desired domains is generated. A second set of yeast strains containing reporter constructs for putative target sites for the domains in the reporter construct is constructed in the opposite mating type. The method requires performing many or all of the possible pairwise matings in order to create a matrix of fused cells, each having a different test zinc finger domain and a different target site reporter construct. Each fused cell is assayed for reporter gene expression. The method thereby rapidly and effortlessly determines the binding preferences of the tested domains.

A collection of domains is identified, e.g., by searching a genomics database for putative domains that fit a given profile. The collection can include, for example, ten to twenty domains, or all the identified domains, possibly thousands or more. Nucleic acids encoding the domains identified from the database are amplified using synthetic oligonucleotides. Manual and automated methods for designing such synthetic oligonucleotides are routine in the art. Nucleic acids encoding additional domains can be amplified with degenerate primers. Nucleic acids encoding the domains of the collection are cloned into the yeast expression plasmid described above, thus creating fusion proteins of the domains and the first two fingers of Zif268 and a transcription activation domain. The amplification and cloning steps can be done in a microtitre plate format in order to clone nucleic acids encoding the multiple domains.

Alternatively, a recombinational cloning method can be used to rapidly insert multiple amplified nucleic acids encoding the domains into the yeast expression vector. This method, which is described in U.S. Patent No. 5,888,732 and the "Gateway" manual (Life Technologies-Invitrogen, CA, USA), entails including customized sites for a site-specific recombinase at the ends of the amplification primers. The expression vector contains an additional site or sites at the position for insertion of amplified nucleic acid encoding the domain. These sites are designed to lack stop codons. Addition of the amplification product, the expression vector, and the site-specific recombinase to the recombination reaction results in insertion of the amplified sequence into the vector. Additional features, e.g., the displacement of a toxic gene upon successful insertion, make this method highly efficient and suitable for high throughput cloning.

Restriction enzyme-mediated and/or recombination cloning can be used to insert nucleic acids encoding each of the identified domains into an expression vector. The vectors can be propagated in bacteria, and frozen in indexed microtitre plates, such that each well contains a cell harboring a nucleic acid encoding one of the different, unique DNA-binding domains.

Isolated plasmid DNA is obtained for each domain and transformed into a yeast cell, e.g., a *Saccharomyces cerevisiae MATa* cell. As the expression vector contains a selectable marker, the transformed cells are grown in minimal medium under nutritional conditions selecting for the marker. Such cells can also be frozen and stored, e.g., in microtitre plates, for later use.

A second set of yeast strains is constructed, e.g., in a *Saccharomyces cerevisiae MATa* cell. This set of yeast strains contains a variety of different reporter vectors. Each yeast strain bearing an expression vector with a unique DNA-binding domain is then mated to each yeast strain of the reporter gene set. As these two strains are from opposite mating types and are engineered to have different auxotrophies, diploids can easily be selected. Such diploids have both the reporter and the expression plasmids. The cells are also maintained under nutritional conditions that select for both the reporter and the expression plasmids. Uetz *et al.* (2000) *Nature* 403:623-7 describe a complete two-hybrid map of all yeast proteins by generating such a matrix of yeast matings.

Reporter gene expression can be detected in a high-volume format, e.g., in microtitre plates. For example, when using GFP as the reporter, a plate containing the matrix of mated cells can be scanned for fluorescence.

### Modular Assembly of Novel DNA-Binding Proteins

A new DNA-binding protein can be rationally constructed to recognize a target 9-bp or longer DNA sequence by mixing and matching appropriate zinc finger domains. The modular structure of zinc finger domains facilitates their rearrangement to construct new DNA-binding proteins. As shown in Fig. 1, zinc finger domains in the naturally-occurring Zif268 protein are positioned tandemly along the DNA double helix. Each domain independently recognizes a different 3-4 bp DNA segment.

**A database of zinc finger domains.** The one-hybrid selection system described above can be utilized to identify one or more zinc finger domains for each possible 3 or 4 basepair binding site. The results can be stored as a matrix or database, e.g., a relational database. The database can include an indication of the relative affinity of the zinc finger domains that bind each site.

Such zinc finger domains can also be tested in the context of multiple different fusion proteins to verify their specificity. Moreover, particular binding sites for which a paucity of domains is available can be the target of additional selection screens. Libraries for such selections can be prepared by mutagenizing a zinc finger domain that binds a similar yet distinct site. A complete matrix of zinc finger domains for each possible binding site is not essential, as the domains can be staggered relative to the target binding site in order to best utilize the domains available. Such staggering can be accomplished both by parsing the binding site in the most useful 3 or 4 basepair binding sites, and also by varying the linker length between zinc finger domains. In order to incorporate both selectivity and high affinity into the design polypeptide, zinc finger domains that have high specificity for a desired site can be flanked by other domains that bind with higher affinity, but lesser specificity. The *in vivo* screening method described herein can used to test the *in vivo* function, affinity, and specificity of an artificially assembled zinc finger protein and derivatives thereof. Likewise, the method can be used to optimize such assembled proteins, e.g., by creating libraries of varied linker composition, zinc finger domain modules, zinc finger domain compositions, and so forth.

**Parsing a target site.** The target 9-bp or longer DNA sequence is parsed into 3 or 4 bp segments. Zinc finger domains are identified (e.g.; from a database described above) that recognize each parsed 3 or 4 bp segment. Longer target sequences, e.g., 20 bp to 500 bp sequences, are also suitable targets as 9 bp, 12 bp, and 15 bp subsequences can be identified within them. In particular, subsequences amenable for parsing into sites well represented in the database can serves as initial design targets.

**Constructing Assembled Modules.** Polypeptide sequences are designed to contain multiple zinc finger domains that recognize adjacent 3 or 4 bp subsites, or nearby subsites. A nucleic acid sequence encoding the designed polypeptide sequence can be synthesized. Methods for constructing synthetic genes are routine in the art. Such methods include gene construction from custom synthesized oligonucleotides, PCR mediated cloning, and mega-primer PCR. Multiple nucleic acid sequences can be synthesized, e.g., to form a library. For example, the library nucleic acids can be designed such that the sequences encoding a domain at any given position vary such that they encode different zinc finger domains-whose recognition specificity is suitable for that position. Sexual PCR and "DNA Shuffling™" (Maxygen, Inc., CA) can be used to vary the identity of zinc finger domains at each position.

**Peptide Linkers.** DNA binding domains can be connected by a variety of linkers. The utility and design of linkers are well known in the art. A particularly useful linker is a peptide linker that is encoded by nucleic acid. Thus, one can construct a synthetic gene that encodes a first DNA binding domain, the peptide linker, and a second DNA binding domain. This design can be repeated in order to construct large, synthetic, multi-domain DNA binding proteins. PCT WO 99/45132 and Kim and Pabo ((1998) *Proc. Natl. Acad. Sci. USA* 95:2812-7) describe the design of peptide linkers suitable for joining zinc finger domains.

Additional peptide linkers are available that form random coil, α-helical or β-pleated tertiary structures. Polypeptides that form suitable flexible linkers are well known in the art (see, e.g., Robinson and Sauer (1998) *Proc Natl Acad Sci USA.* 95:5929-34). Flexible linkers typically include glycine, because this amino acid, which lacks a side chain, is unique in its rotational freedom. Serine or threonine can be interspersed in the linker to increase hydrophilicity. In additional, amino acids capable of interacting with the phosphate backbone of DNA can be utilized in order to increase binding affinity. Judicious use of such amino acids allows for balancing increases in affinity with loss of sequence specificity. If a rigid extension is desirable as a linker, α-helical linkers, such as the helical linker described in Pantoliano *et al.* (1991) *Biochem.* 30:10117-10125, can be used. Linkers can also be designed by computer modeling (see, e.g., U.S. Pat. No. 4,946,778). Software for molecular modeling is commercially available (e.g., from Molecular Simulations, Inc., San Diego, CA). The linker is optionally optimized, e.g., to reduce antigenicity and/or to increase stability, using standard mutagenesis techniques and appropriate biophysical tests as practiced in the art of protein engineering, and functional assays as described herein.

For implementations utilizing zinc finger domains, the peptide that occurs naturally between zinc fingers can be used as a linker to join fingers together. A typical such naturally occurring linker is: Thr-Gly-(Glu or Gln)-(Lys or Arg)-Pro-(Tyr or Phe) (SEQ ID NO:78) (Agata *et al., supra*).

**Dimerization Domains.** An alternative method of linking DNA binding domains is the use of dimerization domains, especially heterodimerization domains (see, e.g., Pomerantz et al (1998) *Biochemistry* 37:965-970). In this implementation, DNA binding domains are present in separate polypeptide chains. For example, a first polypeptide encodes DNA binding domain A, linker, and domain B, while a second polypeptide encodes domain C, linker, and domain D. An artisan can select a dimerization domain from the many well-characterized dimerization domains. Domains that favor heterodimerization can be used if homodimers are not desired. A particularly adaptable dimerization domain is the coiled-coil motif, e.g., a dimeric parallel or anti-parallel coiled-coil. Coiled-coil sequences that preferentially form heterodimers are also available (Lumb and Kim, (1995) *Biochemistry* 34:8642-8648). Another species of dimerization domain is one in which dimerization is triggered by a small molecule or by a signaling event. For example, a dimeric form of FK506 can be used to dimerize two FK506 binding protein (FKBP) domains. Such dimerization domains can be utilized to provide additional levels of regulation.

### Functional Assays and Uses

In addition to biochemical assays, the function of a nucleic acid binding domain or a protein designed by a method described herein, e.g., by modular assembly, can be assayed or used *in vivo.* For example, domains can be selected to bind to a target site, e.g., to a promoter site of a gene required for cell proliferation. By modular assembly, a protein can be designed that includes (1) the selected domains that respectively bind to subsites spanning the target promoter site, and (2) a DNA repression domain, e.g., a WRPW domain.

A nucleic acid sequence encoding a designed protein can be cloned into an expression vector, e.g., an inducible expression vector as described in Kang and Kim, (2000) *J Biol Chem* 275:8742. The inducible expression vector can include an inducible promoter or regulatory sequence. Non-limiting examples of inducible promoters include steroid-hormone responsive promoters (e.g., ecdysone-responsive, estrogen-responsive, and glutacorticoid-responsive promoters), the tetracyclin "Tet-On" and "Tet-Off' systems, and metal-responsive promoters. The construct can be transfected into tissue culture cells or into embryonic stem cells to generate a transgenic organism as a model subject. The efficacy of the designed protein can be determined by inducing expression of the protein and assaying cell proliferation of the tissue culture cell or assaying for developmental changes and/or tumor growth in a transgenic animal model. In addition, the level of expression of the gene being targeted can be assayed by routine methods to detect mRNA, e.g., RT-PCR or Northern blots. A more complete diagnostic includes purifying mRNA from cells expressing and not expressing the designed protein. The two pools of mRNA are used to probe a microarray containing probes to a large collection of genes, e.g., a collection of genes relevant to the condition of interest (e.g., cancer) or a collection of genes identified in the organism's genome. Such an assay is particularly valuable for determining the specificity of the designed protein. If the protein binds with high affinity but little specificity, it may cause pleiotropic and undesirable effects by affecting expression of genes in addition to the contemplated target. Such effects are revealed by a global analysis of transcripts.

In addition, the designed protein can be produced in a subject cell or subject organism in order to regulate an endogenous gene. The designed protein is configured, as described above, to bind to a region of the endogenous gene and to provide a transcriptional activation or repression function. As described in Kang and Kim (*supra*), the expression of a nucleic acid encoding the designed protein can be operably linked to an inducible promoter. By modulating the concentration of the inducer for the promoter, the expression of the endogenous gene can be regulated in a concentration dependent manner.

### Assaying binding site preference

The binding site preference of each domain can be verified by a biochemical assay such as EMSA, DNase footprinting, surface plasmon resonance, or column binding. The substrate for binding can be a synthetic oligonucleotide encompassing the target site. The assay can also include non-specific DNA as a competitor, or specific DNA sequences as a competitor. Specific competitor DNAs can include the recognition site with one, two, or three nucleotide mutations. Thus, a biochemical assay can be used to measure not only the affinity of a domain for a given site, but also its affinity to the site relative to other sites. Rebar and Pabo, (1994) *Science* 263:671-673 describe a method of obtaining apparent K_{d} constants for zinc finger domains from EMSA.

The present invention will be described in more detail through the following practical examples. However, it should be noted that these examples are not intended to limit the scope of the present invention.

### Example 1: Construction of plasmids for hybrid transcription factor expression.

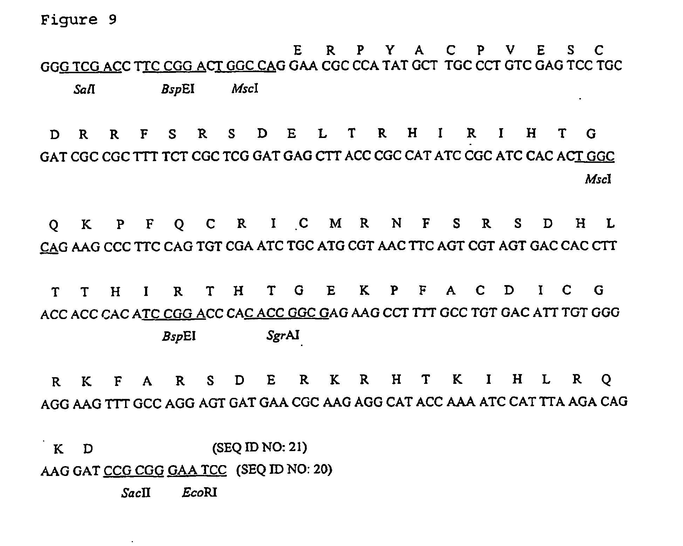

An expression plasmid expressing a zinc finger transcription factor was prepared by modification of pPC86 (Chevray and Nathans, (1991) *Proc. Natl. Acad. Sci. USA* 89:5789-5793). Manipulations of DNA were performed as described in Ausubel *et al.* (Current Protocols in Molecular Biology (1998), John Wiley and Sons, Inc.). A DNA fragment encoding Zif268 zinc finger protein was inserted between the *Sal*I and *Eco*RI recognition sites of pPC86 to generate pPCFM-Zif. The result-of this cloning step is a translational fusion protein encoding the yeast Gal4 activation domain followed by the three Zif268 zinc fingers. Transformation of pPCFM-Zif into yeast cells results in expression of a hybrid transcription factor comprising the yeast Gal4 activation domain and the Zif268 zinc fingers. The DNA sequence encoding the Zif268 zinc finger protein as cloned in pPCFM-Zif is shown in Fig. 9.

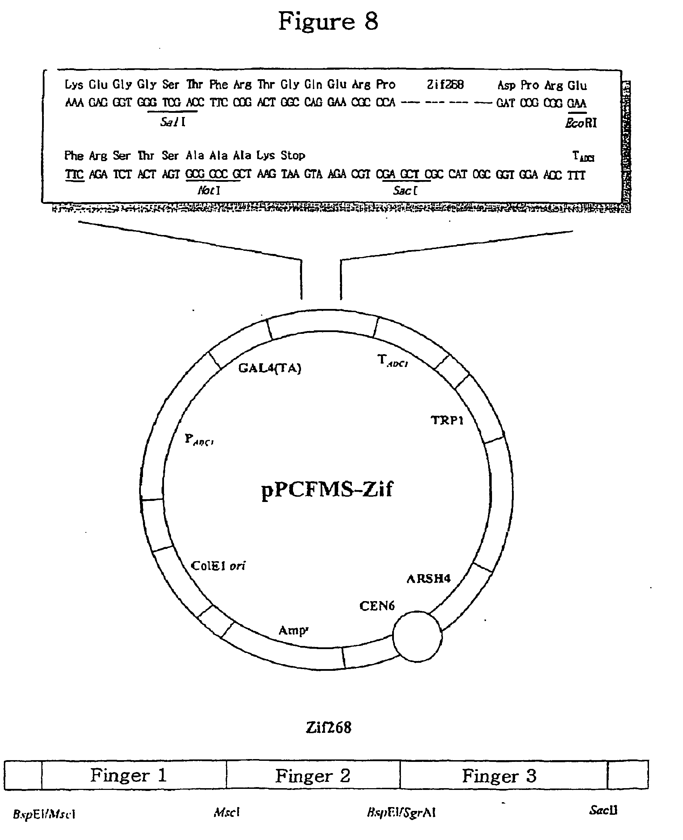

The plasmid pPCFMS-Zif was utilized as a vector for constructing libraries of zinc finger domains (Fig. 8). pPCFMS-Zif was constructed by insertion of an oligonucleotide cassette containing a stop codon and a *Pst*I recognition site in front of the finger 3 coding region of pPCFM-Zif. The oligonucleotide cassette was formed by annealing two synthetic oligonucleotides: 5'-TGCCTGCAGCATTTGTGGGAGGAAGTTTG-3' (SEQ ID NO:79); and 5'-ATGCTGCAGGCTTAAGGCTTCTCGCCGGTG-3'(SEQ ID NO:80). The insertion of a stop codon prevents the generation of library plasmids encoding finger 3 of Zif268.

The plasmid was used as a vector for the generation of zinc finger domain libraries as described in "Example 2" below.

In addition, gap repair cloning of DNA sequences encoding individual zinc finger domains was carried out as described in Hudson *et al.,* ((1997) *Genome Research* 7:1169-1173) with minor modification.

To clone an individual zinc finger domain, two overlapping oligonucleotides were synthesized. Each oligonucleotide included a 21-nucleotide-long common tail at its 5' end for second round PCR (rePCR) and a specific sequence that annealed to the nucleic acid encoding the individual zinc finger domain. The sequences of the forward and back primers were 5'-ACCCACACTGGCCAGAAACCCN₄₈₋₅₁- 3' (SEQ ID NO:108) and 5'-GATCTGAATTCATTCACCGGTN₄₂₋₄₅-3' (SEQ ID NO:109), respectively, where N₄₈₋₅₁ and N₄₂₋₄₅ correspond to the customized sequence for annealing to the nucleic acid encoding the zinc finger domain. Double stranded DNA was prepared by amplifying template nucleic acid with an equimolar misture of two oligonucleotides. PCR conditions consisted of a first cycle at 94°C for 3 minutes followed by 5 cycles of 94°C for 1 minutes , 50°C for 1 minutes, and 72°C for 30 seconds.

The double stranded DNA encoding each zinc finger domain was then used as a template in second round PCR. The rePCR primers had two regions, one region that is identical to yeast vector pPCFM-Zif and a second region that is identical to the 21-nucleotide-long common tail sequence described above. The sequence of forward primer was
5'-TGTCGAATCTGCATGCGTAACTTCAGTCGTAGTGACCACCTTACCACCCA C ATCCGGACCCACACTGGCCAGAAACCC-3' (SEQ ID NO: 138) and that of reverse primer was
5'-GGTGGCGGCCGTTACTTACTTAGAGCTCGACGTCTTACTTACTTAGC GGCCGCACTAGTAGATCTGAATTCATTCACCGGT - 3' (SEQ ID NO: 139). The reaction mixture contained 2.5 pmoles of each primer, 1.5mM Mg²⁺, 2 units of *Taq* polymerase and 0.01 units of *Pfu* polymerase in 25 ul. Reactions were carried out at 94°C for 3 min, then cycled through 20 cycles of 94°C for 1 min, 65°C for 1 min, and 72°C for 30 sec. Gap repair cloning was performed by transforming the mixture of rePCR products and linearized pPCFM-Zif vector that had been digested with *Msc*I and *Eco*RI into yeast YW1 cells. The region identical to the yeast vector pPCFM-Zif allows for homologous recombination with the vector in cells.

### Example 2: Construction of Zinc Finger Domain Library

A plasmid library of naturally occurring zinc finger domains was prepared by cloning zinc finger domains from the human genome. DNA segments encoding zinc finger domains were amplified from template human genomic DNA (purchased from Promega Corporation, Madison, WI, USA) using PCR and degenerate oligonucleotide primers. The DNA sequences of the degenerate PCR primers used to clone human zinc finger domains were as follows; 5'- GCGTCCGGACNCAYACNGGNSARA -3' (SEQ ID NO:81) and 5'- CGGAATTCANNBRWANGGYYTYTC -3' (SEQ ID NO:82), wherein R represent G and A; B represents G, C, and T; S represents G and C; W represents A and T; Y represents C and T; and N represents A, C, G, and T.

The degenerate PCR primers anneal to nucleic acid sequences coding for an amino acid profile, His-Thr-Gly-(Glu or Gln)-(Lys or Arg)-Pro-(Tyr or Phe) (SEQ ID NO:83), that is found at the junction between zinc finger domains in many naturally occurring zinc finger proteins (Agata *et al.* (1998) *Gene* 213:55-64).

The buffer composition of the PCR reaction was 50 mM KCl, 3 mM MgCl₂, 10 mM Tris pH 8.3. Taq DNA polymerase was added and the reaction mixture was incubated at 94°C for 30 seconds, at 42°C for 60 seconds, and then at 72°C for 30 seconds. This cycle was repeated 35 times, and was followed by a final incubation at 72°C for 10 minutes.

The PCR products were cloned into pPCFMS-Zif as follows: The PCR products were electrophoresed, and the DNA segments corresponding to about 120 bp were isolated. After digestion with *Bsp*EI and *Eco*RI, the 120-bp DNA segments were ligated into pPCFMS-Zif. As a result, the DNA-binding domain of the hybrid transcription factor encoded by this plasmid library consists of finger 1 and finger 2 of Zif268 and a zinc finger domain derived from the human genome.The plasmid library was prepared from a total of 10⁶ *Escherichia coli* transformants. This library construction scheme retains the naturally occurring linker sequence found between zinc finger domains.

### Example 3: Construction of Zinc Finger Domain Library

A library of mutant zinc finger domains was prepared by random mutagenesis. Finger 3 of Zif26.8 was used as a polypeptide framework. Random mutations were introduced at positions -1, 2, 3, 4, 5, and 6 along the α-helix, corresponding respectively to the arginine at position 73, aspartic acid at position 75, glutamic acid at position 76, arginine at position 77, lysine at position 78, and arginine at position 79 of SEQ ID NO:21 (within finger 3 of Zif268).

At each of the nucleic acid sequence positions encoding these amino acids, a randomized codon, 5'-(G/A/C) (G/A/C/T) (G/C)-3, was introduced. This randomized codon encodes any one of 16 amino acids (excluding four amino acids: tryptophan, tyrosine, cysteine and phenylalanine). Also excluded are all three possible stop codons. The randomized codons were introduced with an oligonucleotide cassette constructed from two oligonucleotides:
5'-GGGCCCGGGGAGAAGCCTTACGCATGTCCAGTCGAATCTTGTGAT AGAAGATTC-3' (SEQ ID NO:84); and
5'-CTCCCCGCGGTTCGCCGGTGTGGATTCTGATATGSNBSNBAAGSNB SNBSNBSNBTGAGAATCTTCTATCACAAG-3' (SEQ ID NO:85), wherein B represents G, T, and C; S represents G and C; and N represents A, G, C, and T.

After annealing these two oligonucleotides, the DNA duplex cassette was synthesized by reaction with Klenow polymerase for 30 minutes. After digestion with *Ava*I and *Sac*II*,* the DNA duplex was ligated into pPCFMS-Zif digested with *Sgr*AI and *Sac*II. Plasmids were isolated from about 10⁹ *E. coli* transformants.

### Example 4: Construction of reporter plasmids

Reporter plasmids including the yeast *HIS3* gene were prepared by modification ofpRS315His (Wang and Reed (1993) *Nature* 364:121-126). The reporter plasmids also contain the *LEU2* marker under its natural promoter for the purpose of selecting transformants bearing the plasmid. First, the *Sal*I recognition site in pRS315His was removed by ligating the small fragment of pRS31 SHis after digestion with *Sal*I and *Bam*HI and the large fragment of pRS315His after digestion with *Bam*HI and *Xho*I to make pRS315HisΔ Sal. Next, a new *Sal*I recognition site was created within the promoter region of the *HIS3* gene by inserting an oligonucleotide duplex into pRS315HisΔ Sal between the *Bam*HI and *Sma*I site. The sequences of the two oligonucleotides that were annealed to produce the inserted duplex are
_{5'}-_{CTA}G_{A}C_{C}CGGGA_{A}TTCGTCGACG-3'' (SEQ ID NO:86); and
5'-GATCCGTCGACGAATTCCCGGGT-3' (SEQ ID NO:87). The resulting plasmid was named pRS315HisMCS.

Multiple reporter plasmids were constructed by inserting desired composite sequences into pRS315HisMCS. The composite sequences are inserted as a tandem array containing four copies of the composite sequence. The target sequences were derived from 10-bp DNA sequences (Fig. 6) found in the LTR region of HIV-1 :

| | |
|---|---|
| 5'-GAC ATC GAG C-3' (SEQ ID NO:1) | HIV-1 LTR (-124/-115) |
| 5'-GCA GCT GCT T-3' (SEQ ID NO:2) | HIV-1 LTR (-23/-14) |
| 5'-GCT GGG GAC T-3' (SEQ ID NO:3) | HIV-1 LTR (-95/-86)) |

and in the promoter of human *CCR5* gene:

| | |
|---|---|
| 5'-AGG GTG GAGT-3'(SEQ ID NO:4) | human CCR5 (-70/-79) |
| 5'-GCT GAG ACA T-3' (SEQ ID NO:5) | human CCR5 (+7/+16)). |

Each of these 10-bp DNA sequence can be parsed into component 4-bp target sites in order to identify a zinc finger domain that recognizes each region of the site. Using the modular assembly method, such zinc finger domains can be coupled to produce a DNA binding protein that recognizes the site *in vivo.*

The underlined portions in Fig. 6 depict examples of 4-bp target sequences. Each of these 4-bp target sequences was connected to the 5-bp recruitment sequence, 5'-GGGCG-3', that is recognized by finger 1 and finger 2 of Zif268. The resulting 9-bp sequences constitute composite binding sequences. Each composite binding sequence has the following format:
5'-XXXXGGGCG-3', where XXXX is the 4-bp target sequence and the adjacent 5'-GGGCG-3' is the recruitment sequence.

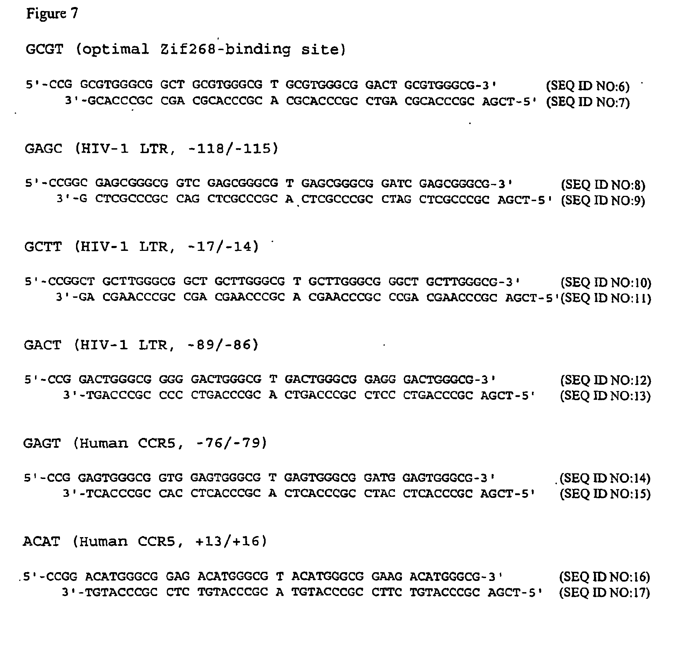

Fig. 7 recites the DNA sequences of the inserted tandem arrays of composite binding sites, each of which was operably linked to the reporter gene in pRS315HisMCS. Each tandem array contains 4 copies of a composite binding sequence. For each binding site, two oligonucleotides were synthesized, annealed and ligated into pRS315HisMCS restricted with *Sal*I and *Xma*I site to make a reporter plasmid.

### Example 5: Construction of reporter plasmids

A set of reporter plasmids that includes a pair of reporters (one having *lacZ,* the other having *HIS3*) for each 3 basepair subsite was constructed as follows: Reporter plasmids were constructed by inserting the desired target sequences into pRS315HisMCS and pLacZi. For each 3 basepair target site, two oligonucleotides were synthesized, annealed, and inserted into the *Sal*I and *Xma*I site of pRS315HisMCS and of pLacZi to make reporter plasmids. The DNA sequences of the oligonucleotides were as follows: 5'- CCGGT NNNTGGGCG TAC NNNTGGGCG TCA NNNTGGGCG -3' (SEQ ID NO:88) and 5'- TCGA CGCCCANNN TGA CGCCCANNN GTA CGCCCANNN A -3' (SEQ ID NO:89).
Total 64 pairs of oligonucleotides were synthesized and inserted into the two reporter plasmids.

### Example 6: Selection of zinc finger domains with desired DNA-binding specificity

To select zinc finger domains that specifically bind given target sequences, yeast cells were transformed first with a reporter plasmid and then a library of hybrid plasmids encoding hybrid transcription factors. Yeast transformation and screening procedures were carried out as described in Ausubel *et al.* (Current Protocols in Molecular Biology (1998), John Wiley and Sons, Inc.). Yeast strain yWAM2 (*MATa (alpha)* Δ *gal4* Δ *gal80 URA3::GAL1-lacZ lys2801 his3*-Δ *200 trp1*-Δ *63 leu2 ade2-101 CYH2)* was used.

In one instance, yeast cells were first transformed with a reporter plasmid containing the composite binding sequence 5'-GAGCGGGCG-3' (the 4-bp target sequence is underlined), which was operably linked to the reporter gene. Then, the plasmid library of mutant zinc finger domains prepared by random mutagenesis was introduced into the transformed yeast cells. About 10⁶ colonies were obtained in medium lacking both leucine and tryptophan. Because the reporter plasmid and the zinc finger domain expression plasmids contain yeast *LEU2* and *TRP1* genes, respectively, as a marker, yeast cells were grown in medium lacking both leucine and tryptophan in order to select for cells that contain both the reporter and the zinc finger domain expression plasmid.

In one implementation, the library of zinc finger domains derived from the human genome was transformed into cells bearing the reporter plasmids. The transformation was performed on five different host cell strains, each strain containing one of five different target sequences operably linked to the reporter gene. About 10⁵ colonies were obtained per transformation in medium lacking both leucine and tryptophan. Transformants were grown on petri plates containing synthetic medium lacking leucine and tryptophan. After incubation, transformed cells were collected by applying a 10% sterile glycerol solution to the plates, scraping the colonies into the solution, and retrieving the solution. Cells were stored as frozen aliquots in the glycerol solution..A single aliquot was spread onto medium lacking leucine, tryptophan and histidine. 3-aminotriazole (AT) was added to the growth medium at the final concentrations of 0, 0.03, 0.1 and 0.3 mM. AT is a competitive inhibitor of His3 and titrates the sensitivity of the *HIS3* selection system. AT suppressed the basal activity of His3. Such basal activity can arise from leaky expression of the *HIS3* gene on the reporter plasmid. Out of about 10⁷ yeast cells spread on medium, on the order of hundreds of colonies grew in the selective medium lacking AT. The number of colonies gradually decreased as the concentration of AT increased. On the order of tens of colonies grew in the selective medium containing 0.3 mM of AT. Several colonies were randomly picked from the medium lacking AT and from the medium containing 0.3 mM of AT. Plasmids were isolated from yeast cells and transformed into *Escherichia coli* strain KC8 (*pyrF leuB600 trpC hisB463*). The plasmids encoding zinc finger transcription factor were isolated, and the DNA sequences of selected zinc finger domains were determined.

The amino acid sequence of each selected zinc finger domain was deduced from the DNA sequence. Each zinc finger domain was named after the four amino acid residues at base-contacting positions, namely positions -1, 2, 3, and 6 along the alpha-helix. The results are shown in Table 1. Identified zinc finger domains are named by the four amino acids found at base-contacting positions. Analysis of the sequences showed that in some cases the same zinc finger domain was obtained repeatedly. The numbers in the parenthesis in Table 1 represent how many times the same zinc finger domains have been obtained. For example, two zinc fingers having CSNR at the four base contacting positions were identified as binding the GAGC nucleic acid site (see column 3, "GAGC/human genome").

**Table 1**

| Target Sequence | GAGC | GAGC | GCTT | GACT | GAGT | ACAT |
|---|---|---|---|---|---|---|
| origin of zinc finger domain library | random mutagenesis | human genome | human genome | human genome | Human genome | human genome |
| amino acid | KTNR(2) | RTNR(2) | VSTR(9) | HSNK(2) | RDER(2) | QSTV(3) |
| residues at base | RTTR | RTNR | | CSNR(7) | SSNR(5) | |
| contacting | RPNR | CSNR(2) | | | | |
| positions* | HSNR | SSNR(3) | | | | |
| | RLKP | RSTV | | | | |
| | TRQR | SSGE | | | | |
| | TALH | | | | | |
| | RQKA | | | | | |
| | PARV | | | | | |
| | RTFR | | | | | |
| | RNNR | | | | | |
| | DPLH | | | | | |
| | RGNR | | | | | |

| | | | | | | |
|---|---|---|---|---|---|---|
| * The four-letter identifiers in the six columns to the right are the descriptors of the zinc finger domains isolated for each target sequence. Although these names are indicative of the amino acid residues at base contacting positions, they are not sequences of polypeptides. | | | | | | |

The full DNA sequences encoding selected human zinc finger domains and their translated amino acid sequences are shown in Fig. 11. The DNA sequence that is complementary to the degenerate PCR primers used to amplify DNA segments encoding zinc finger domains in the human genome is underlined. This sequence may differ from the original base sequence of reported human genome sequence due to either allelic differences or alterations introducing during amplification.

Most human zinc finger domains identified by screening in accordance with the present invention either were novel polypeptides or corresponded to anonymous open reading frames. For example, zinc finger domains designated as HSNK (contained in the sequence reported in GenBank accession number AF155100) and VSTR (contained in the sequence reported in GenBank accession number AF02577) are found in proteins whose function is as yet unknown. The results described herein not only indicate that these zinc finger domains are able to function as sequence-specific DNA-binding domains, but also document their preferred binding site preference in the context of chimeric proteins.

In addition, the present invention reveals that zinc finger domains obtained from the human genome can be used as modular building blocks to construct novel DNA-binding proteins. Human zinc finger domains of the present invention were obtained as a result of their functionality *in vivo* when connected to the C-terminus of finger 1 and finger 2 of Zif268. Thus, the identified zinc finger domains can recognize specific sequence in an artificial context, and are suitable as modular building blocks for designing synthetic transcription factors.

### Example 7: Pairwise Mating

To facilitate identification of zinc finger domains that bind to each 3 basepair target site, yeast mating was used to eliminate the need for repetitively transforming yeast cells and to search for positive binders to each of the 64 reporter constructs with a single transformation. Two yeast strains, YWI (*MATα* mating type) and YPH499 (*MATa* mating type), were used. YW1 was derived from yWAM2 by selecting a clone resistant to 5-fluoroorotic acid (FOA) in order to generate a *ura3-* derivative of yWAM2.

The plasmid library of zinc finger domains were introduced into the YW1 cells by yeast transformation. Cells from approximately 10⁶ independently transformed colonies were collected by scraping plates with a 10% glycerol solution. The solution was frozen in aliquots. Each pair of 64 reporter plasmids (derived from pLacZi or pRS315His) also was cotransfected into yeast strain YPH499. Transformants containing both reporter plasmids were harvested and frozen.

After thawing, the yeast cells were grown on minimal media to mid-log phase. The two cell types were then mixed and allowed to mate in YPD for 5 h. Diploid cells were selected on minimal media containing X-gal and AT.(1 mM) but lacking tryptophan, leucine, uracil, and histidine. After several days, blue colonies that grew on the selective plate were isolated. The plasmids encoding zinc finger domains were isolated from blue colonies, and the DNA sequences of the selected zinc finger domains were determined.

The nucleic acids isolated from the blue colonies were individually retransformed into YW1 cells. For each isolated nucleic acid, retransformed YW1 cells were mated to YPH499 cells containing each of the 64 LacZ reporter plasmids in a 96-well plate, and then spread onto minimal media containing X-gal but lacking tryptophan and uracil. The DNA binding affinities and specificities of a zinc finger domain for 64 target sequences were determined by the intensity of blue color. Control experiments with the Zif268 zinc finger domains indicated that positive interactions between a zinc finger domain and a binding site yielded dark to pale blue colonies, (whose blue intensity is proportional to the binding affinity) and that negative interactions yielded white colonies.

### Example 8: Comparison of Identified Zinc Finger Domains with an Interaction Code

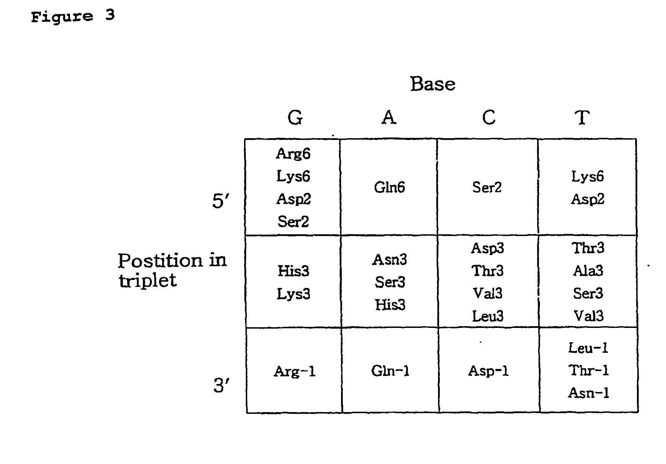

The amino acid residues of selected zinc finger domains at the critical base-contacting positions were compared with those anticipated from the zinc finger domain-DNA interaction code (Fig. 3). Most of zinc finger domains showed expected patterns, i.e. the amino acid residues at the critical positions match well those predicted from the code.

For example, the consensus amino acid residues in zinc finger domains selected from the library generated by random mutagenesis were R (Arg; 7 out of 14) - or K (Lys; 2 out of 14) at position -1, N (Asp; 6 out of 14) at position 3, and R (9 out of 14) at position 6 (Table 1). These zinc finger domains were selected with the GAGC plasmid. (The reporter plasmid in which the composite binding sequence, 5'-GAGCGGGCG-3', is operably linked to the reporter gene is referred to as the GAGC plasmid. Likewise, the other reporter plasmids in which the sequence, 5'-XXXXGGGCG-3', is operably linked to the reporter gene are referred to as the XXXX plasmids.) These amino acid residues at critical base-contacting positions exactly match those expected from the code. [Most of the zinc finger domains in the human genome contain S (serine) at position 2 and a serine residue is capable of forming a hydrogen bond with any of the four bases. Thus the effect of this position will not be considered hereinafter. It is also known that the residues at position 2 usually play only a minor role in base recognition (Pavletich and Pabo (1991) *Science* **252**, 809-817).]

The amino acid residues in zinc finger domains obtained from the human genome also match those expected from the code quite well. For example, the consensus amino acid residues at position -1, 3, and 6 in the zinc finger domains obtained with the GAGC plasmid were R, N, and R, respectively (Table 1, column 3). These amino acids are exactly those anticipated from the code.

The amino acid residues at position -1, 3, and 6 in the zinc finger domain obtained with the GCTT plasmid were V, T, and R, respectively (Table 1, column 4). The T and R residues are exactly those expected from the code. The amino acid residues predicted from the code at position -1 that would interact with the base T (underlined) of the GCTT site are L, T or N. The VSTR zinc finger domain, which was selected with the GCTT plasmid, contained V (valine), a hydrophobic amino acid similar to L (leucine) at this position.

Overall, the amino acid residues in selected zinc finger domains match those predicted from the code at least at two positions out of the three critical positions. The amino acid residues in selected zinc finger domains that are expected from the code are underlined in Table 1. These results strongly suggest that the *in vivo* selection system disclosed herein functions as expected.

### Example 9: Retransformation and Cross-transformation

To rule out the possibility of false positive results and to investigate the sequence specificity of the zinc finger protein described above, retransformation and cross-transformation of yeast cells were carried out using the isolated plasmids.

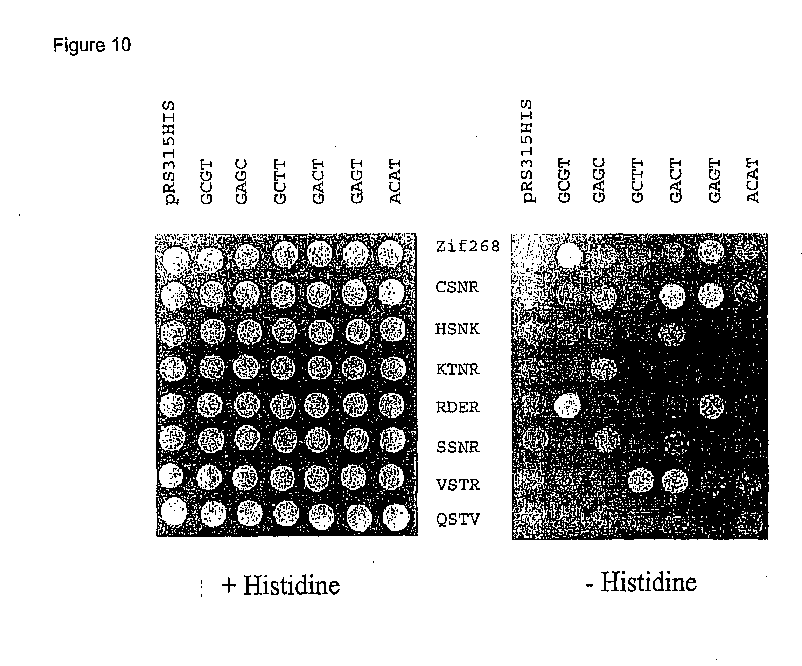

Yeast cells were first co-transformed with a reporter plasmid and a hybrid plasmid encoding a zinc finger domain. Yeast transformants were inoculated into minimal medium lacking leucine and tryptophan and incubated for 36 hours. About 1,000 cells in the growth medium were spotted directly onto solid medium lacking leucine, tryptophan, and histidine (designated as - histidine in Fig. 10) and onto solid medium lacking leucine and tryptophan (designated as + histidine in Fig. 10). These cells were then incubated for 50 hours at 30°C. The results are shown in Fig. 10.

It is expected that colonies can grow in the medium lacking histidine when the zinc finger moiety of the hybrid transcription factor binds the composite binding sequence, allowing the hybrid transcription factor to activate expression of the *HIS3* reporter gene. Colonies cannot grow in the medium lacking histidine when the zinc finger moiety of the transcription factor does not bind the composite binding sequence.

As shown in Fig. 10, the isolated zinc finger domains were capable of binding corresponding target sequences and showed sequence specificity markedly different from that ofZif268. Zif268 showed higher activity with the GCGT plasmid than with the other five plasmids, and relatively high activity with the GAGT plasmid. No colonies were formed by strains having reporters containing other binding sites and expressing the Zif268 protein.

The KTNR zinc finger domain isolated from the random mutant library was originally selected with the GAGC reporter plasmid. As expected, colonies were formed only with the GAGC plasmid. Zinc finger domains obtained from the library derived from the human genome also showed expected specificity. For example, HSNK, which had been selected with the GACT plasmid, allowed cell growth only with the GACT plasmid when retransformed into yeast cells. VSTR, which had been selected with the GCTT plasmid, showed the highest activity with the GCTT plasmid. RDER, which was selected with the GAGT plasmid, has the same amino acid residues at the four base-contacting positions as does finger 3 of Zif268. As expected, this zinc finger domain showed sequence specificity similar to that of finger 3. SSNR, selected with the GAGC and GAGT plasmids, allowed cell growth on histidine-deficient medium with the GAGC plasmid but not with the GAGT plasmid. QSTV, obtained with the ACAT plasmid, did not allow cell growth with any of the plasmids tested in this assay. However, this zinc finger domain was able to bind to the ACAT sequence tightly *in vitro* as demonstrated below.

### Example 10: Gel shift assays

Zinc finger proteins containing zinc finger domains selected using the modified one-hybrid system were expressed in *E*. *coli,* purified, and used in gel shift assays. The DNA segments encoding zinc finger proteins in the hybrid plasmids were isolated by digestion with *Sal*I and *Not*I and inserted into pGEX-4T2 (Pharmacia Biotech) between the *Sal*I and *Not*I sites. Zinc finger proteins were expressed in *E*. *coli* strain BL21 as fusion proteins connected to GST (Glutathione-S-transferase). The fusion proteins were purified using glutathione affinity chromatography (Pharmacia Biotech, Piscataway, NJ) and then digested with thrombin, which cleaves the connecting site between the GST moiety and zinc finger proteins. Purified zinc finger proteins contained finger 1 and finger 2 of Zif268 and selected zinc finger domains at the C-terminus.

The following probe DNAs were synthesized, annealed, labeled with ³²P using T4 polynucleotide kinase, and used in gel shift assays.

Various amounts of a zinc finger protein were incubated with a labeled probe DNA for one hour at room temperature in 20 mM Tris pH 7.7, 120 mM NaCl, 5 mM MgCl₂, 20 µM ZnSO₄, 10% glycerol, 0.1% Nonidet P-40, 5 mM DTT, and 0.10 mg/mL BSA (bovine serum albumin), and then the reaction mixtures were subjected to gel electrophoresis. The radioactive signals were quantitated by PhosphorImager™ analysis (Molecular Dynamics), and dissociation constants (*K*_{d}) were determined as described (Rebar and Pabo (1994) *Science* 263:671-673). The results are described in Table 2. All the constants were determined in at least two separate experiments, and the standard error of the mean is indicated. Cell growth of yeast transformants on histidine-deficient minimal medium (Fig. 10) is also indicated in Table 2.

**Table 2**

| Zinc finger protein | Probe DNA | Dissociation Constant (nM) | Growth of Yeast |
|---|---|---|---|
| Zif268 | GCTT | 2.1 ± 0.3 | - |
| | GCGT | 0.024 ± 0.004 | +++ |
| | GAGT | 0.17 ± 0.04 | ++ |
| | GAGC | 2.3 ± 0.9 | - |
| | GACT | 4.9 ± 0.6 | - |
| | ACAT | 1.3 ± 0.3 | - |
| KTNR | GCGT | 5.5 ± 0.7 | - |
| | GAGC | 0.17 ± 0.01 | ++ |
| | GACT | 30 ± 1 | - |
| CSNR | GCGT | 2.7 ± 0.3 | - |
| | GAGT | 0.46 ± 0.04 | +++ |
| | GAGC | 1.2 ± 0.1 | ++ |
| | GACT | 0.17 ± 0.01 | +++ |
| HSNK | GCGT | 42 ± 14 | - |
| | GAGT | 3.5 ± 0.1 | - |
| | GACT | 0.32 ± 0.08 | ++ |
| RDER | GCGT | 0.027 ± 0.002 | +++ |
| | GAGT | 0.18 ± 0.01 | ++ |
| | GACT | 28 ± 9 | - |
| SSNR | GCGT | 3.8 ± 1.3 | - |
| | GAGC | 0.45 ± 0.09 | ++ |
| | GACT | 0.61 ± 0.21 | + |
| VSTR | GCTT | 0.53 ± 0.07 | ++ |
| | GCGT | 0.76 ± 0.22 | - |
| | GAGT | 1.4 ± 0.2 | - |
| QSTV | GCTT | 29 ± 3 | |
| | GCGT | 9.8 ± 3.4 | - |
| | ACAT | 2.3 ± 0.4 | - |

| | | | |
|---|---|---|---|
| * +++, 20 to 100% growth; ++, 5 to 20% growth; +, 1-5% growth; -, < 1% growth. | | | |

Zinc finger proteins that allowed cell growth on histidine-deficient plates bound the corresponding probe DNAs tightly. For example, the Zif268 protein used as a control allowed cell growth with the GCGT and GAGT reporter plasmids, and the dissociation constants measured *in vitro* using corresponding probe DNAs were 0.024 nM and 0.17 nM, respectively. In contrast, the Zif268 protein did not allow cell growth with other plasmids, and the dissociation constants measured using corresponding probe DNAs were higher than 1 nM.

Zinc finger proteins containing novel zinc finger domains also showed similar results. For example, the KTNR protein showed strong affinity for the GAGC probe DNA, with a dissociation constant of 0.17 nM, but not for the GCGT or GACT probe DNA, with dissociation constants of 5.5 nM or 30 nM, respectively. This protein allowed cell growth only with the GAGC plasmid. The HSNK protein was able to bind the GACT probe DNA tightly (*K*_{d} = 0.32 nM) but not the GCGT or GAGT probe DNA; as would be expected, the HSNK protein allowed cell growth only with the GACT plasmid.

The QSTV protein, which was selected with the ACAT reporter plasmid, was not able to promote cell growth with any of the other reporter plasmids when retransformed into yeast. Gel shift assays demonstrated that this protein bound the ACAT probe DNA more tightly than it did the other probe DNAs. That is, QSTV bound the ACAT probe DNA 13 times or 4.3 times stronger than it did the GCTT or GCGT probe DNA respectively.

In general, when a zinc finger protein, e.g., having three zinc finger domains, binds a DNA sequence with a dissociation constant lower than 1 nM, it allows cell growth, whereas when a zinc finger protein binds a DNA sequence with a dissociation constant higher than 1 nM, it does not allow cell growth. Zinc finger proteins that bind with a dissociation constant of greater than 1 nM, but less than 5 nM can also be useful, e.g., in the context of a chimeric zinc finger protein having four zinc finger domains.

### Example 11: TG-ZFD-001 "CSNR1"

TG-ZFD-001 "CSNR1" was identified by *in vivo* screening from human genomic sequence. Its amino acid sequence is YKCKQCGKAFGCPSNLRRHGRTH (SEQ ID NO:23). It is encoded by the human nucleic acid sequence:
5'-TATAAATGTAAGCAATGTGGGAAAGCTTTTGGATGTCCCTCAAACCTTCGAA GGCATGGAAGGACTCAC-3' (SEQ ID NO:22).

As a polypeptide fusion to fingers 1 and 2 of Zif268, TG-ZFD-001 "CSNR1" demonstrates recognition specificity for the 3-bp target sequence sequences GAA, GAC, and GAG. Its binding site preference is GAA > GAC > GAG > GCG as determined by *in vivo* screening results and EMSA. In EMSA, the TG-ZFD-001 "CSNR" fusion to fingers 1 and 2 of Zif268 and the GST purification handles has an apparent K_{d} of 0.17 nM for the GAC containing site, 0.46 nM for the GAG containing site, and 2.7 nM for the GCG containing site.

TG-ZFD-001 "CSNR1" can be used as a module to construct a chimeric DNA binding protein comprising multiple zinc finger domains, e.g., for the purpose of recognizing a DNA site containing the sequence GAA, GAC, or GAG.

### Example 12: TG-ZFD-002 "HSNK"

TG-ZFD-002 "HSNK" was identified *by in vivo* screening from human genomic sequence. Its amino acid sequence is: YKCKECGKAFNHSSNFNKHHRIH (SEQ ID NO:25). It is encoded by the human nucleic acid sequence:
5'-TATAAGTGTAAGGAGTGTGGGAAAGCCTTCAACCACAGCTCCAACTTCAATA AACACCACAGAATCCAC-3' (SEQ ID NO:24).

As a polypeptide fusion to fingers 1 and 2 of Zif268, TG-ZFD-002 "HSNK" demonstrates recognition specificity for the 3-bp target sequence GAC. Its binding site preference is GAC > GAG > GCG as determined by *in vivo* screening results and EMSA. In EMSA, the TG-ZFD-002 "HSNK" fusion to fingers 1 and 2 ofZif268 and the GST purification handles has an apparent K_{d} of 0.32 nM for the GAC containing site, 3.5 nM for the GAG containing site, and 42 nM for the GCG containing site.

TG-ZFD-002 "HSNK" can be used as a module to construct a chimeric DNA binding protein comprising multiple zinc finger domains, e.g., for the purpose of recognizing a DNA site containing the sequence GAC.

### Example 13: TG-ZFD-003 "SSNR"

TG-ZFD-003 "SSNR" was identified by *in vivo* screening from human genomic sequence. Its amino acid sequence is: YECKECGKAFSSGSNFTRHQRIH (SEQ ID NO:27). It is encoded by the human nucleic acid sequence:
5'-TATGAATGTAAGGAATGTGGGAAAGCCTTTAGTAGTGGTTCAAACTTCACTC GACATCAGAGAATTCAC-3' (SEQ ID NO:26).

As a polypeptide fusion to fingers 1 and 2 of Zif268, TG-ZFD-003 "SSNR" demonstrates recognition specificity for the 3-bp target sequence GAG. Its binding site preference is GAG > GAC > GCG as determined by *in vivo* screening results and EMSA. In EMSA, the TG-ZFD-003 "SSNR" fusion to fingers 1 and 2 of Zif268 and the GST purification handles has an apparent K_{d} of 0.45 nM for the GAG containing site, 0.61 nM for the GAC containing site, and 3.8 nM for the GCG containing site.

TG-ZFD-003 "SSNR" can be used as a module to construct a chimeric DNA binding protein comprising multiple zinc finger domains, e.g., for the purpose of recognizing a DNA site containing the sequence GAG, or GAC.

### Example 14: TG-ZFD-004 "RDER1"

TG-ZFD-004 "RDER1" was identified by *in vivo* screening from human genomic sequence. Its amino acid sequence is:
YVCDVEGCTWKFARSDELNRHKKRH (SEQ ID NO:29). It is encoded by the human nucleic acid sequence:
   5'-TATGTATGCGATGTAGAGGGATGTACGTGGAAATTTGCCCGCTCAGATGAGC TCAACAGACACAAGAAAAGGCAC-3' (SEQ ID NO:28).

As a polypeptide fusion to fingers 1 and 2 of Zif268, TG-ZFD-004 "RDER1" demonstrates recognition specificity for the 3-bp target sequence GCG. Its binding site preference is GCG > GTG, GAG > GAC as determined by *in vivo* screening results and EMSA. In EMSA, the TG-ZFD-004 "RDER1" fusion to fingers 1 and 2 of Zif268 and the GST purification handles has an apparent K_{d} of 0.027 nM for the GCG containing site, 0.18 nM for GAG containing site, and 28 nM for the GAC containing site.

TG-ZFD-004 "RDER1" can be used as a module to construct a chimeric DNA binding protein comprising multiple zinc finger domains, e.g., for the purpose of recognizing a DNA site containing the sequence GCG, GTG or GAG.

### Example 15: TG-ZFD-005 "QSTV"

TG-ZFD-005 "QSTV" was identified by *in vivo* screening from human genomic sequence. Its amino acid sequence is: YECNECGKAFAQNSTLRVHQRIH (SEQ ID NO:31). It is encoded by the human nucleic acid sequence:
5'-TATGAGTGTAATGAATGCGGGAAAGCTTTTGCCCAAAATTCAACTCTCAGAG TACACCAGAGAATTCAC-3' (SEQ ID NO:30).

As a polypeptide fusion to fingers 1 and 2 of Zif268, TG-ZFD-005 "QSTV" demonstrates recognition specificity for the 3-bp target sequence ACA. Its binding site preference is ACA > GCG > GCT as determined by EMSA. In EMSA, the TG-ZFD-005 "QSTV" fusion to fingers 1 and 2 of Zif268 and the GST purification handles has an apparent K_{d} of 2.3 nM for the ACA containing site, 9.8 nM for the GCG containing site, and 29 nM for the GCT containing site.

TG-ZFD-005 "QSTV" can be used as a module to construct a chimeric DNA binding protein comprising multiple zinc finger domains, e.g., for the purpose of recognizing a DNA site containing the sequence ACA.

### Example 16: TG-ZFD-006 "VSTR"

TG-ZFD-006 "VSTR" was identified by *in vivo* screening from human genomic sequence. Its amino acid sequence is: YECNYCGKTFSVSSTLIRHQRIH (SEQ ID NO:33). It is encoded by the human nucleic acid sequence:
5'-TATGAGTGTAATTACTGTGGAAAAACCTTTAGTGTGAGCTCAACCCTTATTA GACATCAGAGAATCCAC-3' (SEQ ID NO:32).

As a polypeptide fusion to fingers 1 and 2 of Zif268, TG-ZFD-006 "VSTR" demonstrates recognition specificity for the 3-bp target sequence GCT. Its binding site preference is GCT > GCG > GAG as determined by *in vivo* screening results and EMSA. In EMSA, the TG-ZFD-006 "VSTR" fusion to fingers 1 and 2 of Zif268 and the GST purification handles has an apparent K_{d} of 0.53 nM for the GCT containing site, 0.76 for the GCG containing site, and 1.4 nM for the GAG containing site.

TG-ZFD-006 "VSTR" can be used as a module to construct a chimeric DNA binding protein comprising multiple zinc finger domains, e.g., for the purpose of recognizing a DNA site containing the sequence GCT or GCG.

### Example 17: TG-ZFD-007 "CSNR2"

TG-ZFD-007 "CSNR2" was identified by *in vivo* screening from human genomic sequence. Its amino acid sequence is: YQCNICGKCFSCNSNLHRHQRTH (SEQ ID NO:35). It is encoded by the human nucleic acid sequence:
5'-TATCAGTGCAACATTTGCGGAAAATGTTTCTCCTGCAACTCCAACCTCCA CAGGCACCAGAGAACGCAC -3' (SEQ ID NO:34).

As a polypeptide fusion to fingers 1 and 2 of Zif268, TG-ZFD-007 "CSNR2" demonstrates recognition specificity for 3-bp target sequences GAA, GAC, and GAG. Its binding site preference is GAA > GAC > GAG as determined by *in vivo* screening results.

TG-ZFD-007 "CSNR2" can be used as a module to construct a chimeric DNA binding protein comprising multiple zinc finger domains, e.g., for the purpose of recognizing a DNA site containing the sequence GAA, GAC, or GAG.

### Example 18: TG-ZFD-008 "QSHR1"

TG-ZFD-008 "QSHR1" was identified by *in vivo* screening from human genomic sequence. Its amino acid sequence is: YACHLCGKAFTQSSHLRRHEKTH (SEQ ID NO:37). It is encoded by the human nucleic acid sequence:
5'-TATGCATGTCATCTATGTGGAAAAGCCTTCACTCAGAGTTCTCACCTTAG AAGACATGAGAAAACTCAC -3' (SEQ ID NO:36).

As a polypeptide fusion to fingers 1 and 2 of Zif268, TG-ZFD-008 "QSHR1" demonstrates recognition specificity for 3-bp target sequences GGA, GAA, and AGA. Its binding site preference is GGA > GAA > AGA as determined by *in vivo* screening results.

TG-ZFD-008 "QSHR1" can be used as a module to construct a chimeric DNA binding protein comprising multiple zinc finger domains, e.g., for the purpose of recognizing a DNA site containing the sequence GGA, GAA, or AGA.

### Example 19: TG-ZFD-009 "QSHR2"

TG-ZFD-009 "QSHR.2" was identified by *in vivo* screening from human genomic sequence. Its amino acid sequence is: YKCGQCGICFYSQVSHLTRHQKIH (SEQ ID NO:39). It is encoded by the human nucleic acid sequence:
5'-TATAAATGCGGCCAGTGTGGGAAGTTCTACTCGCAGGTCTCCCACCTCA CCCGCCACCAGAAAATCCAC -3' (SEQ ID NO:38).

As a polypeptide fusion to fingers 1 and 2 of ZifZ68, TG-ZFD-009 "QSHR2" demonstrates recognition specificity for the 3-bp target sequence GGA.

TG-ZFD-009 "QSHR2" can be used as a module to construct a chimeric DNA binding protein comprising multiple zinc finger domains, e.g., for the purpose of recognizing a DNA site containing the sequence GGA.

### Example 20: TG-ZFD-010 "QSHR3"

TG-ZFD-010 "QSHR3" was identified by *in vivo* screening from human genomic sequence. Its amino acid sequence is: YACHLCGKAFTQCSHLRRHEKTH (SEQ ID NO:41). It is encoded by the human nucleic acid sequence:
5'-TATGCATGTCATCTATGTGGAAAAGCCTTCACTCAGTGTTCTCACCTTAG AAGACATGAGAAAACTCAC-3' (SEQ ID NO:40).

As a polypeptide fusion to fingers 1 and 2 of Zif268, TG-ZFD-010 "QSHR3" demonstrates recognition specificity for 3-bp target sequences GGA and GAA. Its binding site preference is GGA > GAA as determined by *in vivo* screening results.

TG-ZFD-O10 "QSHR3" can be used as a module to construct a chimeric DNA binding protein comprising multiple zinc finger domains, e.g., for the purpose of recognizing a DNA site containing the sequence GGA or GAA.

### Example 21: TG-ZFD-011 "QSHR4"

TG-ZFD-O11 "QSHR4" was identified by in *vivo* screening from human genomic sequence. Its amino acid sequence is: YACHLCAKAFIQCSHLRRHEKTH (SEQ ID NO:43). It is encoded by the human nucleic acid sequence:
5'-TATGCATGTCATCTATGTGCAAAAGCCTTCATTCAGTGTTCTCACCTTAGA AGACATGAGAAAACTCAC -3' (SEQ ID NO:42).

As a polypeptide fusion to fingers 1 and 2 of Zif268, TG-ZFD-011 "QSHR4" demonstrates recognition specificity for 3-bp target sequences GGÄ and GAA.- Its binding site preference is GGA > GAA as determined by *in vivo* screening results.

TG-ZFD-011 "QSHR4" can be used as a module to construct a chimeric DNA binding protein comprising multiple zinc finger domains, e.g., for the purpose of recognizing a DNA site containing the sequence GGA or GAA.

### Example 22: TG-ZFD-012 "QSHR5"

TG-ZFD-012 "QSHR5" was identified by *in vivo* screening from human genomic sequence. Its amino acid sequence is: YVCRECGRGFRQHSHLVRHKRTH (SEQ ID NO:45). It is encoded by the human nucleic acid sequence:
5'-TATGTTTGCAGGGAATGTGGGCGTGGCTTTCGCCAGCATTCACACCTGGT-CAGACACAAGAGGACACAT -3' (SEQ ID NO:44).

As a polypeptide fusion to fingers 1 and 2 of Zif268, TG-ZFD-012 "QSHR5" demonstrates recognition specificity for 3-bp target sequences GGA, AGA, GAA, and CGA. Its binding site preference is GGA > AGA > GAA > CGA as determined by *in vivo* screening results.

TG-ZFD-012 "QSHR5" can be used as a module to construct a chimeric DNA binding protein comprising multiple zinc finger domains, e.g., for the purpose of recognizing a DNA site containing the sequence GGA, AGA, GAA, or CGA.

### Example 23: TGZFD-013 "QSNR1"

TG-ZFD-013 "QSNR1" was identified by *in vivo* screening from human genomic sequence. Its amino acid sequence is: FECKDCGKAFIQKSNLIRHQRTH (SEQ ID NO:47). It is encoded by the human nucleic acid sequence:
5'-TTTGAGTGTAAAGATTGCGGGAAAGCTTTCATTCAGAAGTCAAACCTCA TCAGACACCAGAGAACTCAC-3' (SEQ ID NO:46).

As a polypeptide fusion to fingers 1 and 2 of Zif268, TG-ZFD-013 "QSNR1" Is demonstrates recognition specificity for the 3-bp target sequence GAA.

TG-ZFD-013 "QSNR1" can be used as a module to construct a chimeric DNA binding protein comprising multiple zinc finger domains, e.g., for the purpose of recognizing a DNA site containing the sequence GAA.

### Example 24: TG-ZFD-014 "QSNR2"

TG-ZFD-014 "QSNR2" was identified by *in vivo* screening from human genomic sequence. Its amino acid sequence is: YVCRECRRGFSQKSNLIRHQRTH (SEQ ID NO:49). It is encoded by the human nucleic acid sequence:
5'-TATGTCTGCAGGGAGTGTAGGCGAGGTTTTAGCCAGAAGTCAAATCTCA TCAGACACCAGAGGACGCAC-3' (SEQ ID NO:48).

As a polypeptide fusion to fingers 1 and 2 of Zif268, TG-ZFD-014 "QSNR2" demonstrates recognition specificity for the 3-bp target sequence GAA.

TG-ZFD-014 "QSNR2" can be used as a module to construct a chimeric DNA binding protein comprising multiple zinc finger domains, e.g., for the purpose of recognizing a DNA site containing the sequence GAA.

### Example 25: TG-ZFD-O15 "QSNV1"

TG-ZFD-O15 "QSNV1" was identified by *in vivo* screening from human genomic sequence. Its amino acid sequence is: YECNTCRKTFSQKSNLIVHQRTH (SEQ ID NO:5 1): It is encoded by the human nucleic acid sequence:
5'-TATGAATGTAACACATGCAGGAAAACCTTCTCTCAAAAGTCAAATCTCAT TGTACATCAGAGAACACAC-3' (SEQ ID NO:50).

As a polypeptide fusion to fingers 1 and 2 of Zif268, TG-ZFD-O15 "QSNV1" demonstrates recognition specificity for 3-bp target sequences AAA and CAA. Its binding site preference is AAA > CAA as determined by in *vivo* screening results.

TG-ZFD-015 "QSNV1" can be used as a module to construct a chimeric DNA binding protein comprising multiple zinc finger domains, e.g., for the purpose of recognizing a DNA site containing the sequence AAA or CAA.

### Example 26: TG-ZFD-016 "QSNV2"

TG-ZFD-016 "QSNV2" was identified by *in vivo* screening from human genomic sequence. Its amino acid sequence is: YVCSKCGKAFTQSSNLTVHQKIH (SEQ ID NO:53). It is encoded by the human nucleic acid sequence:
5'-TATGTTTGCTCAAAATGTGGGAAAGCCTTCACTCAGAGTTCAAATCTGAC TGTACATCAAAAATCCAC-3'(SEQ ID NO:52).

As a polypeptide fusion to fingers 1 and 2 of Zif268, TG-ZFD-016 "QSNV2" demonstrates recognition specificity for 3-bp target sequences AAA and CAA. Its binding site preference is AAA > CAA as determined by *in vivo* screening results.

TG-ZFD-016 "QSNV2" can be used as a module to construct a chimeric DNA binding protein comprising multiple zinc finger domains, e.g., for the purpose of recognizing a DNA site containing the sequence AAA or CAA.

### Example 27: TG-ZFD-017 "QSNV3"

TG-ZFD-017 "QSNV3" was identified by *in vivo* screening from human genomic sequence. Its amino acid sequence is: YKCDECGKNFTQSSNLIVHKRIH (SEQ ID NO:55). It is encoded by the human nucleic acid sequence:
5'-TACAAATGTGACGAATGTGGAAAAAACTTTACCCAGTCCTCCAACCTTA TTGTACATAAGAGAATTCAT -3' (SEQ ID NO:54).

As a polypeptide fusion to fingers 1 and 2 of Zif268, TG-ZFD-017 "QSNV3" demonstrates recognition specificity for a 3-bp target sequence AAA.

TG-ZFD-017 "QSNV3" can be used as a module to construct a chimeric DNA binding protein comprising multiple zinc finger domains, e.g., for the purpose of recognizing a DNA site containing the sequence AAA.

### Example 28: TG-ZFD-018 "QSNV4"

TG-ZFD-018 "QSNV4" was identified *by in vivo* screening from human genomic sequence. Its amino acid sequence is:
YECDVCGKTFTQKSNLGVHQRTH (SEQ ID NO:57). It is encoded by the human nucleic acid sequence:
   5'-TATGAATGTGATGTGTGTGGAAAAACCTTCACGCAAAAGTCAAACCTTG GTGTACATCAGAGAACTCAT -3' (SEQ ID NO:56).

As a polypeptide fusion to fingers 1 and 2 of Zif268, TG-ZFD-018 "QSNV4" demonstrates recognition specificity for the 3-bp target sequence AAA.

TG-ZFD-018 "QSNV4" can be used as a module to construct a chimeric DNA binding protein comprising multiple zinc finger domains, e.g., for the purpose of recognizing a DNA site containing the sequence AAA.

### Example 29: TG-ZFD-019 "QSSR1"

TG-ZFD-019 "QSSR1" was identified by *in vivo* screening from human genomic sequence. Its amino acid sequence is: YKCPDCGKSFSQSSSLIRHQRTH (SEQ ID NO:59). It is encoded by the human nucleic acid sequence:
5'-TATAAGTGCCCTGATTGTGGGAAGAGTTTTAGTCAGAGTTCCAGCCTCAT TCGCCACCAGCGGACACAC-3' (SEQ ID NO:58).

As a polypeptide fusion to fingers 1 and 2 of Zif268, TG-ZFD-019 "QSSR1" demonstrates recognition specificity for 3-bp target sequences GTA and GCA. Its binding site preference is GTA > GCA as determined by *in vivo* screening results.

TG-ZFD-OI9 "QSSR1" can be used as a module to construct a chimeric DNA binding protein comprising multiple zinc finger domains, e.g., for the purpose of recognizing a DNA site containing the sequence GTA or GCA.

### Example 30: TG-ZFD-020 "QSSR2"

TG-ZFD-020 "QSSR2" was identified by *in vivo* screening from human genomic sequence. Its amino acid sequence is: YECQDCGRAFNQNSSLGRHKRTH (SEQ ID NO:61). It is encoded by the human nucleic acid sequence:
5'-TATGAGTGTCAGGACTGTGGGAGGGCCTTCAACCAGAACTCCTCCCTGG GGCGGCACAAGAGGACACAC-3' (SEQ ID NO:60).

As a polypeptide fusion to fingers 1 and 2 of Zif268, TG-ZFD-020 "QSSR2" demonstrates recognition specificity for the 3-bp target sequence GTA.

TG-ZFD-020 "QSSR2" can be used as a module to construct a chimeric DNA binding protein comprising multiple zinc finger domains, e.g., for the purpose of recognizing a DNA site containing the sequence GTA.

### Example 31: TG-ZFD-021 "QSTR"

TG-ZFD-021 "QSTR" was identified by *in vivo* screening from human genomic sequence. Its amino acid sequence is: YKCEECGKAFNQSSTLTRHKIVH (SEQ ID NO:63). It is encoded by the human nucleic acid sequence:
5'-TACAAATGTGAAGAATGTGGCAAAGCTTTTAACCAGTCCTCAACCCTTA CTAGACATAAGATAGTTCAT-3' (SEQ ID NO:62).

As a polypeptide fusion to fingers 1 and 2 of Zif268, TG-ZFD-021 "QSTR" demonstrates recognition specificity for 3-bp target sequences GTA and GCA. Its binding site preference is GTA > GCA as determined by *in vivo* screening results.

TG-ZFD-021 "QSTR" can be used as a module to construct a chimeric DNA binding protein comprising multiple zinc finger domains, e.g., for the purpose of recognizing a DNA site containing the sequence GTA or GCA.

### Example 32: TG-ZFD-022 "RSHR"

TG-ZFD-022 "RSHR" was identified by *in vivo* screening from human genomic sequence. Its amino acid sequence is: YKCMECGKAFNRRSHLTRHQRIH (SEQ ID NO:65). It is encoded by the human nucleic acid sequence:
5'-TATAAGTGCATGGAGTGTGGGAAGGCTTTTAACCGCAGGTCACACCTCA CACGGCACCAGCGGATTCAC-3' (SEQ ID NO:64).

As a polypeptide fusion to fingers 1 and 2 of Zif268, TG-ZFD-022 "RSHR" demonstrates recognition specificity for the 3-bp target sequence GGG.

TG-ZFD-022 "RSHR" can be used as a module to construct a chimeric DNA binding protein comprising multiple zinc finger domains, e.g., for the purpose of recognizing a DNA site containing the sequence GGG.

### Example 33: TG-ZFD-023 "VSSR"

TG-ZFD-023 "VSSR" was identified by *in vivo* screening from human genomic sequence. Its amino acid sequence is: YTCKQCGKAFSVSSSLRRHETTH (SEQ ID NO:67). It is encoded by the human nucleic acid sequence:
5'-TATACATGTAAACAGTGTGGGAAAGCCTTCAGTGTTTCCAGTTCCCTTCG AAGACATGAAACCACTCAC-3' (SEQ ID NO:66).

As a polypeptide fusion to fingers 1 and 2 ofZif268, TG-ZFD-023 "VSSR" demonstrates recognition specificity for 3-bp target sequences GTT, GTG, and-GTA. Its binding site preference is GTT > GTG > GTA as determined by *in vivo* screening results.

TG-ZFD-023 "VSSR" can be used as a module to construct a chimeric DNA binding protein comprising multiple zinc finger domains, e.g., for the purpose of recognizing a DNA site containing the sequence GTT, GTG, or GTA.

### Example 34: TG-ZFD-024 "QAHR"

TG-ZFD-024 "QAHR" was identified by *in vivo* screening from human genomic sequence. Its amino acid sequence is: YKCKECGQAFRQRAHLIRHHKLH (SEQ ID NO:103). It is encoded by the human nucleic acid sequence:
5'-TATAAGTGTAAGGAATGTGGGCAGGCCTTTAGACAGCGTGCACATCTT ATTCGACATCACAAACTTCAC-3' (SEQ ID NO:102).

As a polypeptide fusion to fingers 1 and 2 of Zif268, TG-ZFD-024 "QAHR" demonstrates recognition specificity for the 3-bp target sequence GGA as determined by *in vivo* screening results.

TG-ZFD-024 "QAHR" can be used as a module to construct a chimeric DNA binding protein comprising multiple zinc finger domains, e.g., for the purpose of recognizing a DNA site containing the sequence GGA

### Example 35: TG-ZFD-025 "QFNR"

TG-ZFD-025 "QFNR" was identified by *in vivo* screening from human genomic sequence. Its amino acid sequence is: YKCHQCGKAFIQSFNLRRHERTH (SEQ ID NO:105). It is encoded by the human nucleic acid sequence:
5'-TATAAGTGTCATCAATGTGGGAAAGCCTTTATTCAATCCTTTAACCTTC GAAGACATGAGAGAACTCAC-3' (SEQ ID NO:104).

As a polypeptide fusion to fingers 1 and 2 of Zif268, TG-ZFD-025 "QFNR" demonstrates recognition specificity for the 3-bp target sequence GAC as determined by *in vivo* screening results.

TG-ZFD-025 "QFNR" can be used as a module to construct a chimeric DNA binding protein comprising multiple zinc finger domains, e.g., for the purpose of recognizing a DNA site containing the sequence GAC.

### Example 36: TG-ZFD-026 "QGNR"

TG-ZFD-026 "QGNR" was identified by *in vivo* screening from human genomic sequence. Its amino acid sequence is: FQCNQCGASFTQKGNLLRHIKLH (SEQ ID NO:107). It is encoded by the human nucleic acid sequence:
5'-TTCCAGTGTAATCAGTGTGGGGCATCTTTTACTCAGAAAGGTAACCTCC TCCGCCACATTAAACTGCAC-3' (SEQ ID NO:106).

As a polypeptide fusion to fingers 1 and 2 ofZif268, TG-ZFD-026 "QGNR" demonstrates recognition specificity for the 3-bp target sequence GAA as determined by *in vivo* screening results.

TG-ZFD-026 "QGNR" can be used as a module to construct a chimeric DNA-binding protein comprising multiple zinc finger domains, e.g., for the purpose of recognizing a DNA site containing the sequence GAA.

### Example 37: TG-ZFD-028 "QSHT"

TG-ZFD-028 "QSHT" was identified by *in vivo* screening from human genomic sequence. Its amino acid sequence is: YKCEECGKAFRQSSHLTTHKIIH (SEQ ID NO:111). It is encoded by the human nucleic acid sequence:
5'-TACAAATGTGAAGAATGTGGCAAAGCCTTTAGGCAGTCCTCACACCTTA CTACACATAAGATAATTCAT-3' (SEQ ID NO:110).

As a polypeptide fusion to fingers 1 and 2 of Zif268, TG-ZFD-028 "QSHT" demonstrates recognition specificity for the 3-bp target sequence AGA, CGA, TGA, and GGA. Its binding site preference is (AGA and CGA) > TGA > GGA as determined by *in vivo* screening results.

TG-ZFD-028 "QSHT" can be used as a module to construct a chimeric DNA binding protein comprising multiple zinc finger domains, e.g., for the purpose of recognizing a DNA site containing the sequence AGA, CGA, TGA, and GGA.

### Example 38: TG-ZFD-029 "QSHV"

TG-ZFD-029 "QSHV" was identified by *in vivo* screening from human genomic sequence. Its amino acid sequence is: YECDHCGKSFSQSSHLNVHICRTH (SEQ ID NO:113). It is encoded by the human nucleic acid sequence:
5'-TATGAGTGTGATCACTGTGGA.AAATCCTTTAGCCAGAGCTCTCATCTGAA TGTGCACA.AA.AGAACTCAC-3' (SEQ ID NO:112).

As a polypeptide fusion to fingers 1 and 2 of Zif268, TG-ZFD-029 "QSHV" demonstrates recognition specificity for the 3-bp target sequence CGA, AGA, and TGA. Its binding site preference is CGA > AGA > TGA as determined by in *vivo* screening results.

TG-ZFD-029 "QSHV" can be used as a module to construct a chimeric DNA binding protein comprising multiple zinc finger domains, e.g., for the purpose of recognizing a DNA site containing the sequence CGA, AGA, and TGA.

### Example 39: TG-ZFD-030 "QSNI"

TG-ZFD-030 "QSNI" was identified by *in vivo* screening from human genomic sequence. Its amino acid sequence is: YMCSECGRGFSQKSNLIIHQRTH (SEQ ID NO:115). It is encoded by the human nucleic acid sequence:
5'-TACATGTGCAGTGAGTGTGGGCGAGGCTTCAGCCAGAAGTCAAACCTC ATCATACACCAGAGGACACAC-3' (SEQ ID NO:114).

As a polypeptide fusion to fingers 1 and 2 of Zif268, TG-ZFD-030 "QSNI" demonstrates recognition specificity for the 3-bp target sequence AAA and CAA as determined by *in vivo* screening results.

TG-ZFD-030 "QSNI" can be used as a module to construct a chimeric DNA binding protein comprising multiple zinc finger domains, e.g., for the purpose of recognizing a DNA site containing the sequence AAA or CAA.

### Example 40: TG-ZFD-031 "QSNR3"

TG-ZFD-031 "QSNR3" was identified by in *vivo* screening from human genomic sequence. Its amino acid sequence is: YECEKCGKAFNQSSNLTRHKKSH (SEQ ID NO: 117). It is encoded by the human nucleic acid sequence:
5'-TATGAATGTGAAAAATGTGGCAAAGCTTTTAACCAGTCCTCAAATCTTA CTAGACATAAGAAAAGTCAT-3' (SEQ ID NO:116).

As a polypeptide fusion to fingers 1 and 2 ofZif268, TG-ZFD-031 "QSNR.3" demonstrates recognition specificity for the 3-bp target sequence GAA as determined by *in vivo* screening results.

TG-ZFD-031 "QSNR3" can be used as a module to construct a chimeric DNA binding protein comprising multiple zinc finger domains, e.g., for the purpose of recognizing a DNA site containing the sequence GAA.

### Example 41: TG-ZFD-032 "QSSR3"

TG-ZFD-032 "QSSR3" was identified by in vivo screening from human genomic sequence. Its amino acid sequence is:YECNECGKFFSQSSSLIRHRRSH (SEQ ID NO: 119). It is encoded by the human nucleic acid sequence:
5'-TATGAGTGCAATGAATGTGGGAAGTTTTTTAGCCAGAGCTCCAGCCTCA TTAGACATAGGAGA.AGTCAC-3' (SEQ ID NO:118).

As a polypeptide fusion to fingers 1 and 2 of Zif268, TG-ZFD-032 "QSSR3" demonstrates recognition specificity for the 3-bp target sequence GTA and GCA. Its binding site preference is GTA > GCA as determined by in vivo screening results.

TG-ZFD-032 "QSSR3" can be used as a module to construct a chimeric DNA binding protein comprising multiple zinc finger domains, e.g., for the purpose of recognizing a DNA site containing the sequence GTA or GCA.

### Example 42: TG-ZFD-033 "QTHQ"

TG-ZFD-033 "QTHQ" was identified by *in vivo* screening from human genomic sequence. Its amino acid sequence is: YECHDCGKSFRQSTHLTQHRRIH (SEQ ID NO:121). It is encoded by the human nucleic acid sequence:
5'-TATGAGTGTCACGATTGCGGAAAGTCCTTTAGGCAGAGCACCCACCTCA CTCAGCACCGGAGGATCCAC-3' (SEQ ID NO:120).

As a polypeptide fusion to fingers 1 and 2 of Zif268, TG-ZFD-033 "QTHQ" demonstrates recognition specificity for the 3-bp target sequence AGA, TGA, and CGA. Its binding site preference is AGA > (TGA and CGA) as determined by *in vivo* screening results.

TG-ZFD-033 "QTHQ" can be used as a module to construct a chimeric DNA binding protein comprising multiple zinc finger domains, e.g., for the purpose of recognizing a DNA site containing the sequence AGA, TGA, and CGA.

### Example 43: TG-ZFD-034 "QTHR1"

TG-ZFD-034 "QTHR1" was identified *by in vivo* screening from human genomic sequence. Its amino acid sequence is: YECHDCGKSFRQSTHLTRHRRIH (SEQ ID NO: 123). It is encoded by the human nucleic acid sequence:
5'-TATGAGTGTCACGATTGCGGAAAGTCCTTTAGGCAGAGCACCCACCTCA CTCGGCACCGGAGGATCCAC-3' (SEQ ID NO:122).

As a polypeptide fusion to fingers 1 and 2 of Zif268, TG-ZFD-034 "QTHR1" demonstrates recognition specificity for the 3-bp target sequence GGA, GAA, and AGA. Its binding site preference is GGA > (GAA and AGA) as determined by *in vivo* screening results.

TG-ZFD-034 "QTHR1" can be used as a module to construct a chimeric DNA binding protein comprising multiple zinc finger domains, e.g., for the purpose of recognizing a DNA site containing the sequence GGA, GAA, and AGA.

### Example 44: TG-ZFD-035 "QTHR2"

TG-ZFD-035 "QTHR2" was identified by *in vivo* screening from human genomic sequence. Its amino acid sequence is: HKCLECGKCFSQNTHLTRHQRT (SEQ ID NO:125). It is encoded by the human nucleic acid sequence:
5'-CACAAGTGCCTTGAATGTGGGAAATGCTTCAGTCAGAACACCCATCTG ACTCGCCACCAACGCACCCAC-3' (SEQ ID NO:124).

As a polypeptide fusion to fingers 1 and 2 of Zif268, TG-ZFD-035 "QTHR2" demonstrates recognition specificity for the 3-bp target sequence GGA as determined by *in vivo* screening results.

TG-ZFD-035 "QTHR2" can be used as a module to construct a chimeric DNA binding protein comprising multiple zinc finger domains, e.g., for the purpose of recognizing a DNA site containing the sequence GGA.

### Example 45: TG-ZFD-036 "RDER2"

TG-ZFD-036 "RDER2" was identified by *in vivo* screening from human genomic sequence. Its amino acid sequence is:
YHCDWDGCGWKFARSDELTRHYRKH (SEQ ID NO:127). It is encoded by the human nucleic acid sequence:
   5'-TACCACTGTGACTGGGACGGCTGTGGATGGAAATTCGCCCGCTCAGAT GAACTGACCAGGCACTACCGTAAACAC-3' (SEQ ID NO:126).

As a polypeptide fusion to fingers 1 and 2 of Zif268, TG-ZFD-036 "RDER2" demonstrates recognition specificity for the 3-bp target sequence GCG and GTG. Its binding site preference is-GCG > GTG as determined by *in vivo* screening results.

TG-ZFD-036 "RDER2" can be used as a module to construct a chimeric DNA binding protein comprising multiple zinc finger domains, e.g., for the purpose of recognizing a DNA site containing the sequence GCG and GTG.

### Example 46: TG-ZFD-037 "RDER3"

TG-ZFD-037 "RDER3" was identified by *in vivo* screening from human genomic sequence. Its amino acid sequence is:
YRCSWEGCEWRFARSDELTRHFRKH (SEQ ID NO:129). It is encoded by the human nucleic acid sequence:
   5'-TACAGATGCTCATGGGAAGGGTGTGAGTGGCGTTTTGCAAGA.AGTGAT GAGTTAACCAGGCACTTCCGAAAGCAC-3' (SEQ ID NO: 128).

As a polypeptide fusion to fingers 1 and 2 ofZif268, TG-ZFD-037 "RDER3" demonstrates recognition specificity for the 3-bp target sequence GCG and GTG as determined by *in vivo* screening results.

TG-ZFD-037 "RDER3" can be used as a module to construct a chimeric DNA binding protein comprising multiple zinc finger domains, e.g., for the purpose of recognizing a DNA site containing the sequence GCG and GTG.

### Example 47: TG-ZFD-038 "RDER4"

TG-ZFD-038 "RDER4" was identified by *in vivo* screening from human genomic sequence. Its amino acid sequence is:
FSCSWKGCERRFARSDELSRHRRTH (SEQ ID NO:131). It is encoded by the human nucleic acid sequence:
   5'-TTCAGCTGTAGCTGGAAAGGTTGTGAAAGGAGGTTTGCCCGTTCTGATG AACTGTCCAGACACAGGCGAACCCAC-3' (SEQ ID NO:130).

As a polypeptide fusion to fingers 1 and 2 of Zif268, TG-ZFD-038 "RDER4" demonstrates recognition specificity for the 3-bp target sequence GCG and GTG as determined by *in vivo* screening results.

TG-ZFD-038 "RDER4" can be used as a module to construct a chimeric DNA binding protein comprising multiple zinc finger domains, e.g., for the purpose of recognizing a DNA site containing the sequence GCG and GTG.

### Example 48: TG-ZFD-039 "RDER5"

TG-ZFD-039 "RDER5" was identified by *in vivo* screening from human genomic sequence. Its amino acid sequence is:
FACSWQDCNKKFARSDELARHYRTH (SEQ ID NO: 133). It is encoded by the human nucleic acid sequence:
   5'-TTCGCCTGCAGCTGGCAGGACTGCAACAAGAAGTTCGCGCGCTCCGAC GAGCTGGCGCGGCACTACCGCACACAC-3' (SEQ ID NO:132).

As a polypeptide fusion to fingers 1 and 2 of Zif268, TG-ZFD-039 "RDER5" demonstrates recognition specificity for the 3-bp target sequence GCG as determined by *in vivo* screening results.

TG-ZFD-039 "RDER5" can be used as a module to construct a chimeric DNA binding protein comprising multiple zinc finger domains, e.g., for the purpose of recognizing a DNA site containing the sequence GCG.

### Example 49: TG-ZFD-040 "RDER6"

TG-ZFD-040 "RDER6" was identified by *in vivo* screening from human genomic sequence. Its amino acid sequence is:
YHCNWDGCGWKFARSDELTRHYRKH (SEQ ID NO:135). It is encoded by the human nucleic acid sequence:
   5'-TACCACTGCAACTGGGACGGCTGCGGCTGGAAGTTTGCGCGCTCAGAC GAGCTCACGCGCCACTACCGAAAGCAC-3' (SEQ ID NO:134).

As a polypeptide fusion to fingers 1 and 2 of Zif168, TG-ZFD-040 "RDER6" demonstrates recognition specificity for the 3-bp target sequence GCG and GTG. Its binding site preference is GCG > GTG as determined by *in vivo* screening results.

TG-ZFD-040 "RDER6" can be used as a module to construct a chimeric DNA binding protein comprising multiple zinc finger domains, e.g., for the purpose of recognizing a DNA site containing the sequence GCG and GTG.

### Example 50: TG-ZFD-041 "RDHR1"

TG-ZFD-041 "RDHR1" was identified by *in vivo* screening from human genomic sequence. Its amino acid sequence is:
FLCQYCAQRFGRKDHLTRHIVBCICSH (SEQ ID NO: 137). It is encoded by the human nucleic acid sequence:
   5'-TTCCTCTGTCAGTATTGTGCACAGAGATTTGGGCGAAAGGATCACCTGA CTCGACATATGAAGAAGAGTCAC-3' (SEQ ID NO:136).

As a polypeptide fusion to fingers 1 and 2 of Zif268, TG-ZFD-041 "RDHR1" demonstrates recognition specificity for the 3-bp target sequence GAG and GGG as determined by *in vivo* screening results.

TG-ZFD-041 "RDHR1" can be used as a module to construct a chimeric DNA binding protein comprising multiple zinc finger domains, e.g., for the purpose of recognizing a DNA site containing the sequence GAG and GGG.

### Example 51: TG-ZFD-043 "RDHT"

TG-ZFD-043 "RDHT" was identified by *in vivo* screening from human genomic sequence. Its amino acid sequence is: FQCKTCQRKFSRSDHLKTHTRTH (SEQ ID NO:141). It is encoded by the human nucleic acid sequence:
5'-TTCCAGTGTAAAACTTGTCAGCGAAAGTTCTCCCGGTCCGACCACCTGA AGACCCACACCAGGACTCAT-3' (SEQ ID NO: 140).

As a polypeptide fusion to fingers 1 and 2 of Zif268, TG-ZFD-043 "RDHT" demonstrates recognition specificity for the 3-bp target sequence TGG, AGG, CGG, and GGG as determined by *in vivo* screening results.

TG-ZFD-043 "RDHT" can be used as a module to construct a chimeric DNA binding protein comprising multiple zinc finger domains, e.g., for the purpose of recognizing a DNA site containing the sequence TGG, AGG, CGG, and GGG.

### Example 52: TG-ZFD-044 "RDKI"

TG-ZFD-044 "RDKI" was identified by *in vivo* screening from human genomic sequence. Its amino acid sequence is: FACEVCGVRFTRNDKLKIHMRKH (SEQ ID NO:143). It is encoded by the human nucleic acid sequence:
5'-TTTGCCTGCGAGGTCTGCGGTGTTCGATTCACCAGGAACGACAAGCTGA AGATCCACATGCGGAAGCAC-3' (SEQ ID NO:142).

As a polypeptide fusion to fingers 1 and 2 of Zif268, TG-ZFD-044 "RDKI" demonstrates recognition specificity for the 3-bp target sequence GGG as determined by *in vivo* screening results.

TG-ZFD-044 "RDKI" can be used as a module to construct a chimeric DNA binding protein comprising multiple zinc finger domains, e.g., for the purpose of recognizing a DNA site containing the sequence GGG.

### Example 53: TG-ZFD-045 "RDKR"

TG-ZFD-045 "RDKR" was identified by *in vivo* screening from human genomic sequence. Its amino acid sequence is:
YVCDVEGCTWKFARSDKLNRHKKRH (SEQ ID NO*:*145). It is encoded by the human nucleic acid sequence:
   5'-TATGTATGCGATGTAGAGGGATGTACGTGGAAATTTGCCCGCTCAGATA AGCTCAACAGACACAAGAAAAGGCAC-3' (SEQ ID NO:144).

As a polypeptide fusion to fingers 1 and 2 of Zif268, TG-ZFD-045 "RDKR" demonstrates recognition specificity for the 3-bp target sequence GGG and AGG. Its binding site preference is GGG > AGG as determined by *in vivo* screening results.

TG-ZFD-045 "RDKR" can be used as a module to construct a chimeric DNA binding protein comprising multiple zinc finger domains, e.g., for the purpose of recognizing a DNA site containing the sequence GGG and AGG.

### Example 54: TG-ZFD-046 "RSNR"

TG-ZFD-046 "RSNR" was identified by *in vivo* screening from human genomic sequence. Its amino acid sequence is: YICRKCGRGFSRICSNLIRHQRTH (SEQ ID NO:147). It is encoded by the human nucleic acid sequence:
5'-TATATTTGCAGAAAGTGTGGACGGGGCTTTAGTCGGAAGTCCAACCTTA TCAGACATCAGAGGACACAC-3' (SEQ ID NO:146).

As a polypeptide fusion to fingers 1 and 2 of Zif268, TG-ZFD-046 "RSNR" demonstrates recognition specificity for the 3-bp target sequence GAG and GTG. Its binding site preference is GAG > GTG as determined *by in vivo* screening results.

TG-ZFD-046 "RSNR" can be used as a module to construct a chimeric DNA binding protein comprising multiple zinc finger domains, e.g., for the purpose of recognizing a DNA site containing the sequence GAG and GTG.

### Example 55: TG-ZFD-047 "RTNR"

TG-ZFD-047 "RTNR" was identified *by in vivo* screening from human genomic sequence. Its amino acid sequence is: YLCSECDKCFSRSTNLIRHRRTH (SEQ ID NO:149). It is encoded by the human nucleic acid sequence:
5'-TATCTATGTAGTGAGTGTGACAAATGCTTCAGTAGAAGTACAAACCTCA TAAGGCATCGAAGAACTCAC-3' (SEQ ID NO: 148).

As a polypeptide fusion to fingers 1 and 2 ofZif268, TG-ZFD-047 "RTNR" demonstrates recognition specificity for the 3-bp target sequence GAG as determined by *in vivo* screening results.

TG-ZFD-047 "RTNR" can be used as a module to construct a chimeric DNA binding protein comprising multiple zinc finger domains, e.g., for the purpose of recognizing a DNA site containing the sequence GAG.

A number of embodiments of the invention have been described. Nevertheless, it will be understood that various modifications may be made without departing from the spirit and scope of the invention. Accordingly, other embodiments are within the scope of the following claims.

## Claims

1. A method of identifying a zinc finger domain that recognizes a target site on a DNA, the method comprising:
(a) providing cells containing a reporter construct, the construct comprising a reporter gene operably linked to a promoter, wherein the reporter gene expression is activated above a given level or inhibited below a given level when a transcription factor recognizes both a recruitment site and a target site of the promoter, but not when the transcription factor recognizes only the recruitment site of the promoter;
(b) providing a plurality of hybrid nucleic acids, each of which encodes a non-naturally occurring protein comprising (i) a transcription activation or repression domain, (ii) a DNA binding domain that recognizes the recruitment site, and (iii) a test zinc finger domain, wherein the encoded amino acid sequence of the test zinc finger domain varies among the members of the plurality;
(c) contacting the plurality of hybrid nucleic acids with the cells under conditions that permit at least one of the plurality of nucleic acids to enter at least one of the cells;
(d) maintaining the cells under conditions permitting expression of the hybrid nucleic acids in the cells; and
(e) identifying a cell that contains a hybrid nucleic acid of (b) and that activates an expression of the reporter gene above the given level or inhibits the expression of the reporter gene below the given level as an indication that the cell contains a hybrid nucleic acid encoding a test zinc finger domain that recognizes the target site.

2. The method of claim 1, wherein the cells are eukaryotic cells.

3. The method of claim 2, wherein the cells are yeast cells.

4. The method of claim 3, wherein the cells are *Saccharomyces cerevisiae* cells.

5. The method of claim 1, wherein the reporter gene is a selectable marker.

6. The method of claim 5, wherein the selectable marker is selected from the group consisting of *URA3, HIS3, LEU2, ADE2,* and *TRP1.*

7. The method of claim 1, wherein the reporter gene is selected from the group consisting of lacZ, CAT, luciferase, GUS, and GFP.

8. The method of claim 1, wherein the DNA binding domain comprises a zinc finger domain.

9. The method of claim 1, wherein the DNA binding domain comprises two zinc finger domains.

10. The method .of claim 1, wherein the DNA binding domain comprises three zinc finger domains.

11. The method of claim 1, further comprising the steps of (i) amplifying a source nucleic acid encoding the test zinc finger domain from genomic nucleic acid, a messenger RNA (mRNA) mixture, or a complementary DNA (cDNA) mixture, using an oligonucleotide primer that anneals to a sequence encoding a conserved domain boundary to produce an amplified fragment; and (ii) utilizing the amplified fragment to construct a hybrid nucleic acid for inclusion in the plurality of hybrid nucleic acids of step (b).

12. The method of claim 1, further comprising the step of (i) identifying a candidate zinc finger domain amino acid sequence in a sequence database; (ii) providing a candidate nucleic acid encoding the candidate zinc finger domain amino acid sequence, and (iii) utilizing the candidate nucleic acid to construct a hybrid nucleic acid for inclusion in the plurality of hybrid nucleic acids of step (b).

13. The method of claim 5, wherein the selectable marker is an auxotrophy gene required for the synthesis of a metabolite; the genome of the cells lacks a functional copy of the auxotrophy gene; and, during step (d), the cells are maintained in a medium prepared without the metabolite.

14. The method of claim 1, wherein steps (a) to (e) are repeated to identify a second test zinc finger domain that recognizes a second target site.

15. The method of claim 14, further comprising constructing a nucleic acid encoding a polypeptide comprising the first test zinc finger domain and the second test zinc finger domain.

16. A method of identifying a zinc finger domain that recognizes a target site on a DNA, the method comprising:
(a) providing cells containing a reporter construct, the construct comprising a reporter gene operably linked to a promoter, wherein the reporter gene expression is activated above a given level or inhibited below a given level when a transcription factor recognizes both a recruitment site and a target site of the promoter, but not when the transcription factor recognizes only the recruitment site of the promoter;
(b) amplifying a plurality of nucleic acid sequences, each of which encodes a test zinc finger domain, using an oligonucleotide primer that anneals to a nucleic acid encoding a conserved domain boundary;
(c) joining each nucleic acid sequence of (b) to nucleic acid sequences encoding (i) a transcription activation or repression domain, and (ii) a DNA binding domain that recognizes the recruitment site, to form a plurality of hybrid nucleic acids;
(d) contacting the plurality of hybrid nucleic acids of (c) with the cells of (a) under conditions that permit at least one of the plurality of hybrid nucleic acids to enter at least one of the cells;
(e) maintaining the cells under conditions permitting expression of the hybrid nucleic acids in the cells; and
(f) identifying a cell that contains a hybrid nucleic acid of (c) and that activates an expression of the reporter gene above the given level or inhibits the expression of the reporter gene below the given level, wherein the hybrid nucleic acid encodes a zinc finger domain that recognizes the target site on a DNA.

17. The method of claim 16, wherein the cells are yeast cells.

18. The method of claim 16, wherein the reporter gene is selected from the group consisting of *lacZ*, CAT luciferase, GUS, and GFP.

19. The method of claim 16, wherein the DNA binding domain comprises a zinc finger domain.

20. The method of claim 16, wherein the DNA binding domain comprises two zinc finger domains.

21. A method of determining whether a test zinc finger domain recognizes a target site on a promoter, the method comprising:
(a) providing a reporter construct comprising a reporter gene operably linked to a promoter, wherein the reporter gene expression is activated above a given level or inhibited below a given level when a transcription factor recognizes both a recruitment site and a target site of the promoter, but not when the transcription factor recognizes only the recruitment site of the promoter;
(b) providing a hybrid nucleic acid that encodes a non-naturally occurring protein comprising (i) a transcription activation or repression domain, (ii) a DNA binding domain that recognizes the recruitment site, and (iii) a test zinc finger domain;
(c) contacting the reporter construct with a cell under conditions that permit the reporter construct to enter the cell;
(d) prior to, after, or concurrent with step (c), contacting the hybrid nucleic acid with the cell under conditions that permit the hybrid nucleic acid to enter the cell;
(e) maintaining the cell under conditions permitting expression of the hybrid nucleic acid in the cell; and
(f) detecting differences in the reporter gene expression in the cell, wherein a difference in a level of reporter gene expression greater than the given level or lower than the given level is an indication that the test zinc finger domain recognizes the target site.

22. The method of claim 21, further comprising the step of amplifying a nucleic acid encoding the test zinc finger domain from genomic DNA, an mRNA mixture or a cDNA mixture using an oligonucleotide primer that anneals to a sequence encoding a conserved domain boundary.

23. The method of claim 21, further comprising the steps of (i) identifying a candidate zinc finger domain amino acid sequence in a sequence database; (ii) providing a candidate nucleic acid encoding the candidate zinc finger domain amino acid sequence, and (iii) utilizing the candidate nucleic acid to construct a hybrid nucleic acid for inclusion in the plurality of hybrid nucleic acids of step (b).

24. A method of determining whether a test zinc finger domain recognizes a target site on a promoter, the method comprising:
(a) providing a first cell comprising a reporter construct comprising a reporter gene operably linked to a promoter, wherein the reporter gene expression is activated above a given level or inhibited below a given level when a transcription factor recognizes both a recruitment site and a target site of the promoter, but not when the transcription factor recognizes only the recruitment site of the promoter;
(b) providing a second cell comprising a hybrid nucleic acid that encodes a protein comprising (i) a transcription activation or repression domain, (ii) a DNA binding domain that recognizes the recruitment binding site, and (iii) a test zinc finger domain;
(c) fusing the first and second cells to form a fused cell;
(d) maintaining the fused cell under conditions permitting expression of the hybrid nucleic acids in the cell; and
(e) detecting differences in the reporter gene expression in the fused cell, wherein a difference in a level of reporter gene expression greater than the given level or lower than the given level is an indication that the test zinc finger domain recognizes the target site.

25. The method of claim 24, wherein the first and second cells are yeast cells of the opposite mating types.

26. A method of determining whether a test zinc finger domain recognizes a target site on a promoter, the method comprising:
(a) providing a plurality of reporter constructs, each construct comprising a reporter gene operably linked to a promoter, wherein the reporter gene expression is activated above a given level or inhibited below a given level when a transcription factor recognizes both a recruitment site and a target site of the promoter, but not when the transcription factor recognizes only the recruitment site of the promoter;
(b) providing a cell containing a hybrid nucleic acid, that encodes a non-naturally occurring protein comprising (i) a transcription activation or repression domain, (ii) a DNA binding domain that recognizes the recruitment site, and (iii) a test zinc finger domain;
(c) contacting the plurality of reporter constructs with the cell under conditions that permit at least one of the plurality of reporter constructs to enter the cell;
(d) maintaining the cell under conditions permitting expression of the hybrid nucleic acid in the cell; and
(e) identifying a cell that contains a reporter gene of (a) and that activates the expression of the reporter gene above the given level or inhibits the expression of the reporter gene below the given level as an indication that the reporter construct in the cell comprises a target site recognized by the test zinc finger domain.

27. The method of claim 26, wherein the target binding site is between two and six nucleotides long.

28. The method of claim 27, wherein the plurality of reporter constructs comprises every possible combination of A, T, G, and C nucleotides at at least two positions of the target binding site.

29. The method of claim 28, wherein the plurality of reporter constructs comprises every possible combination of A, T, G, and C nucleotides at at least three positions of the target binding sites.

30. The method of claim 26, wherein steps (a) to (e) are repeated for a second test zinc finger domain to identify a second binding preference.

31. The method of claim 30, further comprising constructing a nucleic acid encoding a polypeptide comprising the first second test zinc finger domains.

32. A method of identifying a plurality of zinc finger domains, the method comprising:
carrying out the method of claim 1 to identify a first test zinc finger domain; and
carrying out the method of claim 1 again to identify a second test zinc finger domain that recognizes a target site different from the target site recognized by the first test zinc finger domain.

33. A method of generating a nucleic acid encoding a chimeric zinc finger protein, the method comprising:
carrying out the method of claim 32;
constructing a nucleic acid encoding a polypeptide comprising the first and second test zinc finger domains.

34. A method of identifying DNA sequences recognized by zinc finger domains, the method comprising:
carrying out the method of claim 24 to identify a first target site recognized by a first test zinc finger domain; and
carrying out the method of claim 24 again to identify a second target site recognized by a second test zinc finger domain.

35. A method of generating a nucleic acid encoding a chimeric zinc finger protein, the method comprising:
carrying out the method of claim 34;
constructing a nucleic acid encoding a polypeptide comprising the first and second test zinc finger domains.

36. The method according to claim 1, wherein the identified zinc finger domain comprises the amino acid sequence:
Xₐ-X-Cys-X₂₋₅-Cys-X₃-Xₐ-X-Cys-X-Ser-Asn-X_{b}-X-Arg-His-X₃₋₅-His (SEQ ID NO: 68),
wherein Xₐ is phenylalanine or tyrosine, and X_{b} is a hydrophobic residue.

37. The method according to claim 1, wherein the hybrid nucleic acid encoding the test zinc finger domain is a nucleic acid sequence encoding the amino acid sequence of claim 36.

38. The method according to claim 1, wherein the identified zinc finger domain comprises the amino acid sequence:
Xₐ-X-Cys-X₂₋₅-Cys-X₃-Xa-X-His-X-Ser-Asn-X_{b}-X-Lys-His-X₃₋₅-His (SEQ ID NO: 69),
wherein Xₐ is a phenylalanine or tyrosine, and X_{b} is a hydrophobic residue.

39. The method according to claim 1, wherein the hybrid nucleic acid encoding the test zinc finger domain is a nucleic acid sequence encoding the amino acid sequence of claim 38.

40. The method according to claim 1, wherein the identified zinc finger domain comprises the amino acid sequence:
Xₐ-X-Cys-X₂₋₅-Cys-X₃-Xₐ-X-Ser-X-Ser-Asn-X_{b}-X-Arg-His-X₃₋₅-His (SEQ ID NO: 70),
wherein Xₐ is a phenylalanine or tyrosine, and X_{b} is a hydrophobic residue.

41. The method according to claim 1, wherein the hybrid nucleic acid encoding the test zinc finger domain is a nucleic acid sequence encoding the amino acid sequence of claim 40.

42. The method according to claim 1, wherein the identified zinc finger domain comprises the amino acid sequence:
Xₐ-X-Cys-X₂-₅-Cys-X₃-Xₐ-X-Gh-X-Ser-Thr-X_{b}-X-Vat-His-X₃.₅-His (SEQ ID NO: 71),
wherein Xₐ is a phenylalanine or tyrosine, and X_{b} is a hydrophobic residue.

43. The method according to claim 1, wherein the hybrid nucleic acid encoding the test zinc finger domain is a nucleic acid sequence encoding the amino acid sequence of claim 42.

44. The method according to claim 1, wherein the identified zinc finger domain comprises the amino acid sequence:
Xₐ-X-Cys-X₂₋₅-Cys-X₃-Xₐ-X-Val-X-Ser-X_{c}-X_{b}-X-Arg-His-X₃₋₅-His (SEQ ID NO: 72),
wherein Xₐ is a phenylalanine or tyrosine, X_{b} is a hydrophobic residue, and X_{c} is serine or threonine.

45. The method according to claim 1, wherein the hybrid nucleic acid encoding the test zinc finger domain is a nucleic acid sequence encoding the amino acid sequence of claim 44.

46. The method according to claim 1, wherein the identified zinc finger domain comprises the amino acid sequence:
Xₐ-X-Cys-X₂₋₅-Cys-X₃-Xₐ-X-Gln-X-Ser-His-X_{b}-X-Arg-His-X₃₋₅-His (SEQ ID NO: 73),
wherein Xₐ is a phenylalanine or tyrosine, and X_{b} is a hydrophobic residue.

47. The method according to claim 1, wherein the hybrid nucleic acid encoding the test zinc finger domain is a nucleic acid sequence encoding the amino acid sequence of claim 46.

48. The method according to claim 1, wherein the identified zinc finger domain comprises the amino acid sequence:
Xₐ-X-Cys-X₂₋₅-Cys-X₃-Xₐ-X-Gln-X-Ser-Asn-X_{b}-X-Val-His-X₃₋₅-His (SEQ ID NO: 74),
wherein Xₐ is a phenylalanine or tyrosine, and X_{b} is a hydrophobic residue.

49. The method according to claim 1; wherein the hybrid nucleic acid encoding the test zinc finger domain is a nucleic acid sequence encoding the amino acid sequence of claim 48.

50. The method according to claim 1, wherein the identified zinc finger domain comprises the amino acid sequence:
Xₐ-X-Cys-X₂₋₅-Cys-X₃-Xₐ-X-Gln-X-Ser-X_{c}-X_{b}-X-Arg-His-X₃₋₅-His (SEQ ID NO: 75),
wherein Xₐ is a phenylalanine or tyrosine, and X_{b} is a hydrophobic residue, and X_{c} is serine or threonine.

51. The method according to claim 1, wherein the hybrid nucleic acid encoding the test zinc finger domain is a nucleic acid sequence encoding the amino acid sequence of claim 50.

52. The method according to claim 1, wherein the identified zinc finger domain comprises an amino acid sequence 50%, 60%, 70%, 80%, 90%, 93%, 95%, 96%, 98%, 99% or 100% identical to SEQ ID NO: 65.

53. The method according to claim 1, wherein the hybrid nucleic acid encoding the test zinc finger domain is a nucleic acid sequence encoding the amino acid sequence of claim 52.

54. The method according to claim 1, wherein the identified zinc finger domain comprises an amino acid sequence 50%, 60%, 70%, 80%, 90%, 93%, 95%, 96%, 98%, 99% or 100% identical to an amino acid sequence selected from the group consisting of: SEQ ID NO: 29, 127, 129, 131, 133, and 135.

55. The method according to claim 1, wherein the hybrid nucleic acid encoding the test zinc finger domain is a nucleic acid sequence encoding the amino acid sequence of claim 54.

56. The method according to claim 1, wherein the identified zinc finger domain comprises the amino acid sequence:
Xₐ-X-Cys-X₂₋₅-Cys-X₃-Xₐ-X-Gln-X-Ala-His-X_{b}-X-Arg-His-X₃₋₅-His (SEQ ID NO: 150),
wherein Xₐ is a phenylalanine or tyrosine, and X_{b} is a hydrophobic residue.

57. The method according to claim 1, wherein the hybrid nucleic acid encoding the test zinc finger domain is a nucleic acid sequence encoding the amino acid sequence of claim 56.

58. The method according to claim 1, wherein the identified zinc finger domain comprises the amino acid sequence:
Xₐ-X-Cys-X₂₋₅-Cys-X₃-Xₐ-X-Gln-X-Phe-Asn-X_{b}-X-Arg-His-X₃₋₅-His (SEQ ID NO: 151),
wherein Xₐ is a phenylalanine or tyrosine, and X_{b} is a hydrophobic residue.

59. The method according to claim 1, wherein the hybrid nucleic acid encoding the test zinc finger domain is a nucleic acid sequence encoding the amino acid sequence of claim 58.

60. The method according to claim 1, wherein the identified zinc finger domain comprises the amino acid sequence:
Xₐ-X-Cys-X₂₋₅-Cys-X₃-Xa-X-Gln-X-Ser-His-X_{b}-X-Thr-His-X₃₋₅-His (SEQ ID NO: 152),
wherein Xₐ is a phenylalanine or tyrosine, and X_{b} is a hydrophobic residue.

61. The method according to claim 1, wherein the hybrid nucleic acid encoding the test zinc finger domain is a nucleic acid sequence encoding the amino acid sequence of claim 60.

62. The method according to claim 1, wherein the identified zinc finger domain comprises the amino acid sequence:
Xₐ-X-Cys-X₂₋₅-Cys-X₃-Xₐ-X-Gln-X-Ser-His-X_{b}-X-Val-His-X₃₋₅-His (SEQ ID NO: 153),
wherein Xₐ is a phenylalanine or tyrosine, and X_{b} is a hydrophobic residue.

63. The method according to claim 1, wherein the hybrid nucleic acid encoding the test zinc finger domain is a nucleic acid sequence encoding the amino acid sequence of claim 62.

64. The method according to claim 1, wherein the identified zinc finger domain comprises the amino acid sequence:
Xₐ-X-Cys-X₂₋₅-Cys-X₃-Xₐ-X-Gln-X-Ser-Asn-X_{b}-X-Ile-His-X₃₋₅-His (SEQ ID NO: 154),
wherein Xₐ is a phenylalanine or tyrosine, and X_{b} is a hydrophobic residue.

65. The method according to claim 1, wherein the hybrid nucleic acid encoding the test zinc finger domain is a nucleic acid sequence encoding the amino acid sequence of claim 64.

66. The method according to claim 1, wherein the identified zinc finger domain comprises the amino acid sequence:
Xₐ-X-Cys-X₂₋₅-Cys-X₃-Xₐ-X-Gln-X-Ser-Asn-X_{b}-X-Arg-His-X₃₋₅-His (SEQ ID NO: 155),
wherein Xₐ is a phenylalanine or tyrosine, and X_{b} is a hydrophobic residue.

67. The method according to claim 1, wherein the hybrid nucleic acid encoding the test zinc finger domain is a nucleic acid sequence encoding the amino acid sequence of claim 66.

68. The method according to claim 1, wherein the identified zinc finger domain comprises the amino acid sequence:
Xₐ-X-Cys-X₂₋₅-Cys-X₃-Xₐ-X-Gln-X-Thr-His-X_{b}-X-Gln-His-X₃₋₅-His (SEQ ID NO: 156),
wherein Xₐ is a phenylalanine or tyrosine, and X_{b} is a hydrophobic residue.

69. The method according to claim 1, wherein the hybrid nucleic acid encoding the test zinc finger domain is a nucleic acid sequence encoding the amino acid sequence of claim 68.

70. The method according to claim 1, wherein the identified zinc finger domain comprises the amino acid sequence:
Cys-X₂₋₅-Cys-X₃-Xₐ-X-Gln-X-Thr-His-X_{b}-X-Arg-His-X₃₋₅-His (SEQ ID NO: 157),
wherein Xₐ is a phenylalanine or tyrosine, and X_{b} is a hydrophobic residue.

71. The method according to claim 1, wherein the hybrid nucleic acid encoding the test zinc finger domain is a nucleic acid sequence encoding the amino acid sequence of claim 70.

72. The method according to claim 1, wherein the identified zinc finger domain comprises the amino acid sequence:
Xₐ-X-Cys-X₂₋₅-Cys-X₃-Xₐ-X-Arg-X-Asp-Lys-X_{b}-X-Ile-His-X₃₋₅-His (SEQ ID NO: 158),
wherein Xₐ is a phenylalanine or tyrosine, and X_{b} is a hydrophobic residue.

73. The method according to claim 1. wherein the hybrid nucleic acid encoding the test zinc finger domain is a nucleic acid sequence encoding the amino acid sequence of claim 72.

74. The method according to claim 1, wherein the identified zinc finger domain comprises the amino acid sequence:
Xₐ-X-Cys-X₂₋₅-Cys-X₃-Xₐ-X-Arg-X-Ser-Asn-X_{b}-X-Arg-His-X₃₋₅-His (SEQ ID NO: 159),
wherein Xₐ is a phenylalanine or tyrosine, and X_{b} is a hydrophobic residue.

75. The method according to claim 1, wherein the hybrid nucleic acid encoding the test zinc finger domain is a nucleic acid sequence encoding the amino acid sequence of claim 74.

76. The method according to claim 1, wherein the identified zinc finger domain comprises an amino acid sequence 50%, 60%, 70%, 80%, 90%, 93%, 95%, 96%, 98%, 99% or 100% identical to SEQ ID NO: 137.

77. The method according to claim 1, wherein the hybrid nucleic acid encoding the test zinc finger domain is a nucleic acid sequence encoding the amino acid sequence of claim 76.

78. The method according to claim 1, wherein the identified zinc finger domain comprises an amino acid sequence 50%, 60%, 70%, 80%, 90%, 93%, 95%, 96%, 98%, 99% or 100% identical to SEQ ID NO:145.

79. The method according to claim 1, wherein the hybrid nucleic acid encoding the test zinc finger domain is a nucleic acid sequence encoding the amino acid sequence of claim 78.

80. The method according to claim 1, wherein the identified zinc finger domain comprises an amino acid sequence 50%, 60%, 70%, 80%, 90%, 93%, 95%, 96%, 98%, 99% or 100% identical to SEQ ID NO: 149.

81. The method according to claim 1, wherein the hybrid nucleic acid encoding the test zinc finger domain is a nucleic acid sequence encoding the amino acid sequence of claim 80.

82. The method according to claim 1, wherein the identified zinc finger domain comprises an amino acid sequence 50%, 60%, 70%, 80%, 90%, 93%, 95%, 96%, 98%, 99% or 100% identical to SEQ ID NO: 141.

83. The method according to claim 1, wherein the hybrid nucleic acid encoding the test zinc finger domain is a nucleic acid sequence encoding the amino acid sequence of claim 82.

## Patentansprüche

1. Verfahren zur Identifizierung einer Zinkfingerdomäne, die eine Zielstelle auf einer DNA erkennt, wobei das Verfahren umfasst:
(a) Bereitstellung von Zellen, die ein Reporterkonstrukt enthalten, wobei das Konstrukt ein mit einem Promoter auf operative Weise verbundenes Reportergen umfasst, in der die Expression des Reportergens über ein gegebenes Niveau hinaus aktiviert oder unter ein gegebenes Niveau hinaus gehemmt ist, wenn ein Transkriptionsfaktor sowohl eine Rekrutierungsstelle als auch eine Zielstelle des Promoters erkennt, aber nicht, wenn der Transkriptionsfaktor nur die Rekrutierungsstelle des Promoters erkennt,
(b) Bereitstellung einer Vielzahl von Hybridnukleinsäuren, die jeweils den Code für ein nicht natürlich vorkommendes Protein tragen, das (i) eine Transkriptionsaktivierungs- oder repressionsdomäne, (ii) eine die Rekrutierungsstelle erkennende DNA-Bindungsdomäne und (iii) eine Testzinkfingerdomäne umfasst, wobei sich der Code für die Aminosäuresequenz der Testzinkfingerdomäne bei den Mitgliedern der Vielzahl unterscheidet,
(c) in Kontakt bringen der Vielzahl von Hybridnukleinsäuren mit den Zellen unter Bedingungen, die es mindestens einer aus der Vielzahl von Nukleinsäuren erlauben, in mindestens eine der Zellen einzudringen,
(d) Kultivierung der Zellen unter Bedingungen, die die Expression der Hybridnukleinsäuren in den Zellen erlauben, und
(e) Identifizierung einer Zelle, die eine der Hybridnukleinsäuren von (b) enthält und in der eine Expression des Reportergens über das gegebene Niveau hinaus aktiviert oder die Expression des Reportergens unter das gegebene Niveau hinaus gehemmt ist, was darauf hinweist, dass die Zelle eine Hybridnukleinsäure mit dem Code für eine die Zielstelle erkennende Testzinkfingerdomäne enthält.

2. Verfahren gemäß Anspruch 1, wobei es sich bei den Zellen um eukaryotische Zellen handelt.

3. Verfahren gemäß Anspruch 2, wobei es sich bei den Zellen um Hefezellen handelt.

4. Verfahren gemäß Anspruch 3, wobei es sich bei den Zellen um *Saccharomyces cerevisiae*-Zellen handelt.

5. Verfahren gemäß Anspruch 1, wobei es sich bei dem Reportergen um einen selektierbaren Marker handelt.

6. Verfahren gemäß Anspruch 5, wobei der selektierbare Marker aus der aus *URA3, HIS3, LEU2, ADE2* und *TRP1* bestehenden Gruppe ausgewählt wird.

7. Verfahren gemäß Anspruch 1, wobei das Reportergen aus der aus *LacZ,* CAT, Luciferase, GUS und GFP bestehenden Gruppe ausgewählt wird.

8. Verfahren gemäß Anspruch 1, wobei die DNA-Bindungsdomäne eine Zinkfingerdomäne umfasst.

9. Verfahren gemäß Anspruch 1, wobei die DNA-Bindungsdomäne zwei Zinkfingerdomänen umfasst.

10. Verfahren gemäß Anspruch 1, wobei die DNA-Bindungsdomäne drei Zinkfingerdomänen umfasst.

11. Verfahren gemäß Anspruch 1, das darüber hinaus folgende Schritte umfasst: (i) Amplifizierung einer Quellnukleinsäure mit dem Code für die Testzinkfingerdomäne, aus genomischer Nukleinsäure, einer Messenger RNA (mRNA) Mischung, oder einer komplementären DNA (cDNA) Mischung unter Verwendung eines Oligonukleotidprimers, der an eine Sequenz mit dem Code für einen Randbereich einer konservierten Domäne bindet, um ein amplifiziertes Fragment herzustellen, und (ii) Nutzung des amplifizierten Fragments zur Konstruktion einer Hybridnukleinsäure zur Einbeziehung in die Vielzahl von Hybridnukleinsäuren von Schritt (b).

12. Verfahren gemäß Anspruch 1, das darüber hinaus folgende Schritte umfasst: (i) Identifizierung einer Kandidatenaminosäuresequenz einer Zinkfingerdomäne in einer Sequenzdatenbank, (ii) Bereitstellung einer Kandidatennukleinsäure mit dem Code für die Kandidatenaminosäuresequenz der Zinkfingerdomäne und (iii) Nutzung der Kandidatennukleinsäure zur Konstruktion einer Hybridnukleinsäure zur Einbeziehung in die Vielzahl von Hybridnukleinsäuren von Schritt (b).

13. Verfahren gemäß Anspruch 5, wobei es sich bei dem selektierbaren Marker um ein für die Synthese eines Metaboliten erforderliches Auxotrophiegen handelt; das Genom der Zellen keine funktionelle Kopie des Auxotrophiegens enthält und in Schritt (d) die Zellen in einem Medium kultiviert werden, das ohne den Metaboliten zubereitet wurde.

14. Verfahren gemäß Anspruch 1, wobei die Schritte (a) bis (e) wiederholt werden, um eine zweite Testzinkfingerdomäne zu identifizieren, die eine zweite Zielstelle erkennt.

15. Verfahren gemäß Anspruch 14, das darüber hinaus die Konstruktion einer Nukleinsäure mit dem Code für ein Polypeptid umfasst, das die erste Testzinkfingerdomäne und die zweite Testzinkfingerdomäne umfasst.

16. Verfahren zur Identifizierung einer Zinkfingerdomäne, die eine Zielstelle auf einer DNA erkennt, wobei das Verfahren umfasst:
(a) Bereitstellung von Zellen, die ein Reporterkonstrukt enthalten, wobei das Konstrukt ein mit einem Promoter auf operative Weise verbundenes Reportergen umfasst, in der die Expression des Reportergens über ein gegebenes Niveau hinaus aktiviert oder unter ein gegebenes Niveau hinaus gehemmt ist, wenn ein Transkriptionsfaktor sowohl eine Rekrutierungsstelle als auch eine Zielstelle des Promoters erkennt, aber nicht, wenn der Transkriptionsfaktor nur die Rekrutierungsstelle des Promoters erkennt,
(b) Amplifizierung einer Vielzahl von Nukleinsäuresequenzen, jeweils mit dem Code für eine Testzinkfingerdomäne, unter Verwendung eines Oligonukleotidprimers, der an eine Nukleinsäure mit dem Code für einen Randbereich einer konservierten Domäne bindet,
(c) Verbindung der einzelnen Nukleinsäuresequenzen von (b) mit Nukleinsäuresequenzen mit dem Code für (i) eine Transkriptionsaktivierungs- oder -repressionsdomäne und (ii) eine die Rekrutierungsstelle erkennende DNA-Bindungsdomäne, um eine Vielzahl von Hybridnukleinsäuren zu bilden,
(d) in Kontakt bringen der Vielzahl von Hybridnukleinsäuren von (c) mit den Zellen von (a) unter Bedingungen, die es mindestens einer aus der Vielzahl von Nukleinsäuren erlauben, in mindestens eine der Zellen einzudringen,
(e) Kultivierung der Zellen unter Bedingungen, die die Expression der Hybridnukleinsäuren in den Zellen erlauben, und
(f) Identifizierung einer Zelle, die eine der Hybridnukleinsäuren von (c) enthält und in der eine Expression des Reportergens über das gegebene Niveau hinaus aktiviert oder die Expression des Reportergens unter das gegebene Niveau hinaus gehemmt ist, wobei die Hybridnukleinsäure den Code für eine Testzinkfingerdomäne enthält, die die Zielstelle auf der DNA erkennt.

17. Verfahren gemäß Anspruch 16, wobei es sich bei den Zellen um Hefezellen handelt.

18. Verfahren gemäß Anspruch 16, wobei das Reportergen aus der aus *LacZ,* CAT, Luciferase, GUS und GFP bestehenden Gruppe ausgewählt ist.

19. Verfahren gemäß Anspruch 16, wobei die DNA-Bindungsdomäne eine Zinkfingerdomäne umfasst.

20. Verfahren gemäß Anspruch 16, wobei die DNA-Bindungsdomäne zwei Zinkfingerdomänen umfasst.

21. Verfahren zur Ermittlung, ob eine Testzinkfingerdomäne eine Zielstelle auf einem Promoter erkennt, wobei das Verfahren umfasst:
(a) Bereitstellung eines Reporterkonstrukts, das ein mit einem Promoter auf operative Weise verbundenes Reportergen umfasst, in der die Expression des Reportergens über ein gegebenes Niveau hinaus aktiviert oder unter ein gegebenes Niveau hinaus gehemmt ist, wenn ein Transkriptionsfaktor sowohl eine Rekrutierungsstelle als auch eine Zielstelle des Promoters erkennt, aber nicht, wenn der Transkriptionsfaktor nur die Rekrutierungsstelle des Promoters erkennt,
(b) Bereitstellung einer Hybridnukleinsäure mit dem Code für ein nicht natürlich vorkommendes Protein, das (i) eine Transkriptionsaktivierungs- oder -repressionsdomäne, (ii) eine die Rekrutierungsstelle erkennende DNA-Bindungsdomäne und (iii) eine Testzinkfingerdomäne umfasst,
(c) in Kontakt bringen des Reporterkonstrukts mit einer Zelle unter Bedingungen, die dem Reporterkonstrukt das Eindringen in die Zelle erlauben,
(d) vor, nach oder gleichzeitig mit Schritt (c), in Kontakt bringen der Hybridnukleinsäure mit der Zelle unter Bedingungen, die der Hybridnukleinsäure das Eindringen in die Zelle erlauben,
(e) Kultivierung der Zelle unter Bedingungen, die die Expression der Hybridnukleinsäure in der Zelle erlauben, und
(f) Feststellung von Unterschieden bei der Reportergenexpression in der Zelle, wobei ein Unterschied bei einem Niveau der Reportergenexpression über das gegebene Niveau hinaus oder unter das gegebene Niveau hinaus einen Hinweis darstellt, dass die Testzinkfirigerdomäne die Zielstelle erkennt.

22. Verfahren gemäß Anspruch 21, darüber hinaus umfassend den Schritt der Amplifizierung einer Nukleinsäure mit dem Code für die Testzinkfingerdomäne aus genomischer Nukleinsäure, einer mRNA-Mischung, oder einer cDNA-Mischung unter Verwendung eines Oligonukleotidprimers, der an eine Sequenz mit dem Code für einen Randbereich einer konservierten Domäne bindet.

23. Verfahren gemäß Anspruch 21, darüber hinaus umfassend die Schritte (i) der Identifizierung einer Kandidatenaminosäuresequenz einer Zinkfingerdomäne in einer Sequenzdatenbank, (ii) Bereitstellung einer Kandidatennukleinsäure mit dem Code für die Kandidatenaminosäuresequenz der Zinkfingerdomäne und (iii) Nutzung der Kandidatennukleinsäure zur Konstruktion einer Hybridnukleinsäure zur Einbeziehung in die Vielzahl von Hybridnukleinsäuren von Schritt (b).

24. Verfahren zur Ermittlung, ob eine Testzinkfingerdomäne eine Zielstelle an einem Promoter erkennt, wobei das Verfahren umfasst:
(a) Bereitstellung einer ersten Zelle, die ein Reporterkonstrukt umfasst, das ein mit einem Promoter auf operative Weise verbundenes Reportergen umfasst, in der die Expression des Reportergens über ein gegebenes Niveau hinaus aktiviert oder unter ein gegebenes Niveau hinaus gehemmt ist, wenn ein Transkriptionsfaktor sowohl eine Rekrutierungsstelle als auch eine Zielstelle des Promoters erkennt, aber nicht, wenn der Transkriptionsfaktor nur die Rekrutierungsstelle des Promoters erkennt,
(b) Bereitstellung einer zweiten Zelle, die eine Hybridnukleinsäure mit dem Code für ein Protein umfasst, das (i) eine Transkriptionsaktivierungs- oder -repressionsdomäne, (ii) eine die Rekrutierungs-Bindungsstelle erkennende DNA-Bindungsdomäne und (iii) eine Testzinkfingerdomäne umfasst,
(c) Fusion der ersten und der zweiten Zelle unter Bildung einer fusionierten Zelle,
(d) Kultivierung der fusionierten Zelle unter Bedingungen, die die Expression der Hybridnukleinsäuren in der Zelle erlauben, und
(e) Feststellung von Unterschieden bei der Reportergenexpression in der fusionierten Zelle, wobei ein Unterschied bei einem Niveau der Reportergenexpression über das gegebene Niveau hinaus oder unter das gegebene Niveau hinaus einen Hinweis darstellt, dass die Testzinkfingerdomäne die Zielstelle erkennt.

25. Verfahren gemäß Anspruch 24, wobei es sich bei der ersten und der zweiten Zelle um Hefezellen mit gegensätzlichem Paarungstyp handelt.

26. Verfahren zur Ermittlung, ob eine Testzinkfingerdomäne eine Zielstelle an einem Promoter erkennt, wobei das Verfahren umfasst:
(a) Bereitstellung einer Vielzahl von Reporterkonstrukten, die jeweils ein mit einem Promoter auf operative Weise verbundenes Reportergen umfassen, bei denen die Expression des Reportergens über ein gegebenes Niveau hinaus aktiviert oder unter ein gegebenes Niveau hinaus gehemmt ist, wenn ein Transkriptionsfaktor sowohl eine Rekrutierungsstelle als auch eine Zielstelle des Promoters erkennt, aber nicht, wenn der Transkriptionsfaktor nur die Rekrutierungsstelle des Promoters erkennt,
(b) Bereitstellung einer Zelle, die eine Hybridnukleinsäure mit dem Code für ein nicht natürlich vorkommendes Protein enthält, das (i) eine Transkriptionsaktivierungs- oder -repressionsdomäne, (ii) eine die Rekrutierungsstelle erkennende DNA-Bindungsdomäne und (iii) eine Testzinkfingerdomäne umfasst,
(c) in Kontakt bringen der Vielzahl von Reporterkonstrukten mit der Zelle unter Bedingungen, die mindestens einem aus der Vielzahl der Reporterkonstrukte das Eindringen in die Zelle erlauben,
(d) Kultivierung der Zelle unter Bedingungen, die die Expression der Hybridnukleinsäure in der Zelle erlauben, und
(e) Identifizierung einer Zelle, die ein Reportergen aus (a) enthält und die Expression des Reportergens über das gegebene Niveau hinaus aktiviert oder die Expression des Reportergens unter das gegebene Niveau hinaus hemmt, was darauf hinweist, dass das Reporterkonstrukt in der Zelle eine Zielstelle umfasst, die von der Testzinkfingerdomäne erkannt wird.

27. Verfahren gemäß Anspruch 26, wobei die Größe der Zielbindungsstelle zwischen zwei und sechs Nukleotide beträgt.

28. Verfahren gemäß Anspruch 27, wobei die Vielzahl von Reporterkonstrukten alle möglichen Kombinationen der Nukleotide A, T, G und C an mindestens zwei Positionen der Zielbindungsstelle umfasst.

29. Verfahren gemäß Anspruch 28, wobei die Vielzahl von Reporterkonstrukten alle möglichen Kombinationen der Nukleotide A, T, G und C an mindestens drei Positionen der Zielbindungsstellen umfasst.

30. Verfahren gemäß Anspruch 26, wobei die Schritte (a) bis (e) für eine zweite Testzinkfingerdomäne wiederholt werden, um eine zweite Bindungspräferenz zu identifizieren.

31. Verfahren gemäß Anspruch 30, darüber hinaus umfassend die Konstruktion einer Nukleinsäure mit dem Code für ein Polypeptid, das die erste und zweite Testzinkfingerdomäne umfasst.

32. Verfahren zur Identifizierung einer Vielzahl von Zinkfingerdomänen, wobei das Verfahren umfasst:
Durchführung des Verfahrens gemäß Anspruch 1 zur Identifizierung einer ersten Testzinkfingerdomäne, und
erneute Durchführung des Verfahrens gemäß Anspruch 1 zur Identifizierung einer zweiten Testzinkfingerdomäne, die eine Zielstelle erkennt, die sich von der Zielstelle unterscheidet, die von der ersten Testzinkfingerdomäne erkannt wird.

33. Verfahren zur Herstellung einer Nukleinsäure mit dem Code für ein chimäres Zinkfingerprotein, wobei das Verfahren umfasst:
Durchführung des Verfahrens gemäß Anspruch 32,
Konstruktion einer Nukleinsäure mit dem Code für ein Polypeptid, das die erste und die zweite Testzinkfingerdomäne umfasst.

34. Verfahren zur Identifizierung von DNA-Sequenzen, die von Zinkfingerdomänen erkannt werden, wobei das Verfahren umfasst:
Durchführung des Verfahrens gemäß Anspruch 24 zur Identifizierung einer ersten Zielstelle, die von einer ersten Testzinkfingerdomäne erkannt wird, und
erneute Durchführung des Verfahrens gemäß Anspruch 24 zur Identifizierung einer zweiten Zielstelle, die von einer zweiten Testzinkfingerdomäne erkannt wird.

35. Verfahren zur Herstellung einer Nukleinsäure mit dem Code für ein chimäres Zinkfingerprotein, wobei das Verfahren umfasst:
Durchführung des Verfahrens gemäß Anspruch 34,
Konstruktion einer Nukleinsäure mit dem Code für ein Polypeptid, das die erste und die zweite Zinkfingerdomäne umfasst.

36. Verfahren gemäß Anspruch 1, wobei die identifizierte Zinkfingerdomäne die Aminosäuresequenz:
Xₐ-X-Cys-X₂₋₅-Cys-X₃-Xₐ-X-Cys-X-Ser-Asn-X_{b}-X-Arg-His-X₃₋₅-His (SEQ. ID. NR. 68) umfasst,
wobei es sich bei Xₐ um Phenylalanin oder Tyrosin und bei X_{b} um einen hydrophoben Rest handelt.

37. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 36 handelt.

38. Verfahren gemäß Anspruch 1, wobei die identifizierte Zinkfingerdomäne die Aminosäuresequenz:
Xₐ-X-Cys-X₂₋₅-Cys-X₃-Xₐ-X-His-X-Ser-Asn-X_{b}-X-Lys-His-X₃₋₅-His (SEQ. ID. NR. 69) umfasst,
wobei es sich bei Xₐ um ein Phenylalanin oder Tyrosin und bei X_{b} um einen hydrophoben Rest handelt.

39. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 38 handelt.

40. Verfahren gemäß Anspruch 1, wobei die identifizierte Zinkfingerdomäne die Aminosäuresequenz:
Xₐ-X-Cys-X₂₋₅-Cys-X₃-Xₐ-X-Ser-X-Ser-Asn-X_{b}-X-Arg-His-X₃₋₅-His (SEQ. ID. NR. 70) umfasst,
wobei es sich bei Xₐ um ein Phenylalanin oder Tyrosin und bei X_{b} um einen hydrophoben Rest handelt.

41. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 40 handelt.

42. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-Thr-X_{b}-X-Val-His-X₃₋₅-His (SEQ. ID. NR. 71) umfasst,
wobei es sich bei Xₐ um ein Phenylalanin oder Tyrosin und bei X_{b} um einen hydrophoben Rest handelt.

43. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 42 handelt.

44. Verfahren gemäß Anspruch 1, wobei die identifizierte Zinkfingerdomäne die Aminosäuresequenz:
Xₐ-X-Cys-X₂₋₅-Cys-X₃-Xₐ-X-Val-X-Ser-X_{c}-X_{b}-X-Arg-His-X₃₋₅-His (SEQ. ID. NR. 72) umfasst,
wobei es sich bei Xₐ um ein Phenylalanin oder Tyrosin, bei X_{b} um einen hydrophoben Rest und bei X_{c} um Serin oder Threonin handelt.

45. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 44 handelt.

46. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-His-X_{b}-X-Arg-His-X₃₋₅-His (SEQ. ID. NR. 73) umfasst,
wobei es sich bei Xₐ um ein Phenylalanin oder Tyrosin und bei X_{b} um einen hydrophoben Rest handelt.

47. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 46 handelt.

48. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-Asn-X_{b}-X-Val-His-X₃₋₅-His (SEQ. ID. NR. 74) umfasst,
wobei es sich bei Xₐ um ein Phenylalanin oder Tyrosin und bei X_{b} um einen hydrophoben Rest handelt.

49. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 48 handelt.

50. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-X_{c}-X_{b}-X-Arg-His-X₃₋₅-His (SEQ. ID. NR. 75) umfasst,
wobei es sich bei Xₐ um ein Phenylalanin oder Tyrosin, bei X_{b} um einen hydrophoben Rest und bei X_{c} um Serin oder Threonin handelt.

51. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 50 handelt.

52. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu SEQ. ID. NR. 65 ist.

53. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 52 handelt.

54. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu einer Aminosäuresequenz ist, die aus der aus SEQ. ID. NR. 29, 127, 129, 131, 133 und 135 bestehenden Gruppe ausgewählt ist.

55. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 54 handelt.

56. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ala-His-X_{b}-X-Arg-His-X₃₋₅-His (SEQ. ID. NR. 150) umfasst,
wobei es sich bei Xₐ um ein Phenylalanin oder Tyrosin und bei X_{b} um einen hydrophoben Rest handelt.

57. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 56 handelt.

58. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Phe-Asn-X_{b}-X-Arg-HiS-X₃₋₅-His (SEQ. ID. NR. 151) umfasst,
wobei es sich bei Xₐ um ein Phenylalanin oder Tyrosin und bei X_{b} um einen hydrophoben Rest handelt.

59. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 58 handelt.

60. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-His-X_{b}-X-Thr-His-X₃₋₅-His (SEQ. ID. NR. 152) umfasst,
wobei es sich bei Xₐ um ein Phenylalanin oder Tyrosin und bei X_{b} um einen hydrophoben Rest handelt.

61. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 60 handelt.

62. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-His-X_{b}-X-Val-His-X₃₋₅-His (SEQ. ID. NR. 153) umfasst,
wobei es sich bei Xₐ um ein Phenylalanin oder Tyrosin und bei X_{b} um einen hydrophoben Rest handelt.

63. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 62 handelt.

64. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-Asn-X_{b}-X-Ile-His-X₃₋₅-His (SEQ. ID. NR. 154) umfasst,
wobei es sich bei Xₐ um ein Phenylalanin oder Tyrosin und bei X_{b} um einen hydrophoben Rest handelt.

65. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 64 handelt.

66. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Ser-Asn-X_{b}-X-Arg-His-X₃₋₅-His (SEQ. ID. NR. 155) umfasst,
wobei es sich bei Xₐ um ein Phenylalanin oder Tyrosin und bei X_{b} um einen hydrophoben Rest handelt.

67. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 66 handelt.

68. Verfahren gemäß Anspruch 1, wobei die identifizierte Zinkfingerdomäne die Aminosäuresequenz:
Xₐ-X-Cys-X₂₋₅-Cys-X₃-Xₐ-X-Gln-X-Thr-His-X_{b}-X-Gln-His-X₃₋₅-His (SEQ. ID. NR. 156) umfasst,
wobei es sich bei Xₐ um ein Phenylalanin oder Tyrosin und bei X_{b} um einen hydrophoben Rest handelt.

69. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 68 handelt.

70. Verfahren gemäß Anspruch 1, wobei die identifizierte Zinkfingerdomäne die Aminosäuresequenz:
Cys-X₂₋₅-Cys-X₃-Xₐ-X-Gln-X-Thr-HiS-X_{b}-X-Arg-HiS-X₃₋₅-His (SEQ. ID. NR. 157) umfasst,
wobei es sich bei Xₐ um ein Phenylalanin oder Tyrosin und bei X_{b} um einen hydrophoben Rest handelt.

71. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 70 handelt.

72. Verfahren gemäß Anspruch 1, wobei die identifizierte Zinkfingerdomäne die Aminosäuresequenz:
Xₐ-X-Cys-X₂₋₅-Cys-X₃-Xₐ-X-Arg-X-Asp-Lys-X_{b}-X-Ile-His-X₃₋₅-His (SEQ. ID. NR. 158) umfasst,
wobei es sich bei Xₐ um ein Phenylalanin oder Tyrosin und bei X_{b} um einen hydrophoben Rest handelt.

73. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 72 handelt.

74. Verfahren gemäß Anspruch 1, wobei die identifizierte Zinkfingerdomäne die Aminosäuresequenz:
Xₐ-X-Cys-X₂₋₅-Cys-X₃-Xₐ-X-Arg-X-Ser-Asn-X_{b}-X-Arg-His-X₃₋₅-His (SEQ. ID. NR. 159) umfasst,
wobei es sich bei Xₐ um ein Phenylalanin oder Tyrosin und bei X_{b} um einen hydrophoben Rest handelt.

75. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 74 handelt.

76. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu SEQ. ID. NR. 137 ist.

77. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 76 handelt.

78. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu SEQ. ID. NR. 145 ist.

79. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 78 handelt.

80. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu SEQ. ID. NR. 149 ist.

81. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 80 handelt.

82. Verfahren gemäß Anspruch 1, wobei die identifizierte Zinkfingerdomäne eine Aminosäuresequenz umfasst, die zu 50 %, 60 %, 70 %, 80 %, 90 %, 93 %, 95 %, 96 %, 98 %, 99 % oder 100 % identisch zu SEQ. 1 D. NR. 141 ist.

83. Verfahren gemäß Anspruch 1, wobei es sich bei der Hybridnukleinsäure mit dem Code für die Testzinkfingerdomäne um eine Nukleinsäuresequenz mit dem Code für die Aminosäuresequenz gemäß Anspruch 82 handelt.

## Revendications

1. Procédé d'identification d'un domaine à doigt à zinc qui reconnaît un site cible sur un ADN, le procédé comprenant :
(a) la fourniture de cellules contenant un gène chimère rapporteur, le gène chimère comprenant un gène rapporteur lié de manière opérationnelle à un promoteur, dans lequel l'expression du gène rapporteur est activée au-dessus d'un niveau donné ou inhibée en dessous d'un niveau donné lorsqu'un facteur de transcription reconnaît à la fois un site de recrutement et un site cible du promoteur, mais pas lorsque le facteur de transcription reconnaît uniquement le site de recrutement du promoteur ;
(b) la fourniture d'une pluralité d'acides nucléiques hybrides dont chacun code pour une protéine se produisant de manière non naturelle comprenant (i) un domaine d'activation ou de répression de la transcription, (ii) un domaine de liaison à l'ADN qui reconnaît le site de recrutement, et (iii) un domaine à doigt à zinc test, dans lequel la séquence d'acides aminés codée du domaine à doigt à zinc test varie parmi les membres de la pluralité ;
(c) la mise en contact de la pluralité d'acides nucléiques hybrides avec les cellules dans des conditions qui permettent à au moins un de la pluralité d'acides nucléiques de pénétrer dans au moins une des cellules ;
(d) le maintien des cellules dans des conditions permettant l'expression des acides nucléiques hybrides dans les cellules ; et
(e) l'identification d'une cellule qui contient un acide nucléique hybride de (b) et qui active une expression du gène rapporteur au-dessus du niveau donné ou inhibe l'expression du gène rapporteur en dessous du niveau donné comme indication que la cellule contient un acide nucléique hybride codant pour un domaine à doigt à zinc test qui reconnaît le site cible.

2. Procédé selon la revendication 1, dans lequel les cellules sont des cellules eucaryotes.

3. Procédé selon la revendication 2, dans lequel les cellules sont des cellules de levure.

4. Procédé selon la revendication 3, dans lequel les cellules sont des cellules de Saccharomyces cerevisiae.

5. Procédé selon la revendication 1, dans lequel le gène rapporteur est un marqueur sélectionnable.

6. Procédé selon la revendication 5, dans lequel le marqueur sélectionnable est sélectionné parmi le groupe constitué de URA3, HIS3, LEU2, ADE2 et TRP1.

7. Procédé selon la revendication 1, dans lequel le gène rapporteur est sélectionné parmi le groupe constitué de lacZ, CAT, la luciférase, GUS et GFP.

8. Procédé selon la revendication 1, dans lequel le domaine de liaison à l'ADN comprend un domaine à doigt à zinc.

9. Procédé selon la revendication 1, dans lequel le domaine de liaison à l'ADN comprend deux domaines à doigt à zinc.

10. Procédé selon la revendication 1, dans lequel le domaine de liaison à l'ADN comprend trois domaines à doigt à zinc.

11. Procédé selon la revendication 1, comprenant en outre les étapes consistant à (i) amplifier un acide nucléique source codant pour le domaine à doigt à zinc test provenant d'acide nucléique génomique, d'un mélange d'ARN messager (ARNm) ou d'un mélange d'ADN complémentaire (ADNc), en utilisant une amorce oligonucléotidique qui annelle à une séquence codant pour une limite de domaine conservée pour produire un fragment amplifié ; et (ii) utiliser le fragment amplifié pour construire un acide nucléique hybride pour l'inclusion dans la pluralité d'acides nucléiques hybrides de l'étape (b).

12. Procédé selon la revendication 1, comprenant en outre l'étape consistant à (i) identifier une séquence d'acides aminés de domaine à doigt à zinc candidate dans une base de données de séquences ; (ii) fournir un acide nucléique candidat codant pour la séquence d'acides aminés de domaine à doigt à zinc candidate, et (iii) utiliser l'acide nucléique candidat pour construire un acide nucléique hybride pour l'inclusion dans la pluralité d'acides nucléiques hybrides de l'étape (b).

13. Procédé selon la revendication 5, dans lequel le marqueur sélectionnable est un gène auxotrophe nécessaire pour la synthèse d'un métabolite ; le génome des cellules est dépourvu d'une copie fonctionnelle du gène auxotrophe ; et, au cours de l'étape (d), les cellules sont maintenues dans un milieu préparé sans le métabolite.

14. Procédé selon la revendication 1, dans lequel les étapes (a) à (e) sont répétées pour identifier un second domaine à doigt à zinc test qui reconnaît un second site cible.

15. Procédé selon la revendication 14, comprenant en outre la construction d'un acide nucléique codant pour un polypeptide comprenant le premier domaine à doigt à zinc test et le second domaine à doigt à zinc test.

16. Procédé d'identification d'un domaine à doigt à zinc qui reconnaît un site cible sur un ADN, le procédé comprenant :
(a) la fourniture de cellules contenant un gène chimère rapporteur, le gène chimère comprenant un gène rapporteur lié de manière opérationnelle à un promoteur, dans lequel l'expression du gène rapporteur est activée au-dessus d'un niveau donné ou inhibée en dessous d'un niveau donné lorsqu'un facteur de transcription reconnaît à la fois un site de recrutement et un site cible du promoteur, mais pas lorsque le facteur de transcription reconnaît uniquement le site de recrutement du promoteur ;
(b) l'amplification d'une pluralité de séquences d'acides nucléiques dont chacune code pour un domaine à doigt à zinc test, en utilisant une amorce oligonucléotidique qui annelle à un acide nucléique codant pour une limite de domaine conservée ;
(c) la jonction de chaque séquence d'acide nucléique de (b) à des séquences d'acides nucléiques codant (i) pour un domaine d'activation ou de répression de la transcription, et (ii) pour un domaine de liaison à l'ADN qui reconnaît le site de recrutement, afin de former une pluralité d'acides nucléiques hybrides ;
(d) la mise en contact de la pluralité d'acides nucléiques hybrides de (c) avec les cellules de (a) dans des conditions qui permettent à au moins un de la pluralité d'acides nucléiques hybrides de pénétrer dans au moins une des cellules ;
(e) le maintien des cellules dans des conditions permettant l'expression des acides nucléiques hybrides dans les cellules ; et
(f) l'identification d'une cellule qui contient un acide nucléique hybride de (c) et qui active une expression du gène rapporteur au-dessus du niveau donné ou inhibe l'expression du gène rapporteur en dessous du niveau donné, dans lequel l'acide nucléique hybride code pour un domaine à doigt à zinc qui reconnaît le site cible sur un ADN.

17. Procédé selon la revendication 16, dans lequel les cellules sont des cellules de levure.

18. Procédé selon la revendication 16, dans lequel le gène rapporteur est sélectionné parmi le groupe constitué de lacZ, CAT, la luciférase, GUS et GFP.

19. Procédé selon la revendication 16, dans lequel le domaine de liaison à l'ADN comprend un domaine à doigt à zinc.

20. Procédé selon la revendication 16, dans lequel le domaine de liaison à l'ADN comprend deux domaines à doigt à zinc.

21. Procédé de détermination du fait qu'un domaine à doigt à zinc test reconnaît un site cible sur un promoteur, le procédé comprenant :
(a) la fourniture d'un gène chimère rapporteur comprenant un gène rapporteur lié de manière opérationnelle à un promoteur, dans lequel l'expression du gène rapporteur est activée au-dessus d'un niveau donné ou inhibée en dessous d'un niveau donné lorsqu'un facteur de transcription reconnaît à la fois un site de recrutement et un site cible du promoteur, mais pas lorsque le facteur de transcription reconnaît uniquement le site de recrutement du promoteur ;
(b) la fourniture d'un acide nucléique hybride qui code pour une protéine se produisant de manière non naturelle comprenant (i) un domaine d'activation ou de répression de la transcription, (ii) un domaine de liaison à l'ADN qui reconnaît le site de recrutement, et (iii) un domaine à doigt à zinc test ;
(c) la mise en contact du gène chimère rapporteur avec une cellule dans des conditions qui permettent au gène chimère rapporteur de pénétrer dans la cellule ;
(d) avant, après ou en même temps que l'étape (c), la mise en contact de l'acide nucléique hybride avec la cellule dans des conditions qui permettent à l'acide nucléique hybride de pénétrer dans la cellule ;
(e) le maintien de la cellule dans des conditions permettant l'expression de l'acide nucléique hybride dans la cellule ; et
(f) la détection des différences dans l'expression du gène rapporteur dans la cellule, dans lequel une différence de niveau de l'expression du gène rapporteur supérieure au niveau donné ou inférieure au niveau donné est une indication que le domaine à doigt à zinc test reconnaît le site cible.

22. Procédé selon la revendication 21, comprenant en outre l'étape consistant à amplifier un acide nucléique codant pour le domaine à doigt à zinc test provenant d'ADN génomique, d'un mélange d'ARNm ou d'un mélange d'ADNc, en utilisant une amorce oligonucléotidique qui annelle à une séquence codant pour une limite de domaine conservée.

23. Procédé selon la revendication 21, comprenant en outre les étapes consistant à (i) identifier une séquence d'acides aminés de domaine à doigt à zinc candidate dans une base de données de séquences ; (ii) fournir un acide nucléique candidat codant pour la séquence d'acides aminés de domaine à doigt à zinc candidate, et (iii) utiliser l'acide nucléique candidat pour construire un acide nucléique hybride pour l'inclusion dans la pluralité d'acides nucléiques hybrides de l'étape (b).

24. Procédé de détermination du fait qu'un domaine à doigt à zinc test reconnaît un site cible sur un promoteur, le procédé comprenant :
(a) la fourniture d'une première cellule comprenant un gène chimère rapporteur comprenant un gène rapporteur lié de manière opérationnelle à un promoteur, dans lequel l'expression du gène rapporteur est activée au-dessus d'un niveau donné ou inhibée en dessous d'un niveau donné lorsqu'un facteur de transcription reconnaît à la fois un site de recrutement et un site cible du promoteur, mais pas lorsque le facteur de transcription reconnaît uniquement le site de recrutement du promoteur ;
(b) la fourniture d'une seconde cellule comprenant un acide nucléique hybride qui code pour une protéine comprenant (i) un domaine d'activation ou de répression de la transcription, (ii) un domaine de liaison à l'ADN qui reconnaît le site de liaison de recrutement, et (iii) un domaine à doigt à zinc test ;
(c) la fusion des première et seconde cellules pour former une cellule fusée ;
(d) le maintien de la cellule fusée dans des conditions permettant l'expression des acides nucléiques hybrides dans la cellule ; et
(e) la détection des différences dans l'expression du gène rapporteur dans la cellule fusée, dans lequel une différence de niveau de l'expression du gène rapporteur supérieure au niveau donné ou inférieure au niveau donné est une indication que le domaine à doigt à zinc test reconnaît le site cible.

25. Procédé selon la revendication 24, dans lequel les première et seconde cellules sont des cellules de levure des types de conjugaison opposés.

26. Procédé de détermination du fait qu'un domaine à doigt à zinc test reconnaît un site cible sur un promoteur, le procédé comprenant :
(a) la fourniture d'une pluralité de gènes chimères rapporteurs, chaque gène chimère comprenant un gène rapporteur lié de manière opérationnelle à un promoteur, dans lequel l'expression du gène rapporteur est activée au-dessus d'un niveau donné ou inhibée en dessous d'un niveau donné lorsqu'un facteur de transcription reconnaît à la fois un site de recrutement et un site cible du promoteur, mais pas lorsque le facteur de transcription reconnaît uniquement le site de recrutement du promoteur ;
(b) la fourniture d'une cellule contenant un acide nucléique hybride qui code pour une protéine se produisant de manière non naturelle comprenant (i) un domaine d'activation ou de répression de la transcription, (ii) un domaine de liaison à l'ADN qui reconnaît le site de recrutement, et (iii) un domaine à doigt à zinc test ;
(c) la mise en contact de la pluralité de gènes chimères rapporteurs avec la cellule dans des conditions qui permettent à au moins un de la pluralité de gènes chimères rapporteurs de pénétrer dans la cellule ;
(d) le maintien de la cellule dans des conditions permettant l'expression de l'acide nucléique hybride dans la cellule ; et
(e) l'identification d'une cellule qui contient un gène rapporteur de (a) et qui active l'expression du gène rapporteur au-dessus du niveau donné ou inhibe l'expression du gène rapporteur en dessous du niveau donné comme indication que le gène chimère rapporteur dans la cellule comprend un site cible reconnu par le domaine à doigt à zinc test.

27. Procédé selon la revendication 26, dans lequel le site de liaison cible est entre deux et six nucléotides de long.

28. Procédé selon la revendication 27, dans lequel la pluralité de gènes chimères rapporteurs comprend toute combinaison possible des nucléotides A, T, G et C en au moins deux positions du site de liaison cible.

29. Procédé selon la revendication 28, dans lequel la pluralité de gènes chimères rapporteurs comprend toute combinaison possible des nucléotides A, T, G et C en au moins trois positions des sites de liaison cibles.

30. Procédé selon la revendication 26, dans lequel les étapes (a) à (e) sont répétées pour l'identification par un second domaine à doigt à zinc test d'une seconde préférence de liaison.

31. Procédé selon la revendication 30, comprenant en outre la construction d'un acide nucléique codant pour un polypeptide comprenant les premier et second domaines à doigt à zinc tests.

32. Procédé d'identification d'une pluralité de domaines à doigt à zinc, le procédé comprenant :
la réalisation du procédé de la revendication 1 pour identifier un premier domaine à doigt à zinc test ; et
la réalisation du procédé de la revendication 1 de nouveau pour identifier un second domaine à doigt à zinc test qui reconnaît un site cible différent du site cible reconnu par le premier domaine à doigt à zinc test.

33. Procédé de génération d'un acide nucléique codant pour une protéine à doigt à zinc chimère, le procédé comprenant :
la réalisation du procédé de la revendication 32 ;
la construction d'un acide nucléique codant pour un polypeptide comprenant les premier et second domaines à doigt à zinc tests.

34. Procédé d'identification de séquences d'ADN reconnues par des domaines à doigt à zinc, le procédé comprenant :
la réalisation du procédé de la revendication 24 pour identifier un premier site cible reconnu par un premier domaine à doigt à zinc test ; et
la réalisation du procédé de la revendication 24 de nouveau pour identifier un second site cible reconnu par un second domaine à doigt à zinc test.

35. Procédé de génération d'un acide nucléique codant pour une protéine à doigt à zinc chimère, le procédé comprenant :
la réalisation du procédé de la revendication 34 ;
la construction d'un acide nucléique codant pour un polypeptide comprenant les premier et second domaines à doigt à zinc tests.

36. Procédé selon la revendication 1, dans lequel le domaine à doigt à zinc identifié comprend la séquence d'acides aminés :
Xₐ-X-Cys-X₂₋₅-Cys-X₃-Xₐ-X-Cys-X-Ser-Asn-X_{b}-X-Arg-His-X₃₋₅-His (SEQ ID N° : 68),
dans laquelle Xₐ est phénylalanine ou tyrosine et X_{b} est un résidu hydrophobe.

37. Procédé selon la revendication 1, dans lequel l'acide nucléique hybride codant pour le domaine à doigt à zinc test est une séquence d'acide nucléique codant pour la séquence d'acides aminés de la revendication 36.

38. Procédé selon la revendication 1, dans lequel le domaine à doigt à zinc identifié comprend la séquence d'acides aminés :
Xₐ-X-Cys-X₂₋₅-Cys-X₃-Xₐ-X-His-X-Ser-Asn-X_{b}-X-Lys-His-X₃₋₅-His (SEQ ID N° : 69),
dans laquelle Xₐ est phénylalanine ou tyrosine et X_{b} est un résidu hydrophobe.

39. Procédé selon la revendication 1, dans lequel l'acide nucléique hybride codant pour le domaine à doigt à zinc test est une séquence d'acide nucléique codant pour la séquence d'acides aminés de la revendication 38.

40. Procédé selon la revendication 1, dans lequel le domaine à doigt à zinc identifié comprend la séquence d'acides aminés :
Xₐ-X-Cys-X₂₋₅-Cys-X₃-Xₐ-X-Ser-X-Ser-Asn-X_{b}-X-Arg-His-X₃₋₅-His (SEQ ID N° : 70),
dans laquelle Xₐ est phénylalanine ou tyrosine et X_{b} est un résidu hydrophobe.

41. Procédé selon la revendication 1, dans lequel l'acide nucléique hybride codant pour le domaine à doigt à zinc test est une séquence d'acide nucléique codant pour la séquence d'acides aminés de la revendication 40.

42. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-Thr-X_{b}-X-Val-His-X₃₋₅-His (SEQ ID N° : 71),
dans laquelle Xₐ est phénylalanine ou tyrosine et X_{b} est un résidu hydrophobe.

43. Procédé selon la revendication 1, dans lequel l'acide nucléique hybride codant pour le domaine à doigt à zinc test est une séquence d'acide nucléique codant pour la séquence d'acides aminés de la revendication 42.

44. Procédé selon la revendication 1, dans lequel le domaine à doigt à zinc identifié comprend la séquence d'acides aminés :
Xₐ-X-Cys-X₂₋₅-Cys-X₃-Xₐ-X-Val-X-Ser- X_{c}-X_{b}-X-Arg-His-X₃₋₅-His (SEQ ID N° : 72),
dans laquelle Xₐ est phénylalanine ou tyrosine, X_{b} est un résidu hydrophobe et X_{c} est sérine ou thréonine.

45. Procédé selon la revendication 1, dans lequel l'acide nucléique hybride codant pour le domaine à doigt à zinc test est une séquence d'acide nucléique codant pour la séquence d'acides aminés de la revendication 44.

46. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-His-X_{b}-X-Arg-His-X₃₋₅-His (SEQ ID N° : 73),
dans laquelle Xₐ est phénylalanine ou tyrosine et X_{b} est un résidu hydrophobe.

47. Procédé selon la revendication 1, dans lequel l'acide nucléique hybride codant pour le domaine à doigt à zinc test est une séquence d'acide nucléique codant pour la séquence d'acides aminés de la revendication 46.

48. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-Asn-X_{b}-X-Val-His-X₃₋₅-His (SEQ ID N° : 74),
dans laquelle Xₐ est phénylalanine ou tyrosine et X_{b} est un résidu hydrophobe.

49. Procédé selon la revendication 1, dans lequel l'acide nucléique hybride codant pour le domaine à doigt à zinc test est une séquence d'acide nucléique codant pour la séquence d'acides aminés de la revendication 48.

50. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-X_{c}-X_{b}-X-Arg-His-X₃₋₅-His (SEQ ID N° : 75),
dans laquelle Xₐ est phénylalanine ou tyrosine et X_{b} est un résidu hydrophobe et X_{c} est sérine ou thréonine.

51. Procédé selon la revendication 1, dans lequel l'acide nucléique hybride codant pour le domaine à doigt à zinc test est une séquence d'acide nucléique codant pour la séquence d'acides aminés de la revendication 50.

52. Procédé selon la revendication 1, dans lequel le domaine à doigt à zinc identifié comprend une séquence d'acides aminés 50 %, 60 %, 70 %, 80 %, 90 %, 93 %, 95 %, 96 %, 98 %, 99 % ou 100 % identique à SEQ ID N° : 65.

53. Procédé selon la revendication 1, dans lequel l'acide nucléique hybride codant pour le domaine à doigt à zinc test est une séquence d'acide nucléique codant pour la séquence d'acides aminés de la revendication 52.

54. Procédé selon la revendication 1, dans lequel le domaine à doigt à zinc identifié comprend une séquence d'acides aminés 50 %, 60 %, 70 %, 80 %, 90 %, 93 %, 95 %, 96 %, 98 %, 99 % ou 100 % identique à une séquence d'acides aminés sélectionnée parmi le groupe constitué de : SEQ ID N° : 29, 127, 129, 131, 133 et 135.

55. Procédé selon la revendication 1, dans lequel l'acide nucléique hybride codant pour le domaine à doigt à zinc test est une séquence d'acide nucléique codant pour la séquence d'acides aminés de la revendication 54.

56. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ala-His-X_{b}-X-Arg-His-X₃₋₅-His (SEQ ID N° : 150),
dans laquelle Xₐ est phénylalanine ou tyrosine et X_{b} est un résidu hydrophobe.

57. Procédé selon la revendication 1, dans lequel l'acide nucléique hybride codant pour le domaine à doigt à zinc test est une séquence d'acide nucléique codant pour la séquence d'acides aminés de la revendication 56.

58. Procédé selon la revendication 1, dans lequel le domaine à doigt à zinc identifié comprend la séquence d'acides aminés :
Xₐ-X-C_{YS}-X₂-₅-C_{YS}-X₃-Xₐ-X-Gln-X-Phe-Asn-X_{b}-X-Arg-HiS-X₃-₅-HiS (SEQ ID N° : 151),
dans laquelle Xₐ est phénylalanine ou tyrosine et X_{b} est un résidu hydrophobe.

59. Procédé selon la revendication 1, dans lequel l'acide nucléique hybride codant pour le domaine à doigt à zinc test est une séquence d'acide nucléique codant pour la séquence d'acides aminés de la revendication 58.

60. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-His-X_{b}-X-Thr-His-X₃₋₅-His (SEQ ID N° : 152),
dans laquelle Xₐ est phénylalanine ou tyrosine et X_{b} est un résidu hydrophobe.

61. Procédé selon la revendication 1, dans lequel l'acide nucléique hybride codant pour le domaine à doigt à zinc test est une séquence d'acide nucléique codant pour la séquence d'acides aminés de la revendication 60.

62. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-His-X_{b}-X-Val-His-X₃₋₅-His (SEQ ID N° : 153),
dans laquelle Xₐ est phénylalanine ou tyrosine et X_{b} est un résidu hydrophobe.

63. Procédé selon la revendication 1, dans lequel l'acide nucléique hybride codant pour le domaine à doigt à zinc test est une séquence d'acide nucléique codant pour la séquence d'acides aminés de la revendication 62.

64. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-Asn-X_{b}-X-lle-His-X₃₋₅-His (SEQ ID N° : 154),
dans laquelle Xₐ est phénylalanine ou tyrosine et X_{b} est un résidu hydrophobe.

65. Procédé selon la revendication 1, dans lequel l'acide nucléique hybride codant pour le domaine à doigt à zinc test est une séquence d'acide nucléique codant pour la séquence d'acides aminés de la revendication 64.

66. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Ser-Asn-X_{b}-X-Arg-His-X₃₋₅-His (SEQ ID N° : 155),
dans laquelle Xₐ est phénylalanine ou tyrosine et X_{b} est un résidu hydrophobe.

67. Procédé selon la revendication 1, dans lequel l'acide nucléique hybride codant pour le domaine à doigt à zinc test est une séquence d'acide nucléique codant pour la séquence d'acides aminés de la revendication 66.

68. Procédé selon la revendication 1, dans lequel le domaine à doigt à zinc identifié comprend la séquence d'acides aminés :
Xₐ-X-Cys-X₂₋₅-Cys-X₃-Xₐ-X-Gln-X-Thr-His-X_{b}-X-Gln-His-X₃₋₅-His (SEQ ID N° : 156),
dans laquelle Xₐ est phénylalanine ou tyrosine et X_{b} est un résidu hydrophobe.

69. Procédé selon la revendication 1, dans lequel l'acide nucléique hybride codant pour le domaine à doigt à zinc test est une séquence d'acide nucléique codant pour la séquence d'acides aminés de la revendication 68.

70. Procédé selon la revendication 1, dans lequel le domaine à doigt à zinc identifié comprend la séquence d'acides aminés :
Cys-X₂₋₅-Cys-X₃-Xₐ-X-Gln-X-Thr-His-X_{b}-X-Arg-His-X₃₋₅-His (SEQ ID N° : 157),
dans laquelle Xₐ est phénylalanine ou tyrosine et X_{b} est un résidu hydrophobe.

71. Procédé selon la revendication 1, dans lequel l'acide nucléique hybride codant pour le domaine à doigt à zinc test est une séquence d'acide nucléique codant pour la séquence d'acides aminés de la revendication 70.

72. Procédé selon la revendication 1, dans lequel le domaine à doigt à zinc identifié comprend la séquence d'acides aminés :
Xₐ-X-Cys-X₂₋₅-Cys-X₃-Xₐ-X-Arg-X-Asp-Lys-X_{b}-X-Ile-His-X₃₋₅-His (SEQ ID N° : 158),
dans laquelle Xₐ est phénylalanine ou tyrosine et X_{b} est un résidu hydrophobe.

73. Procédé selon la revendication 1, dans lequel l'acide nucléique hybride codant pour le domaine à doigt à zinc test est une séquence d'acide nucléique codant pour la séquence d'acides aminés de la revendication 72.

74. Procédé selon la revendication 1, dans lequel le domaine à doigt à zinc identifié comprend la séquence d'acides aminés :
Xₐ-X-Cys-X₂₋₅-Cys-X₃-Xₐ-X-Arg-X-Ser-Asn-X_{b}-X-Arg-His-X₃₋₅-His (SEQ ID N° : 159),
dans laquelle Xₐ est phénylalanine ou tyrosine et X_{b} est un résidu hydrophobe.

75. Procédé selon la revendication 1, dans lequel l'acide nucléique hybride codant pour le domaine à doigt à zinc test est une séquence d'acide nucléique codant pour la séquence d'acides aminés de la revendication 74.

76. Procédé selon la revendication 1, dans lequel le domaine à doigt à zinc identifié comprend une séquence d'acides aminés 50 %, 60 %, 70 %, 80 %, 90 %, 93 %, 95 %, 96 %, 98 %, 99 % ou 100 % identique à SEQ ID N° : 137.

77. Procédé selon la revendication 1, dans lequel l'acide nucléique hybride codant pour le domaine à doigt à zinc test est une séquence d'acide nucléique codant pour la séquence d'acides aminés de la revendication 76.

78. Procédé selon la revendication 1, dans lequel le domaine à doigt à zinc identifié comprend une séquence d'acides aminés 50 %, 60 %, 70 %, 80 %, 90 %, 93 %, 95 %, 96 %, 98 %, 99 % ou 100 % identique à SEQ ID N° : 145.

79. Procédé selon la revendication 1, dans lequel l'acide nucléique hybride codant pour le domaine à doigt à zinc test est une séquence d'acide nucléique codant pour la séquence d'acides aminés de la revendication 78.

80. Procédé selon la revendication 1, dans lequel le domaine à doigt à zinc identifié comprend une séquence d'acides aminés 50 %, 60 %, 70 %, 80 %, 90 %, 93 %, 95 %, 96 %, 98%, 99% ou 100 % identique à SEQ ID N°: 149.

81. Procédé selon la revendication 1, dans lequel l'acide nucléique hybride codant pour le domaine à doigt à zinc test est une séquence d'acide nucléique codant pour la séquence d'acides aminés de la revendication 80.

82. Procédé selon la revendication 1, dans lequel le domaine à doigt à zinc identifié comprend une séquence d'acides aminés 50 %, 60 %, 70 %, 80 %, 90%, 93%, 95 %, 96%, 98%, 99% ou 100 % identique à SEQ ID N° : 141.

83. Procédé selon la revendication 1, dans lequel l'acide nucléique hybride codant pour le domaine à doigt à zinc test est une séquence d'acide nucléique codant pour la séquence d'acides aminés de la revendication 82.


### SEQUENCE LISTING

<110> Kim, Jin-Soo
   Kwon, Young-Do
   Kim, Hyun-Won
   Ryu, Eun Hyun
   Hwang, Moon Sun
<120> SELECTION OF TARGET-SPECIFIC ZINC FINGER DOMAINS
<130> 12279-002001
<160> 167
<170> Fast SEQ for Windows Version 4.0
<210> 1
   <211> 10
   <212> DNA
   <213> HIV-1
<400> 1
   gacatcgagc
   10
<210> 2
   <211> 10
   <212> DNA
   <213> HIV-1
<400> 2 gctgagacat 10
<210> 6
   <211> 47
   <212> DNA
   <213> Artificial Sequence
<220>
   <223> optimal binding site
<400> 6
   ccggcgtggg cggctgcgtg ggcgtgcgtg ggcggactgc gtgggcg
   47
<210> 7
   <211> 47
   <212> DNA
   <213> Artificial Sequence
<220>
   <223> optimal binding site
<400> 7
   tcgacgccca cgcagtccgc ccacgcacgc ccacgcagcc gcccacg
   47
<210> 8
   <211> 49
   <212> DNA
   <213> HIV-1
<400> 8
   ccggcgagcg ggcggtcgag cgggcgtgag cgggcggatc gagcgggcg
   49
<210> 9
   <211> 49
   <212> DNA
   <213> HIV-1
<400> 9
   tcgacgcccg ctcgatccgc ccgctcacgc ccgctcgacc gcccgctcg
   49
<210> 10
   <211> 50
   <212> DNA
   <213> HIV-1
<400> 10
   ccggctgctt gggcggctgc ttgggcgtgc ttgggcgggc tgcttgggcg
   50
<210> 11
   <211> 50
   <212> DNA
   <213> HIV-1
<400> 11
   tcgacgccca agcagcccgc ccaagcacgc ccaagcagcc gcccaagcag
   50
<210> 12
   <211> 47
   <212> DNA
   <213> HIV-1
<400> 12
   ccggactggg cgggggactg ggcgtgactg ggcggaggga ctgggcg
   47
<210> 13
   <211> 47
   <212> DNA
   <213> HIV-1
<400> 13
   tcgacgccca gtccctccgc ccagtcacgc ccagtccccc gcccagt
   47
<210> 14
   <211> 47
   <212> DNA
   <213> Homo sapiens
<400> 14
   ccggagtggg cggtggagtg ggcgtgagtg ggcggatgga gtgggcg
   47
<210> 15
   <211> 47
   <212> DNA
   <213> Homo sapiens
<400> 15
   tcgacgccca ctccatccgc ccactcacgc ccactccacc gcccact
   47
<210> 16
   <211> 48
   <212> DNA
   <213> Homo sapiens
<400> 16
   ccggacatgg gcggagacat gggcgtacat gggcggaaga catgggcg
   48
<210> 17
   <211> 48
   <212> DNA
   <213> Homo sapiens
<400> 17
   tcgacgccca tgtcttccgc ccatgtacgc ccatgtctcc gcccatgt
   48
<210> 18
   <211> 120
   <212> DNA
   <213> Artificial Sequence
<220>
   <223> plasmid sequence
<221> CDS
   <222> (1)...(81)
<400> 18
   aaa gag ggt ggg tcg acc ttc cgg act ggc cag gaa cgc cca gat ccg
   48
   Lys Glu Gly Gly Ser Thr Phe Arg Thr Gly Gln Glu Arg Pro Asp Pro
    1               5                  10                  15
   cgg gaa ttc aga tct act agt gcg gcc gct aag taagtaagac gtcgagctcg
   101
   Arg Glu Phe Arg Ser Thr Ser Ala Ala Ala Lys
               20                  25
   ccatcgcggt ggaagcttt
   120
<210> 19
   <211> 27
   <212> PRT
   <213> Artificial Sequence
<220>
   <223> plasmid sequence
<400> 19
   Lys Glu Gly Gly Ser Thr Phe Arg Thr Gly Gln Glu Arg Pro Asp Pro
    1               5                  10                  15
   Arg Glu Phe Arg Ser Thr Ser Ala Ala Ala Lys
               20                  25
<210> 20
   <211> 303
   <212> DNA
   <213> Artificial Sequence
<220>
   <223> plasmid sequence
<221> CDS
   <222> (25)...(291)
<400> 20
   gggtcgacct tccggactgg ccag gaa cgc cca tat gct tgc cct gtc gag
   51
                             Glu Arg Pro Tyr Ala Cys Pro Val Glu
                              1               5
   tcc tgc gat cgc cgc ttt tct cgc tcg gat gag ctt acc cgc cat atc
   99
   Ser Cys Asp Arg Arg Phe Ser Arg Ser Asp Glu Leu Thr Arg His Ile
    10                  15                  20                  25
   cgc atc cac act ggc cag aag ccc ttc cag tgt cga atc tgc atg cgt
   147
   Arg Ile His Thr Gly Gln Lys Pro Phe Gln Cys Arg Ile Cys Met Arg
                    30                  35                  40
   aac ttc agt cgt agt gac cac ctt acc acc cac atc cgg acc cac acc
   195
   Asn Phe Ser Arg Ser Asp His Leu Thr Thr His Ile Arg Thr His Thr
                45                  50                  55
   ggc gag aag cct ttt gcc tgt gac att tgt ggg agg aag ttt gcc agg
   243
   Gly Glu Lys Pro Phe Ala Cys Asp Ile Cys Gly Arg Lys Phe Ala Arg
            60                  65                  70
   agt gat gaa cgc aag agg cat acc aaa atc cat tta aga cag aag gat
   291
   Ser Asp Glu Arg Lys Arg His Thr Lys Ile His Leu Arg Gln Lys Asp
        75                  80                  85
   ccgcgggaat cc
   303
<210> 21
   <211> 89
   <212> PRT
   <213> Artificial Sequence
<220>
   <223> plasmid sequence
<400> 21
   Glu Arg Pro Tyr Ala Cys Pro Val Glu Ser Cys Asp Arg Arg Phe Ser
    1              5                  10                  15
   Arg Ser Asp Glu Leu Thr Arg His Ile Arg Ile His Thr Gly Gln Lys
               20                  25                  30
   Pro Phe Gln Cys Arg Ile Cys Met Arg Asn Phe Ser Arg Ser Asp His
           35                  40                  45
   Leu Thr Thr His Ile Arg Thr His Thr Gly Glu Lys Pro Phe Ala Cys
       50                  55                  60
   Asp Ile Cys Gly Arg Lys Phe Ala Arg Ser Asp Glu Arg Lys Arg His
   65                  70                  75                  80
   Thr Lys Ile His Leu Arg Gln Lys Asp
                   85
<210> 22
   <211> 102
   <212> DNA
   <213> Homo sapiens
<220>
   <221> CDS
   <222> (1)...(102)
<400> 22
   acc ggg cag aaa ccg tac aaa tgt aag caa tgt ggg aaa gct ttt gga
   48
   Thr Gly Gln Lys Pro Tyr Lys Cys Lys Gln Cys Gly Lys Ala Phe Gly
    1              5                  10                  15
   tgt ccc tca aac ctt cga agg cat gga agg act cac acc ggc gag aaa
   96
   Cys Pro Ser Asn Leu Arg Arg His   Gly Arg Thr His Thr Gly Glu Lys
               20                    25                  30
   ccg cgg
   102
   Pro Arg
<210> 23
   <211> 34
   <212> PRT
   <213> Homo sapiens
<400> 23
   Thr Gly Gln Lys Pro Tyr Lys Cys Lys Gln Cys Gly Lys Ala Phe Gly
    1              5                  10                  15
   Cys Pro Ser Asn Leu Arg Arg His Gly Arg Thr His Thr Gly Glu Lys
               20                  25                  30
   Pro Arg
<210> 24
   <211> 102
   <212> DNA
   <213> Homo sapiens
<220>
   <221> CDS
   <222> (1)...(102)
<400> 24
   acc ggg gag aag cca tac aag tgt aag gag tgt ggg aaa gcc ttc aac
   48
   Thr Gly Glu Lys Pro Tyr Lys Cys Lys Glu Cys Gly Lys Ala Phe Asn
    1               5                  10                  15
   cac agc tcc aac ttc aat aaa cac cac aga atc cac acc ggc gaa aag
   96
   His Ser Ser Asn Phe Asn Lys His His Arg Ile His Thr Gly Glu Lys
               20                  25                  30
   ccg cgg
   102
   Pro Arg
<210> 25
   <211> 34
   <212> PRT
   <213> Homo sapiens
<400> 25
   Thr Gly Glu Lys Pro Tyr Lys Cys Lys Glu Cys Gly Lys Ala Phe Asn
    1               5                  10                  15
   His Ser Ser Asn Phe Asn Lys His His Arg Ile His Thr Gly Glu Lys
               20                  25                  30
   Pro Arg
<210> 26
   <211> 102
   <212> DNA
   <213> Homo sapiens
<220>
   <221> CDS
   <222> (1)...(102)
<400> 26
<210> 27
   <211> 34
   <212> PRT
   <213> Homo sapiens
<400> 27
   Thr Gly Glu Arg Pro Phe Glu Cys Lys Glu Cys Gly Lys Ala Phe Ser
    1               5                  10                  15
   Ser Gly Ser Asn Phe Thr Arg His Gln Arg Ile His Thr Gly Glu Lys
               20                  25                  30
   Pro Arg
<210> 28
   <211> 108
   <212> DNA
   <213> Homo sapiens
<220>
   <221> CDS
   <222> (1)...(108)
<400> 28
   acc ggg cag aag cca tac gta tgc gat gta gag gga tgt acg tgg aaa
   48
   Thr Gly Gln Lys Pro Tyr Val Cys Asp Val Glu Gly Cys Thr Trp Lys
    1               5                  10                  15
   ttt gcc cgc tca gat gag ctc aac aga cac aag aaa agg cac acc ggc
   96
   Phe Ala Arg Ser Asp Glu Leu Asn Arg His Lys Lys Arg His Thr Gly
               20                  25                  30
   gaa aga ccg cgg                              108
   Glu Arg Pro Arg
           35
<210> 29
   <211> 36
   <212> PRT
   <213> Homo sapiens
<400> 29
   Thr Gly Gln Lys Pro Tyr Val Cys Asp Val Glu Gly Cys Thr Trp Lys
    1               5                  10                  15
   Phe Ala Arg Ser Asp Glu Leu Asn Arg His Lys Lys Arg His Thr Gly
               20                  25                  30
   Glu Arg Pro Arg
           35
<210> 30
   <211> 102
   <212> DNA
   <213> Homo sapiens
<220>
   <221> CDS
   <222> (1)...(102)
<400> 30
   acc ggg gag aga cct tac gag tgt aat gaa tgc ggg aaa gct ttt gcc
   48
   Thr Gly Glu Arg Pro Tyr Glu Cys Asn Glu Cys Gly Lys Ala Phe Ala
    1               5                  10                  15
   caa aat tca act ctc aga gta cac cag aga att cac acc ggc gaa aag
   96
   Gln Asn Ser Thr Leu Arg Val His Gln Arg Ile His Thr Gly Glu Lys
               20                  25                  30
   ccg cgg
   102
   Pro Arg
<210> 31
   <211> 34
   <212> PRT
   <213> Homo sapiens
<400> 31
   Thr Gly Glu Arg Pro Tyr Glu Cys Asn Glu Cys Gly Lys Ala Phe Ala
    1               5                  10                  15
   Gln Asn Ser Thr Leu Arg Val His Gln Arg Ile His Thr Gly Glu Lys
               20                  25                  30
   Pro Arg
<210> 32
   <211> 102
   <212> DNA
   <213> Homo sapiens
<220>
   <221> CDS
   <222> (1)...(102)
<400> 32
   acc ggg gag agg cct tat gag tgt aat tac tgt gga aaa acc ttt agt
   48
   Thr Gly Glu Arg Pro Tyr Glu Cys Asn Tyr Cys Gly Lys Thr Phe Ser
    1               5                  10                  15
   gtg agc tca acc ctt att aga cat cag aga atc cac acc ggc gag aga
   96
   Val Ser Ser Thr Leu Ile Arg His Gln Arg Ile His Thr Gly Glu Arg
               20                  25                  30
   ccg cgg
   102
   Pro Arg
<210> 33
   <211> 34
   <212> PRT
   <213> Homo sapiens
<400> 33
   Thr Gly Glu Arg Pro Tyr Glu Cys Asn Tyr Cys Gly Lys Thr Phe Ser
    1               5                  10                  15
   Val Ser Ser Thr Leu Ile Arg His Gln Arg Ile His Thr Gly Glu Arg
               20                  25                  30
   Pro Arg
<210> 34
   <211> 69
   <212> DNA
   <213> Homo sapiens
<220>
   <221> CDS
   <222> (1)...(69)
<400> 34
   tat cag tgc aac att tgc gga aaa tgt ttc tcc tgc aac tcc aac ctc
   48
   Tyr Gln Cys Asn Ile Cys Gly Lys Cys Phe Ser Cys Asn Ser Asn Leu
    1               5                  10                  15
   cac agg cac cag aga acg cac
   69
   His Arg His Gln Arg Thr His
               20
<210> 35
   <211> 23
   <212> PRT
   <213> Homo sapiens
<400> 35
   Tyr Gln Cys Asn Ile Cys Gly Lys Cys Phe Ser Cys Asn Ser Asn Leu
    1               5                  10                  15
   His Arg His Gln Arg Thr His
               20
<210> 36
   <211> 69
   <212> DNA
   <213> Homo sapiens
<220>
   <221> CDS
   <222> (1)...(69)
<400> 36
   tat gca tgt cat cta tgt gga aaa gcc ttc act cag agt tct cac ctt
   48
   Tyr Ala Cys His Leu Cys Gly Lys Ala Phe Thr Gln Ser Ser His Leu
   1 5 10 15
   aga aga cat gag aaa act cac
   69
   Arg Arg His Glu Lys Thr His
   20
<210> 37
   <211> 23
   <212> PRT
   <213> Homo sapiens
<400> 37
   Tyr Ala Cys His Leu Cys Gly Lys Ala Phe Thr Gln Ser Ser His Leu
   1 5 10 15
   Arg Arg His Glu Lys Thr His
   20
<210> 38
   <211> 69
   <212> DNA
   <213> Homo sapiens
<220>
   <221> CDS
   <222> (1)...(69)
<400> 38
   tat aaa tgc ggc cag tgt ggg aag ttc tac tcg cag gtc tcc cac ctc
   48
   Tyr Lys Cys Gly Gln Cys Gly Lys Phe Tyr Ser Gln Val Ser His Leu
   1 5 10 15
   acc cgc cac cag aaa atc cac
   69
   Thr Arg His Gln Lys Ile His
   20
<210> 39
   <211> 23
   <212> PRT
   <213> Homo sapiens
<400> 39
   Tyr Lys Cys Gly Gln Cys Gly Lys Phe Tyr Ser Gln Val Ser His Leu
   1 5 10 15
   Thr Arg His Gln Lys Ile His
   20
<210> 40
   <211> 69
   <212> DNA
   <213> Homo sapiens
<220>
   <221> CDS
   <222> (1)...(69)
<400> 40
   tat gca tgt cat cta tgt gga aaa gcc ttc act cag tgt tct cac ctt
   48
   Tyr Ala Cys His Leu Cys Gly Lys Ala Phe Thr Gln Cys Ser His Leu
    1               5                  10                  15
   aga aga cat gag aaa act cac
   69
   Arg Arg His Glu Lys Thr His
                   20
<210> 41
   <211> 23
   <212> PRT
   <213> Homo sapiens
<400> 41
   Tyr Ala Cys His Leu Cys Gly Lys Ala Phe Thr Gln Cys Ser His Leu
    1               5                  10                  15
   Arg Arg His Glu Lys Thr His
                   20
<210> 42
   <211> 69
   <212> DNA
   <213> Homo sapiens
<220>
   <221> CDS
   <222> (1)...(69)
<400> 42
   tat gca tgt cat cta tgt gca aaa gcc ttc att cag tgt tct cac ctt
   48
   Tyr Ala Cys His Leu Cys Ala Lys Ala Phe Ile Gln Cys Ser His Leu
    1               5                  10                  15
   aga aga cat gag aaa act cac          69
   Arg Arg His Glu Lys Thr His
                   20
<210> 43
   <211> 23
   <212> PRT
   <213> Homo sapiens
<400> 43
   Tyr Ala Cys His Leu Cys Ala Lys Ala Phe Ile Gln Cys Ser His Leu
    1               5                   10                  15
   Arg Arg His Glu Lys Thr His
               20
<210> 44
   <211> 69
   <212> DNA
   <213> Homo sapiens
<220>
   <221> CDS
   <222> (1)...(69)
<400> 44
   tat gtt tgc agg gaa tgt ggg cgt ggc ttt cgc cag cat tca cac ctg
   48
   Tyr Val Cys Arg Glu Cys Gly Arg Gly Phe Arg Gln His Ser His Leu
    1               5                   10                  15
   gtc aga cac aag agg aca cat
   69
   Val Arg His Lys Arg Thr His
                20
<210> 45
   <211> 23
   <212> PRT
   <213> Homo sapiens
<400> 45
   Tyr Val Cys Arg Glu Cys Gly Arg Gly Phe Arg Gln His Ser His Leu
    1               5                   10                  15
   Val Arg His Lys Arg Thr His
               20
<210> 46
   <211> 69
   <212> DNA
   <213> Homo sapiens
<220>
   <221> CDS
   <222> (1)...(69)
<400> 46
   ttt gag tgt aaa gat tgc ggg aaa gct ttc att cag aag tca aac ctc
   48
   Phe Glu Cys Lys Asp Cys Gly Lys Ala Phe Ile Gln Lys Ser Asn Leu
   1 5 10 15
   atc aga cac cag aga act cac
   69
   Ile Arg His Gln Arg Thr His
   20
<210> 47
   <211> 23
   <212> PRT
   <213> Homo sapiens
<400> 47
   Phe Glu Cys Lys Asp Cys Gly Lys Ala Phe Ile Gln Lys Ser Asn Leu
   1 5 10 15
   Ile Arg His Gln Arg Thr His
   20
<210> 48
   <211> 69
   <212> DNA
   <213> Homo sapiens
<220>
   <221> CDS
   <222> (1)...(69)
<400> 48
   tat gtc tgc agg gag tgt agg cga ggt ttt agc cag aag tca aat ctc
   48
   Tyr Val Cys Arg Glu Cys Arg Arg Gly Phe Ser Gln Lys Ser Asn Leu
   1 5 10 15
   atc aga cac cag agg acg cac
   69
   Ile Arg His Gln Arg Thr His
   20
<210> 49
   <211> 23
   <212> PRT
   <213> Homo sapiens
<400> 49
   Tyr Val Cys Arg Glu Cys Arg Arg Gly Phe Ser Gln Lys Ser Asn Leu
   1 5 10 15
   Ile Arg His Gln Arg Thr His
   20
<210> 50
   <211> 69
   <212> DNA
   <213> Homo sapiens
<220>
   <221> CDS
   <222> (1)...(69)
<400> 50
   tat gaa tgt aac aca tgc agg aaa acc ttc tct caa aag tca aat ctc    48
   Tyr Glu Cys Asn Thr Cys Arg Lys Thr Phe Ser Gln Lys Ser Asn Leu
    1               5                  10                  15
   att gta cat cag aga aca cac                                        69
   Ile Val His Gln Arg Thr His
                   20
<210> 51
   <211> 23
   <212> PRT
   <213> Homo sapiens
<400> 51
   Tyr Glu Cys Asn Thr Cys Arg Lys Thr Phe Ser Gln Lys Ser Asn Leu
    1               5                  10                  15
   Ile Val His Gln Arg Thr His
               20
<210> 52
   <211> 69
   <212> DNA
   <213> Homo sapiens
<220>
   <221> CDS
   <222> (1)...(69)
<400> 52
   tat gtt tgc tca aaa tgt ggg aaa gcc ttc act cag agt tca aat ctg    48
   Tyr Val Cys Ser Lys Cys Gly Lys Ala Phe Thr Gln Ser Ser Asn Leu
    1               5                  10                  15
   act gta cat caa aaa atc cac                                        69
   Thr Val His Gln Lys Ile His
                   20
<210> 53
   <211> 23
   <212> PRT
   <213> Homo sapiens
<400> 53
   Tyr Val Cys Ser Lys Cys Gly Lys Ala Phe Thr Gln Ser Ser Asn Leu
    1               5                  10                  15
   Thr Val His Gln Lys Ile His
               20
<210> 54
   <211> 69
   <212> DNA
   <213> Homo sapiens
<220>
   <221> CDS
   <222> (1)...(69)
<400> 54
   tac aaa tgt gac gaa tgt gga aaa aac ttt acc cag tcc tcc aac ctt
   48
   Tyr Lys Cys Asp Glu Cys Gly Lys Asn Phe Thr Gln Ser Ser Asn Leu
    1               5                  10                  15
   att gta cat aag aga att cat
   69
   Ile Val His Lys Arg Ile His
                   20
<210> 55
   <211> 23
   <212> PRT
   <213> Homo sapiens
<400> 55
   Tyr Lys Cys Asp Glu Cys Gly Lys Asn Phe Thr Gln Ser Ser Asn Leu
    1               5                  10                  15
   Ile Val His Lys Arg Ile His
               20
<210> 56
   <211> 69
   <212> DNA
   <213> Homo sapiens
<220>
   <221> CDS
   <222> (1)...(69)
<400> 56
   tat gaa tgt gat gtg tgt gga aaa acc ttc acg caa aag tca aac ctt
   48
   Tyr Glu Cys Asp Val Cys Gly Lys Thr Phe Thr Gln Lys Ser Asn Leu
    1               5                  10                  15
   ggt gta cat cag aga act cat
   69
   Gly Val His Gln Arg Thr His
                   20
<210> 57
   <211> 23
   <212> PRT
   <213> Homo sapiens
<400> 57
   Tyr Glu Cys Asp Val Cys Gly Lys Thr Phe Thr Gln Lys Ser Asn Leu
   1 5 10 15
   Gly Val His Gln Arg Thr His
   20
<210> 58
   <211> 69
   <212> DNA
   <213> Homo sapiens
<220>
   <221> CDS
   <222> (1)...(69)
<400> 58
   tat aag tgc cct gat tgt ggg aag agt ttt agt cag agt tcc agc ctc
   48
   Tyr Lys Cys Pro Asp Cys Gly Lys Ser Phe Ser Gln Ser Ser Ser Leu
   1 5 10 15
   att cgc cac cag cgg aca cac
   69
   Ile Arg His Gln Arg Thr His
   20
<210> 59
   <211> 23
   <212> PRT
   <213> Homo sapiens
<400> 59
   Tyr Lys Cys Pro Asp Cys Gly Lys Ser Phe Ser Gln Ser Ser Ser Leu
   1 5 10 15
   Ile Arg His Gln Arg Thr His
   20
<210> 60
   <211> 69
   <212> DNA
   <213> Homo sapiens
<220>
   <221> CDS
   <222> (1)...(69)
<400> 60
   tat gag tgt cag gac tgt ggg agg gcc ttc aac cag aac tcc tcc ctg
   48
   Tyr Glu Cys Gln Asp Cys Gly Arg Ala Phe Asn Gln Asn Ser Ser Leu
   1 5 10 15
   ggg cgg cac aag agg aca cac
   69
   Gly Arg His Lys Arg Thr His
   20
<210> 61
   <211> 23
   <212> PRT
   <213> Homo sapiens
<400> 61
   Tyr Glu Cys Gln Asp Cys Gly Arg Ala Phe Asn Gln Asn Ser Ser Leu
    1               5                   10                  15
   Gly Arg His Lys Arg Thr His
               20
<210> 62
   <211> 69
   <212> DNA
   <213> Homo sapiens
<220>
   <221> CDS
   <222> (1)...(69)
<400> 62
   tac aaa tgt gaa gaa tgt ggc aaa gct ttt aac cag tcc tca acc ctt
   48
   Tyr Lys Cys Glu Glu Cys Gly Lys Ala Phe Asn Gln Ser Ser Thr Leu
    1               5                  10                  15
   act aga cat aag ata gtt cat
   69
   Thr Arg His Lys Ile Val His
               20
<210> 63
   <211> 23
   <212> PRT
   <213> Homo sapiens
<400> 63
   Tyr Lys Cys Glu Glu Cys Gly Lys Ala Phe Asn Gln Ser Ser Thr Leu
    1               5                  10                  15
   Thr Arg His Lys Ile Val His
               20
<210> 64
   <211> 69
   <212> DNA
   <213> Homo sapiens
<220>
   <221> CDS
   <222> (1)...(69)
<400> 64
   tat aag tgc atg gag tgt ggg aag gct ttt aac cgc agg tca cac ctc
   48
   Tyr Lys Cys Met Glu Cys Gly Lys Ala Phe Asn Arg Arg Ser His Leu
   1 5 10 15
   aca cgg cac cag cgg att cac
   69
   Thr Arg His Gln Arg Ile His
   20
<210> 65
   <211> 23
   <212> PRT
   <213> Homo sapiens
<400> 65
   Tyr Lys Cys Met Glu Cys Gly Lys Ala Phe Asn Arg Arg Ser His Leu
   1 5 10 15
   Thr Arg His Gln Arg Ile His
   20
<210> 66
   <211> 69
   <212> DNA
   <213> Homo sapiens
<220>
   <221> CDS
   <222> (1)...(69)
<400> 66
   tat aca tgt aaa cag tgt ggg aaa gcc ttc agt gtt tcc agt tcc ctt
   48
   Tyr Thr Cys Lys Gln Cys Gly Lys Ala Phe Ser Val Ser Ser Ser Leu
   1 5 10 15
   cga aga cat gaa acc act cac
   69
   Arg Arg His Glu Thr Thr His
   20
<210> 67
   <211> 23
   <212> PRT
   <213> Homo sapiens
<400> 67
   Tyr Thr Cys Lys Gln Cys Gly Lys Ala Phe Ser Val Ser Ser Ser Leu
   1 5 10 15
   Arg Arg His Glu Thr Thr His
   20
<210> 68
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 68
   Xaa Xaa Cys Xaa Cys Xaa Xaa Xaa Xaa Xaa Cys Xaa Ser Asn Xaa Xaa
    1               5                  10                  15
   Arg His Xaa His
               20
<210> 69
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 69
   Xaa Xaa Cys Xaa Cys Xaa Xaa Xaa Xaa Xaa His Xaa Ser Asn Xaa Xaa
    1               5                  10                  15
   Lys His Xaa His
               20
<210> 70
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 70
   Xaa Xaa Cys Xaa Cys Xaa Xaa Xaa Xaa Xaa Ser Xaa Ser Asn Xaa Xaa
    1               5                  10                  15
   Arg His Xaa His
               20
<210> 71
   <211> 20 -
<212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 71
   Xaa Xaa Cys Xaa Cys Xaa Xaa Xaa Xaa Xaa Gln Xaa Ser Thr Xaa Xaa
    1               5                  10                  15
   Val His Xaa His
               20
<210> 72
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 14
   <223> Xaa = Ser or Thr
<221> VARIANT
   <222> 15
   <223> Xaa = hydrophobic residue
<221> VARIANT
   <222> (19)...(19)
   <223> Xaa = any amino acid; 3-5 amino acids in length
<400> 72
   Xaa Xaa Cys Xaa Cys Xaa Xaa Xaa Xaa Xaa Val Xaa Ser Xaa Xaa Xaa
    1               5                  10                  15
   Arg His Xaa His
               20
<210> 73
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 73
   Xaa Xaa Cys Xaa Cys Xaa Xaa Xaa Xaa Xaa Gln Xaa Ser His Xaa Xaa
    1               5                  10                  15
   Arg His Xaa His
               20
<210> 74
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 15
   <223> Xaa = hydrophobic residue
<221> VARIANT
   <222> 19
   <223> Xaa = any amino acid; 3-5 amino acids in length
<400> 74
   Xaa Xaa Cys Xaa Cys Xaa Xaa Xaa Xaa Xaa Gln Xaa Ser Asn Xaa Xaa
    1               5                  10                  15
   Val His Xaa His
               20
<210> 75
   <211> 20
   <212> PRT
   <213> Artificial Sequence
<220>
   <223> purified polypeptide
<221> VARIANT
   <222> 4
   <223> Xaa = any amino acid; 2-5 amino acids in length
<221> VARIANT
   <222> 2, 6-8, 10, 12, 16
   <223> Xaa = any amino acid
<221> VARIANT
   <222> 1, 9
   <223> Xaa = Phe or Tyr
<221> VARIANT
   <222> 14
   <223> Xaa = Ser or Thr .
<221> VARIANT
   <222> 15
   <223> Xaa = hydrophobic residue
<221> VARIANT
   <222> (19)...(19)
   <223> Xaa = any amino acid; 3-5 amino acids in length
<400> 75
   Xaa Xaa Cys Xaa Cys Xaa Xaa Xaa Xaa Xaa Gln Xaa Ser Xaa Xaa Xaa
    1              5                  10                  15
   Arg His Xaa His
               20
<210> 76
   <211> 20
   <212> PRT
   <213> Artificial Sequence
<220>
   <223> coordinating residue
<221> VARIANT
   <222> 1, 9
   <223> Xaa = Phe or Tyr
<221> VARIANT
   <222> 2, 6-8, 10-14, 16, 17
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 76
   Xaa Xaa Cys Xaa Cys Xaa Xaa Xaa Xaa Xaa Xaa Xaa Xaa Xaa Xaa Xaa
    1              5                  10                  15
   Xaa His Xaa His
               20
<210> 77
   <211> 24
   <212> PRT
   <213> Artificial Sequence
<220>
<223> polypeptide motif
<221> VARIANT
   <222> 1
   <223> Xaa = Leu, Ile, Val, Met, Phe, Tyr, or Gly
<221> VARIANT
   <222> 2
   <223> Xaa = Ala, Ser, Leu, Val, or Arg
<221> VARIANT
   <222> 3-4, 6, 8-11, 17, 19-23
   <223> Xaa = any amino acid
<221> VARIANT
   <222> 5
   <223> Xaa = Leu, Ile, Val, Met, Ser, Thr, Ala, Cys, or Asn
<221> VARIANT
   <222> 7
   <223> Xaa = Leu, Ile, Val, or Met
<221> VARIANT
   <222> (12)...(12)
   <223> Xaa = Leu, Ile, or Val
<221> VARIANT
   <222> (13)...(13)
   <223> Xaa = Arg, Lys, Asn, Gln, Glu, Ser, Thr, Ala, Ile, or Tyr
<221> VARIANT
   <222> (14)...(14)
   <223> Xaa = Leu, Ile, Val, Phe, Ser, Thr, Asn, Lys, or His
<221> VARIANT
   <222> (16)...(16)
   <223> Xaa = Phe, Tyr, Val, or Cys
<221> VARIANT
   <222> (18)...(18)
   <223> Xaa = Asn, Asp, Gln, Thr, Ala, or His
<221> VARIANT
   <222> (24)...(24)
   <223> Xaa = Arg, Lys, Asn, Ala, Ile, Met, or Trp
<400> 77
   Xaa Xaa Xaa Xaa Xaa Xaa Xaa Xaa Xaa Xaa Xaa Xaa Xaa Xaa Trp Xaa
    1               5                  10                  15
   Xaa Xaa Xaa Xaa Xaa Xaa Xaa Xaa
               20
<210> 78
   <211> 6
   <212> PRT
   <213> Eukaryote
<220>
   <221> VARIANT
   <222> 3
   <223> Xaa = Glu or Gln
<221> VARIANT
   <222> 4
   <223> Xaa = Lys or Arg
<221> VARIANT
   <222> 6
   <223> Xaa = Tyr or Phe
<400> 78
   Thr Gly Xaa Xaa Pro Xaa
    1               5
<210> 79
   <211> 29
   <212> DNA
   <213> Artificial Sequence
<220>
   <223> synthetic oligonucleotide
<400> 79
   tgcctgcagc atttgtggga ggaagtttg
   29
<210> 80
   <211> 30
   <212> DNA
   <213> Artificial Sequence
<220>
   <223> synthetic oligonucleotide
<400> 80
   atgctgcagg cttaaggctt ctcgccggtg
   30
<210> 81
   <211> 24
   <212> DNA
   <213> Artificial Sequence
<220>
   <223> primer for PCR
<221> mise feature
   <222> (0)...(0)
   <223> n = A, T, G, or C; y = T or C; s = G or C; r = G or A
<400> 81
   gcgtccggac ncayacnggn sara
   24
<210> 82
   <211> 24
   <212> DNA
   <213> Artificial Sequence
<220>
   <223> primer for PCR
   <221> misc_feature
   <222> (0)...(0)
   <223> n = A, T, G, or C; b = G, C, or T;r = G or A; w = A or T; y = T or C
<400> 82
   cggaattcan nbrwanggyy tytc
   24
<210> 83
   <211> 7
   <212> PRT
   <213> Artificial Sequence
<220>
   <223> amino acid motif
<221> VARIANT
   <222> 4
   <223> Xaa = Glu or GIn
<221> VARIANT
   <222> 5
   <223> Xaa = Lys or Arg
<221> VARIANT
   <222> 3
   <223> Xaa = Tyr or Phe
<400> 83
   His Thr Gly Xaa Xaa Pro Xaa
   1 5
<210> 84
   <211> 54
   <212> DNA
   <213> Artificial Sequence
<220>
   <223> synthetic oligonucleotide
<400> 84
   gggcccgggg agaagcctta cgcatgtcca gtcgaatctt gtgatagaag attc
   54
<210> 85
   <211> 75
   <212> DNA
   <213> Artificial Sequence
<220>
   <223> synthetic oligonucleotide
<221> misc_feature
   <222> (0)...(0)
   <223> n = A, T, G, or C ; b = G, C, or T; s = G or C
<400> 85
   ctccccgcgg ttcgccggtg tggattctga tatgsnbsnb aagsnbsnbs nbsnbtgaga
   60
   atcttctatc acaag
   75
<210> 86
   <211> 23
   <212> DNA
   <213> Artificial Sequence
<220>
   <223> synthetic oligonucleotide
<400> 86
   ctagacccgg gaattcgtcg acg
   23
<210> 87
   <211> 23
   <212> DNA
   <213> Artificial Sequence
<220>
   <223> synthetic oligonucleotide
<400> 87
   gatccgtcga cgaattcccg ggt
   23
<210> 88
   <211> 38
   <212> DNA
   <213> syArtificial Sequence
<220>
   <223> synthetic oligonucleotide
<221> misc feature
   <222> (0) .. (0)
   <223> n = A, T, G, or C
<400> 88
   ccggtnnntg ggcgtacnnn tgggcgtcan nntgggcg
   38
<210> 89
   <211> 38
   <212> DNA
   <213> Artificial Sequence
<220>
   <223> synthetic oligonucleotide
<221> misc_feature
   <222> (0)...(0)
   <223> n = A, T, G, or C
<400> 89
   tcgacgccca nnntgacgcc cannngtacg cccannna
   38
<210> 90
   <211> 24
   <212> DNA
   <213> Artificial Sequence
<220>
   <223> synthetic probe for gel shift assay
<400> 90
   ccgggtcgcg cgtgggcggt accg
   24
<210> 91
   <211> 24
   <212> DNA
   <213> Artificial Sequence
<220>
   <223> synthetic probe for gel shift assay
<400> 91
   tcgacggtac cgcccacgcg cgac
   24
<210> 92
   <211> 24
   <212> DNA
   <213> Artificial Sequence
<220>
   <223> synthetic probe for gel shift assay
<400> 92
   ccgggtcgcg agcgggcggt accg
   24
<210> 93
   <211> 24
   <212> DNA
   <213> Artificial Sequence
<220>
   <223> synthetic probe for gel shift assay
<400> 93
   tcgacggtac cgcccgctcg cgac
   24
<210> 94
   <211> 24
   <212> DNA
   <213> Artificial Sequence
<220>
   <223> synthetic probe for gel shift assay
<400> 94
   ccgggtcgtg cttgggcggt accg
   24
<210> 95
   <211> 24
   <212> DNA
   <213> Artificial Sequence
<220>
   <223> synthetic probe for gel shift assay
<400> 95
   tcgacggtac cgcccaagca cgac
   24
<210> 96
   <211> 24
   <212> DNA
   <213> Artificial Sequence
<220>
   <223> synthetic probe for gel shift assay
<400> 96
   ccgggtcggg actgggcggt accg
   24
<210> 97
   <211> 24
   <212> DNA
   <213> Artificial Sequence
<220>
   <223> synthetic probe for gel shift assay
<400> 97
   tcgacggtac cgcccagtcc cgac
   24
<210> 98
   <211> 24
   <212> DNA
   <213> Artificial Sequence
<220>
   <223> synthetic probe for gel shift assay
<400> 98
   ccgggtcggg agtgggcggt accg
   24
<210> 99
   <211> 24
   <212> DNA
   <213> Artificial Sequence
<220>
   <223> synthetic probe for gel shift assay
<400> 99
   tcgacggtac cgcccactcc cgac
   24
<210> 100
   <211> 24
   <212> DNA
   <213> Artificial Sequence
<220>
   <223> synthetic probe for gel shift assay
<400> 100
   ccgggtcgga catgggcggt accg
   24
<210> 101
   <211> 24
   <212> DNA
   <213> Artificial Sequence
<220>
   <223> synthetic probe for gel shift assay
<400> 101
   tcgacggtac cgcccatgtc cgac
   24
<210> 102
   <211> 69
   <212> DNA
   <213> Homo sapiens
<220>
   <221> CDS
   <222> (1)...(69)
<400> 102
   tat aag tgt aag gaa tgt ggg cag gcc ttt aga cag cgt gca cat ctt
   48
   Tyr Lys Cys Lys Glu Cys Gly Gln Ala Phe Arg Gln Arg Ala His Leu
    1               5                  10                  15
   att cga cat cac aaa ctt cac
   69
   Ile Arg His His Lys Leu His
               20
<210> 103
   <211> 23
   <212> PRT
   <213> Homo sapiens
<400> 103
   Tyr Lys Cys Lys Glu Cys Gly Gln Ala Phe Arg Gln Arg Ala His Leu
    1               5                  10                  15
   Ile Arg His His Lys Leu His
               20
<210> 104
   <211> 69
   <212> DNA
   <213> Homo sapiens
<220>
   <221> CDS
   <222> (1)...(69)
<400> 104
   tat aag tgt cat caa tgt ggg aaa gcc ttt att caa tcc ttt aac ctt
   48
   Tyr Lys Cys His Gln Cys Gly Lys Ala Phe Ile Gln Ser Phe Asn Leu
    1               5                  10                  15
   cga aga cat gag aga act cac
   69
   Arg Arg His Glu Arg Thr His
               20
<210> 105
   <211> 23
   <212> PRT
   <213> Homo sapiens
<400> 105
   Tyr Lys Cys His Gln Cys Gly Lys Ala Phe Ile Gln Ser Phe Asn Leu
   1 5 10 15
   Arg Arg His Glu Arg Thr His
   20
<210> 106
   <211> 69
   <212> DNA
   <213> Homo sapiens
<220>
   <221> CDS
   <222> (1) ... (69)
<400> 106
   ttc cag tgt aat cag tgt ggg gca tct ttt act cag aaa ggt aac ctc
   48
   Phe Gln Cys Asn Gln Cys Gly Ala Ser Phe Thr Gln Lys Gly Asn Leu
   1 5 10 15
   ctc cgc cac att aaa ctg cac
   69
   Leu Arg His Ile Lys Leu His
   20
<210> 107
   <211> 23
   <212> PRT
   <213> Homo sapiens
<400> 107
   Phe Gln Cys Asn Gln Cys Gly Ala Ser Phe Thr Gln Lys Gly Asn Leu
   1 5 10 15
   Leu Arg His Ile Lys Leu His
   20
<210> 108
   <211> 22
   <212> DNA
   <213> Artificial Sequence
<220>
   <223> primer for PCR
<221> misc_feature
   <222> (0)...(0)
   <223> n =A, T, G, or C; 48-51 nucleotides in length
<400> 108
   acccacactg gccagaaacc cn
   22
<210> 109
   <211> 22
   <212> DNA
   <213> Artificial Sequence
<220>
   <223> primer for PCR
<221> misc_feature
   <222> (0)..(0)
   <223> n = A, T, G, or C; 42-45 nucleotides in length
<400> 109
   gatctgaatt cattcaccgg tn
   22
<210> 110
   <211> 69
   <212> DNA
   <213> Homo sapiens
<220>
   <221> CDS
   <222> (1)...(69)
<400> 110
   tac aaa tgt gaa gaa tgt ggc aaa gcc ttt agg cag tcc tca cac ctt
   48
   Tyr Lys Cys Glu Glu Cys Gly Lys Ala Phe Arg Gln Ser Ser His Leu
   1 5 10 15
   act aca cat aag ata att cat
   69
   Thr Thr His Lys Ile Ile His
   20
<210> 111
   <211> 23
   <212> PRT
   <213> Homo sapiens
<400> 111
   Tyr Lys Cys Glu Glu Cys Gly Lys Ala Phe Arg Gln Ser Ser His Leu
   1 5 10 15
   Thr Thr His Lys Ile Ile His
   20
<210> 112
   <211> 69
   <212> DNA
   <213> Homo sapiens
<220>
   <221> CDS
   <222> (1)...(69)
<400> 112
   tat gag tgt gat cac tgt gga aaa tcc ttt agc cag agc tct cat ctg
   48
   Tyr Glu Cys Asp His Cys Gly Lys Ser Phe Ser Gln Ser Ser His Leu
    1               5                   10                  15
   aat gtg cac aaa aga act cac
   69
   Asn Val His Lys Arg Thr His
                   20
<210> 113
   <211> 23
   <212> PRT
   <213> Homo sapiens
<400> 113
   Tyr Glu Cys Asp His Cys Gly Lys Ser Phe Ser Gln Ser Ser His Leu
    1               5                   10                  15
   Asn Val His Lys Arg Thr His
                   20
<210> 114
   <211> 69
   <212> DNA
   <213> Homo sapiens
<220>
   <221> CDS
   <222> (1)...(69)
<400> 114
   tac atg tgc agt gag tgt ggg cga ggc ttc agc cag aag tca aac ctc
   48
   Tyr Met Cys Ser Glu Cys Gly Arg Gly Phe Ser Gln Lys Ser Asn Leu
    1               5                   10                  15
   atc ata cac cag agg aca cac
   69
   Ile Ile His Gln Arg Thr His
                   20
<210> 115
   <211> 23
   <212> PRT
   <213> Homo sapiens
<400> 115
   Tyr Met Cys Ser Glu Cys Gly Arg Gly Phe Ser Gln Lys Ser Asn Leu
    1               5                   10                  15
   Ile Ile His Gln Arg Thr His
                   20
<210> 116
   <211>69
   <212> DNA
   <213> Homo sapiens
<220>
   <221> CDS
   <222> (1)...(69)
<400> 116
   tat gaa tgt gaa aaa tgt ggc aaa gct ttt aac cag tcc tca aat ctt
   48
   Tyr Glu Cys Glu Lys Cys Gly Lys Ala Phe Asn Gln Ser Ser Asn Leu
    1               5                  10                  15
   act aga cat aag aaa agt cat
   69
   Thr Arg His Lys Lys Ser His
                20
<210> 117
   <211> 23
   <212> PRT
   <213> Homo sapiens
<400> 117
   Tyr Glu Cys Glu Lys Cys Gly Lys Ala Phe Asn Gln Ser Ser Asn Leu
    1               5                  10                  15
   Thr Arg His Lys Lys Ser His
                20
<210> 118
   <211> 69
   <212> DNA
   <213> Homo sapiens
<220>
   <221> CDS
   <222> (1)...(69)
<400> 118
   tat gag tgc aat gaa tgt ggg aag ttt ttt agc cag agc tcc agc ctc
   48
   Tyr Glu Cys Asn Glu Cys Gly Lys Phe Phe Ser Gln Ser Ser Ser Leu
    1               5                  10                  15
   att aga cat agg aga agt cac         69
   Ile Arg His Arg Arg Ser His
                20
<210> 119
   <211> 23
   <212> PRT
   <213> Homo sapiens
<400> 119
   Tyr Glu Cys Asn Glu Cys Gly Lys Phe Phe Ser Gln Ser Ser Ser Leu
    1               5                   10                  15
   Ile Arg His Arg Arg Ser His
                20
<210> 120
   <211> 69
   <212> DNA
   <213> Homo sapiens
<220>
   <221> CDS
   <222> (1)...(59)
<400> 120
   tat gag tgt cac gat tgc gga aag tcc ttt agg cag agc acc cac ctc
   48
   Tyr Glu Cys His Asp Cys Gly Lys Ser Phe Arg Gln Ser Thr His Leu
    1               5                   10                  15
   act cag cac cgg agg atc cac
   69
   Thr Gln His Arg Arg Ile His
                20
<210> 121
   <211> 23
   <212> PRT
   <213> Homo sapiens
<400> 121
   Tyr Glu Cys His Asp Cys Gly Lys Ser Phe Arg Gln Ser Thr His Leu
    1               5                   10                  15
   Thr Gln His Arg Arg Ile His
                20
<210> 122
   <211> 69
   <212> DNA
   <213> Homo sapiens
<220>
   <221> CDS
   <222> (1)...(69)
<400> 122
   tat gag tgt cac gat tgc gga aag tcc ttt agg cag agc acc cac ctc
   48
   Tyr Glu Cys His Asp Cys Gly Lys Ser Phe Arg Gln Ser Thr His Leu
   1 5 10 15
   act cgg cac cgg agg atc cac
   69
   Thr Arg His Arg Arg Ile His
   20
<210> 123
   <211> 23
   <212> PRT
   <213> Homo sapiens
<400> 123
   Tyr Glu Cys His Asp Cys Gly Lys Ser Phe Arg Gln Ser Thr His Leu
   1 5 10 15
   Thr Arg His Arg Arg Ile His
   20
<210> 124
   <211> 69
   <212> DNA
   <213> Homo sapiens
<220>
   <221> CDS
   <222> (1)...(69)
<400> 124
   cac aag tgc ctt gaa tgt ggg aaa tgc ttc agt cag aac acc cat ctg
   48
   His Lys Cys Leu Glu Cys Gly Lys Cys Phe Ser Gln Asn Thr His Leu
   1 5 10 15
   act cgc cac caa cgc acc cac
   69
   Thr Arg His Gln Arg Thr His
   20
<210> 125
   <211> 23
   <212> PRT
   <213> Homo sapiens
<400> 125
   His Lys Cys Leu Glu Cys Gly Lys Cys Phe Ser Gln Asn Thr His Leu
   1 5 10 15
   Thr Arg His Gln Arg Thr His
   20
<210> 126
   <211> 75
   <212> DNA
   <213> Homo sapiens
<220>
   <221> CDS
   <222> (1)...(75)
<400> 126
   tac cac tgt gac tgg gac ggc tgt gga tgg aaa ttc gcc cgc tca gat 48
   Tyr His Cys Asp Trp Asp Gly Cys Gly Trp Lys Phe Ala Arg Ser Asp
   1 5 10 15
   gaa ctg acc agg cac tac cgt aaa cac 75
   Glu Leu Thr Arg His Tyr Arg Lys His
   20 25
<210> 127
   <211> 25
   <212> PRT
   <213> Homo sapiens
<400> 127
   Tyr His Cys Asp Trp Asp Gly Cys Gly Trp Lys Phe Ala Arg Ser Asp
   1 5 10 15
   Glu Leu Thr Arg His Tyr Arg Lys His
   20 25
<210> 128
   <211> 75
   <212> DNA
   <213> Homo sapiens
<220>
   <221> CDS
   <222> (1)...(75)
<400> 128
   tac aga tgc tca tgg gaa ggg tgt gag tgg cgt ttt gca aga agt gat 48
   Tyr Arg Cys Ser Trp Glu Gly Cys Glu Trp Arg Phe Ala Arg Ser Asp
   1 5 10 15
   gag tta acc agg cac ttc cga aag cac 75
   Glu Leu Thr Arg His Phe Arg Lys His
   20 25
<210> 129
   <211> 25
   <212> PRT
   <213> Homo sapiens
<400> 129
   Tyr Arg Cys Ser Trp Glu Gly Cys Glu Trp Arg Phe Ala Arg Ser Asp
   1 5 10 15
   Glu Leu Thr Arg His Phe Arg Lys His
   20 25
<210> 130
   <211> 75
   <212> DNA
   <213> Homo sapiens
<220>
   <221> CDS
   <222> (1)...(75)
<400> 130
   ttc agc tgt agc tgg aaa ggt tgt gaa agg agg ttt gcc cgt tct gat
   48
   Phe Ser Cys Ser Trp Lys Gly Cys Glu Arg Arg Phe Ala Arg Ser Asp
    1               5                  10                  15
   gaa ctg tcc aga cac agg cga acc cac
   75
   Glu Leu Ser Arg His Arg Arg Thr His
               20                  25
<210> 131
   <211> 25
   <212> PRT
   <213> Homo sapiens
<400> 131
   Phe Ser Cys Ser Trp Lys Gly Cys Glu Arg Arg Phe Ala Arg Ser Asp
    1               5                  10                  15
   Glu Leu Ser Arg His Arg Arg Thr His
               20                  25
<210> 132
   <211> 75
   <212> DNA
   <213> Homo sapiens
<220>
   <221> CDS
   <222> (1)...(75)
<400> 132
   ttc gcc tgc agc tgg cag gac tgc aac aag aag ttc gcg cgc tcc gac
   48
   Phe Ala Cys Ser Trp Gln Asp Cys Asn Lys Lys Phe Ala Arg Ser Asp
    1               5                  10                  15
   gag ctg gcg cgg cac tac cgc aca cac
   75
   Glu Leu Ala Arg His Tyr Arg Thr His
               20                  25
<210> 133
   <211> 25
   <212> PRT
   <213> Homo sapiens
<400> 133
   Phe Ala Cys Ser Trp Gln Asp Cys Asn Lys Lys Phe Ala Arg Ser Asp
   1 5 10 15
   Glu Leu Ala Arg His Tyr Arg Thr His
   20 25
<210> 134
   <211> 75
   <212> DNA
   <213> Homo sapiens
<220>
   <221> CDS
   <222> (1)...(75)
<400> 134
   tac cac tgc aac tgg gac ggc tgc ggc tgg aag ttt gcg cgc tca gac
   48
   Tyr His Cys Asn Trp Asp Gly Cys Gly Trp Lys Phe Ala Arg Ser Asp
   1 5 10 15
   gag ctc acg cgc cac tac cga aag cac
   75
   Glu Leu Thr Arg His Tyr Arg Lys His
   20 25
<210> 135
<211> 25
   <212> PRT
   <213> Homo sapiens
<400> 135
   Tyr His Cys Asn Trp Asp Gly Cys Gly Trp Lys Phe Ala Arg Ser Asp
   1 5 10 15
   Glu Leu Thr Arg His Tyr Arg Lys His
   20 25
<210> 136
   <211> 72
   <212> DNA
   <213> Homo sapiens
<220>
   <221> CDS
   <222> (1)...(72)
<400> 136
   ttc ctc tgt cag tat tgt gca cag aga ttt ggg cga aag gat cac ctg
   48
   Phe Leu Cys Gln Tyr Cys Ala Gln Arg Phe Gly Arg Lys Asp His Leu
   1 5 10 15
   act cga cat atg aag aag agt cac
   72
   Thr Arg His Met Lys Lys Ser His
   20
<210> 137
   <211> 24
   <212> PRT
   <213> Homo sapiens
<400> 137
   Phe Leu Cys Gln Tyr Cys Ala Gln Arg Phe Gly Arg Lys Asp His Leu
   1 5 10 15
   Thr Arg His Met Lys Lys Ser His
   20
<210> 138
   <211> 78
   <212> DNA
   <213> Artificial Sequence
<220>
   <223> primer for PCR
<400> 138
   tgtcgaatct gcatgcgtaa cttcagtcgt agtgaccacc ttaccaccca catccggacc
   60
   cacactggcc agaaaccc
   78
<210> 139
   <211> 81
   <212> DNA
   <213> Artificial Sequence
<220>
   <223> primer for PCR
<400> 139
   ggtggcggcc gttacttact tagagctcga cgtcttactt acttagcggc cgcactagta
   60
   gatctgaatt cattcaccgg t
   81
<210> 140
   <211> 69
   <212> DNA
   <213> Homo sapiens
<220>
   <221> CDS
   <222> (1)...(69)
<400> 140
   ttc cag tgt aaa act tgt cag cga aag ttc tcc cgg tcc gac cac ctg
   48
   Phe Gln Cys Lys Thr Cys Gln Arg Lys Phe Ser Arg Ser Asp His Leu
   1 5 10 15
   aag acc cac acc agg act cat
   69
   Lys Thr His Thr Arg Thr His
   20
<210> 141
   <211> 23
   <212> PRT
   <213> Homo sapiens
<400> 141
   Phe Gln Cys Lys Thr Cys Gln Arg Lys Phe Ser Arg Ser Asp His Leu
   1 5 10 15
   Lys Thr His Thr Arg Thr His
   20
<210> 142
   <211> 69
   <212> DNA
   <213> Homo sapiens
<220>
   <221> CDS
   <222> (1)...(69)
<400> 142
   ttt gcc tgc gag gtc tgc ggt gtt cga ttc acc agg aac gac aag ctg
   48
   Phe Ala Cys Glu Val Cys Gly Val Arg Phe Thr Arg Asn Asp Lys Leu
   1 5 10 15
   aag atc cac atg cgg aag cac
   69
   Lys Ile His Met Arg Lys His
   20
<210> 143
   <211> 23
   <212> PRT
   <213> Homo sapiens
<400> 143
   Phe Ala Cys Glu Val Cys Gly Val Arg Phe Thr Arg Asn Asp Lys Leu
   1 5 10 15
   Lys Ile His Met Arg Lys His
   20
<210> 144
   <211> 75
   <212> DNA
   <213> Homo sapiens
<220>
   <221> CDS
   <222> (1)...(75)
<400> 144
   tat gta tgc gat gta gag gga tgt acg tgg aaa ttt gcc cgc tca gat
   48
   Tyr Val Cys Asp Val Glu Gly Cys Thr Trp Lys Phe Ala Arg Ser Asp
    1               5                   10                  15
   aag ctc aac aga cac aag aaa agg cac
   75
   Lys Leu Asn Arg His Lys Lys Arg His
               20                  25
<210> 145
   <211> 25
   <212> PRT
   <213> Homo sapiens
<400> 145
   Tyr Val Cys Asp Val Glu Gly Cys Thr Trp Lys Phe Ala Arg Ser Asp
    1               5                   10                  15
   Lys Leu Asn Arg His Lys Lys Arg His
               20                  25
<210> 146
   <211> 69
   <212> DNA
   <213> Homo sapiens
<220>
   <221> CDS
   <222> (1)...(69)
<400> 146
   tat att tgc aga aag tgt gga cgg ggc ttt agt cgg aag tcc aac ctt
   48
   Tyr Ile Cys Arg Lys Cys Gly Arg Gly Phe Ser Arg Lys Ser Asn Leu
    1               5                   10                  15
   atc aga cat cag agg aca cac
   69
   Ile Arg His Gln Arg Thr His
               20
<210> 147
   <211> 23
   <212> PRT
   <213> Homo sapiens
<400> 147
   Tyr Ile Cys Arg Lys Cys Gly Arg Gly Phe Ser Arg Lys Ser Asn Leu
   1 5 10 15
   Ile Arg His Gln Arg Thr His
   20
<210> 148
   <211> 69
   <212> DNA
   <213> Homo sapiens
<220>
   <221> CDS
   <222> (1)...(69)
<400> 148
   tat cta tgt agt gag tgt gac aaa tgc ttc agt aga agt aca aac ctc
   48
   Tyr Leu Cys Ser Glu Cys Asp Lys Cys Phe Ser Arg Ser Thr Asn Leu
   1 5 10 15
   ata agg cat cga aga act cac
   69
   Ile Arg His Arg Arg Thr His
   20
<210> 149
   <211> 23
   <212> PRT
   <213> Homo sapiens
<400> 149
   Tyr Leu Cys Ser Glu Cys Asp Lys Cys Phe Ser Arg Ser Thr Asn Leu
   1 5 10 15
   Ile Arg His Arg Arg Thr His
   20
<210> 150
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 150
   Xaa Xaa Cys Xaa Cys Xaa Xaa Xaa Xaa Xaa Gln Xaa Ala His Xaa Xaa
    1               5                  10                  15
   Arg His Xaa His
               20
<210> 151
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 151
   Xaa Xaa Cys Xaa Cys Xaa Xaa Xaa Xaa Xaa Gln Xaa Phe Asn Xaa Xaa
    1               5                  10                  15
   Arg His Xaa His
               20
<210> 152
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 152
   Xaa Xaa Cys Xaa Cys Xaa Xaa Xaa Xaa Xaa Gln Xaa Ser His Xaa Xaa
    1               5                  10                  15
   Thr His Xaa His
               20
<210> 153
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 153
   Xaa Xaa Cys Xaa Cys Xaa Xaa Xaa Xaa Xaa Gln Xaa Ser His Xaa Xaa
    1               5                  10                  15
   Val His Xaa His
               20
<210> 154
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 154
   Xaa Xaa Cys Xaa Cys Xaa Xaa Xaa Xaa Xaa Gln Xaa Ser Asn Xaa Xaa
    1               5                  10                  15
   Ile His Xaa His
<210> 155
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> CONFLICT
   <222> 15
   <223> Xaa = hydrophobic residue
<221> VARIANT
   <222> 19
   <223> Xaa = any amino acid; 3-5 amino acids in length
<400> 155
   Xaa Xaa Cys Xaa Cys Xaa Xaa Xaa Xaa Xaa Gln Xaa Ser Asn Xaa Xaa
    1               5                  10                  15
   Arg His Xaa His
               20
<210> 156
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 156
   Xaa Xaa Cys Xaa Cys Xaa Xaa Xaa Xaa Xaa Gln Xaa Thr His Xaa Xaa
    1               5                   10                  15
   Gln His Xaa His
               20
<210> 157
   <211> 18
   <212> PRT
   <213> Artificial Sequence
<220>
   <223> purified polypeptide
<221> VARIANT
   <222> 2
   <223> Xaa = any amino acid; 2-5 amino acids in length
<221> VARIANT
   <222> 4-6, 8, 10, 14
   <223> Xaa = any amino acid
<221> VARIANT
   <222> 7
   <223> Xaa = Phe or Tyr
<221> VARIANT
   <222> 13
   <223> Xaa = hydrophobic residue
<221> VARIANT
   <222> 17
   <223> Xaa = any amino acid; 3-5 amino acids in length
<400> 157
   Cys Xaa Cys Xaa Xaa Xaa Xaa Xaa Gln Xaa Thr His Xaa Xaa Arg His
    1               5                   10                  15
   Xaa His
<210> 158
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 158
   Xaa Xaa Cys Xaa Cys Xaa Xaa Xaa Xaa Xaa Arg Xaa Asp Lys Xaa Xaa
    1               5                  10                  15
   Ile His Xaa His
               20
<210> 159
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 159
   Xaa Xaa Cys Xaa Cys Xaa Xaa Xaa Xaa Xaa Arg Xaa Ser Asn Xaa Xaa
    1               5                  10                  15
   Arg His Xaa His
               20
<210> 160
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 160
   Xaa Xaa Cys Xaa Cys Xaa Xaa Xaa Xaa Xaa Thr Xaa Gly Asn Xaa Xaa
    1              5                   10                  15
   Arg His Xaa His
               20
<210> 161
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 161
   Xaa Xaa Cys Xaa Cys Xaa Xaa Xaa Xaa Xaa Gln Xaa Gly Asn Xaa Xaa
    1              5                   10                  15
   Arg His Xaa His
               20
<210> 162
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 162
   Xaa Xaa Cys Xaa Cys Xaa Xaa Xaa Xaa Xaa Arg Xaa Asp Glu Xaa Xaa
    1               5                  10                  15
   Arg His Xaa His
               20
<210> 163
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 163
   Xaa Xaa Cys Xaa Cys Xaa Xaa Xaa Xaa Xaa Arg Xaa Asp His Xaa Xaa
    1               5                  10                  15
   Arg His Xaa His
               20
<210> 164
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 164
   Xaa Xaa Cys Xaa Cys Xaa Xaa Xaa Xaa Xaa Arg Xaa Asp His Xaa Xaa
    1              5                   10                  15
   Thr His Xaa His
               20
<210> 165
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 165
   Xaa Xaa Cys Xaa Cys Xaa Xaa Xaa Xaa Xaa Arg Xaa Asp Lys Xaa Xaa
    1              5                   10                  15
   Arg His Xaa His
               20
<210> 166
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 166
   Xaa Xaa Cys Xaa Cys Xaa Xaa Xaa Xaa Xaa Arg Xaa Ser His Xaa Xaa
    1               5                   10                  15
   Arg His Xaa His
               20
<210> 167
   <211> 20
   <212> PRT
   <213> Artificial Sequence
<220>
   <223> purified polypeptide
<221> VARIANT
   <222> 1, 9
   <223> Xaa = Phe or Tyr
<221> VARIANT
   <222> 2, 6-8, 10, 12, 16
   <223> Xaa = any amino acid
<221> VARIANT
   <222> 4
   <223> Xaa = any amino acid; 2-5 amino acids in length
<221> VARIANT
   <222> 15
   <223> Xaa = hydrophobic residue
<221> VARIANT
   <222> 19
   <223> Xaa = any amino acid; 3-5 amino acids in length
<400> 167
   Xaa Xaa Cys Xaa Cys Xaa Xaa Xaa Xaa Xaa Arg Xaa Thr Asn Xaa Xaa
    1               5                   10                  15
   Arg His Xaa His
               20